# EUROPEAN PATENT SPECIFICATION

(11) **EP 1 470 218 B1**
(45) Date of publication and mention of the grant of the patent: **07.07.2010**
(21) Application number: 02738952.7
(22) Date of filing: 17.05.2002
(51) Int. Cl.: C12N 7/04, C12N 15/86, A61K 39/215, A61K 39/225, C07K 14/165

(54) **CORONA-VIRUS-LIKE PARTICLES COMPRISING FUNCTIONALLY DELETED GENOMES**
CORONAVIRUS-ÄHNLICHE PARTIKEL ENTHALTENDE FUNKTIONELL DELETIERTE GENOME
PARTICULES DE TYPE VIRUS CORONA COMPRENANT DES GENOMES A DELETIONS FONCTIONNELLES

(30) Priority: 17.05.2001 EP 01201861
(43) Date of publication of application: 27.10.2004
(73) Proprietor: Universiteit Utrecht, 3584 CX Utrecht (NL); Stichting voor de Technische Wetenschappen, 3527 JP Utrecht (NL)
(72) Inventor: ROTTIER, Petrus, Josephus, Marie, NL-3737 RG Groenekan (NL); DE HAAN, Cornelis, Alexander, Maria, NL-3993 DE Houten (NL); HAIJEMA, Bert, Jan, NL-3512 GV Utrecht (NL); BOSCH, Berend, Jan, NL-3564 CN Utrecht (NL)
(74) Representative: Prins, Adrianus Willem
(86) International application number: PCT/NL2002/000318
(87) International publication number: WO 2002/092827

(56) References cited:
- WO-A-01/39797
- WO-A-01/39797
- WO-A-98/49195
- WO-A-98/49195
- WO-A-02/086068
- HAAN DE C A M ET AL: "Coronaviruses maintain viability despite dramatic rearrangements of the strictly conserved genome organization" JOURNAL OF VIROLOGY, THE AMERICAN SOCIETY FOR MICROBIOLOGY, US, vol. 76, no. 24, December 2002 (2002-12), pages 12491-12502, XP002355183 ISSN: 0022-538X
- F. FISCHER AT AL.: "Analysis of a recombinant mouse hepatitis virus expressing a foreign gene reveals a novel aspect of coronavirus transcription" J. VIROL., vol. 71, no. 7, 1997, pages 5148-5160, XP002185473
- F. ALMAZAN ET AL.: "Engineering the largest RNA virus genome as an infectious bacterial artifical chromosome" PROC. NATL. ACAD. SCI. USA, vol. 97, no. 10, 2000, pages 5516-5521, XP002166823
- KUO L ET AL: "Retargeting of coronavirus by substitution of the spike glycoprotein ectodomain: crossing the host cell species barrier." JOURNAL OF VIROLOGY FEB 2000, vol. 74, no. 3, February 2000 (2000-02), pages 1393-1406, XP002457853 ISSN: 0022-538X
- GODEKE G-J ET AL: "Assembly of spikes into coronavirus particles is mediated by the carboxy-terminal domain of the spike protein" JOURNAL OF VIROLOGY, THE AMERICAN SOCIETY FOR MICROBIOLOGY, US, vol. 74, no. 3, February 2000 (2000-02), pages 1566-1571, XP002960963 ISSN: 0022-538X
- YOUNT ET AL: "Strategy for systematic assembly of large RNA and DNA genomes: Transmissible gastroenteritis virus model" JOURNAL OF VIROLOGY, THE AMERICAN SOCIETY FOR MICROBIOLOGY, US, vol. 74, no. 22, November 2000 (2000-11), pages 10600-10611, XP002203456 ISSN: 0022-538X
- CURTIS KRISTOPHER M ET AL: "Heterologous gene expression from transmissible gastroenteritis virus replicon particles." JOURNAL OF VIROLOGY FEB 2002, vol. 76, no. 3, February 2002 (2002-02), pages 1422-1434, XP002960965 ISSN: 0022-538X
- WERTZ G W ET AL: "GENE REARRANGEMENT ATTENUATES EXPRESSION AND LETHALITY OF A NONSEGMENTED NEGATIVE STRAND RNA VIRUS" PROCEEDINGS OF THE NATIONAL ACADEMY OF SCIENCES OF USA, NATIONAL ACADEMY OF SCIENCE, WASHINGTON, DC, US, vol. 95, March 1998 (1998-03), pages 3501-3506, XP002911691 ISSN: 0027-8424
- F. FISCHER AT AL.: "Analysis of a recombinant mouse hepatitis virus expressing a foreign gene reveals a novel aspect of coronavirus transcription" J. VIROL., vol. 71, no. 7, 1997, pages 5148-5160, XP002185473
- F. ALMAZAN ET AL.: "Engineering the largest RNA virus genome as an infectious bacterial artifical chromosome" PROC. NATL. ACAD. SCI. USA, vol. 97, no. 10, 2000, pages 5516-5521, XP002166823

## Description

The invention relates to the field of coronaviruses and diagnosis, therapeutic use and vaccines derived thereof.

Coronavirions have a rather simple structure. They consist of a nucleocapsid surrounded by a lipid membrane. The helical nucleocapsid is composed of the RNA genome packaged by one type of protein, the nucleocapsid protein N. The viral envelope generally contains 3 membrane proteins: the spike protein (S), the membrane protein (M) and the envelope protein (E). Some coronaviruses have a fourth protein in their membrane, the hemagglutinin-esterase protein (HE). Like all viruses coronaviruses encode a wide variety of different gene products and proteins. Most important among these are obviously the proteins responsible for functions related to viral replication and virion structure. But besides these elementary functions viruses generally specify a diverse collection of proteins the function of which is often still unknown but which are known or assumed to be in some way beneficial to the virus. These proteins may either be essential - operationally defined as being required for virus replication in cell culture - or dispensable. Coronaviruses constitute a family of large, positive-sense RNA viruses that usually cause respiratory and intestinal infections in many different species. Based on antigenic, genetic and structural protein criteria they have been divided into three distinct groups: group I, II and III. Actually, in view of the great differences between the groups their classification into three different genera is presently being discussed by the responsible ICTV Study Group. The features that all these viruses have in common are a characteristic set of essential genes encoding replication and structural functions. Interspersed between and flanking these genes sequences occur that differ profoundly among the groups and that are, more or less, specific for each group. Of the elementary genes the most predominant one occupies about two-thirds of the genome. Located at the 6' end this so-called polymerase gene encodes two large precursors the many functional cleavage products of which are collectively held responsible for RNA replication and transcription. The other elementary genes specify the basic structural proteins N, M, E, and S. The nucleocapsid (N) protein packages the viral RNA forming the core of the virion. This RNP structure is surrounded by a lipid envelope in which the membrane (M) protein abundantly occurs consituting a dense matrix. Associated with the M protein are the small envelope (E) protein and the spike (S) protein, the latter forming the viral peplomers which are involved in virus-cell and cell-cell fusion. The genes for these structural proteins invariably occur in the viral genome in the order 5'-S-E-M-N-3'.

In infected cells the coronavirus nucleocapsids are assembled in the cytoplasm. The nucleocapsids interact with the viral envelope proteins which after their synthesis in the endoplasmic reticulum accumulate in the intermediate compartment, a membrane system localized between the endoplasmic reticulum (ER) and the Golgi complex. This membrane system acts as the budding compartment: the interaction of the nucleocapsids with the viral envelope proteins leads to the pinching off of virions which are then released from the cell by exocytosis.

We have recently demonstrated that the assembly of coronaviral particles does not require the involvement of nucleocapsids. Particles devoid of a nucleocapsid are assembled in cells when the viral envelope protein genes are co-expressed. The minimal requirements for the formation of virus-like particles (VLP's) are the M and E protein: the S protein is dispensable but is incorporated if present through its interactions with the M protein. Biochemical and electron microscopical analysis revealed that the VLPs are homogeneous in size and have similar dimensions as authentic corona virions. Clearly, the M and E protein have the capacity to associate in the plane of cellular membranes and induce curvature leading to the budding of specific "vesicles" which are subsequently secreted from the cells. An article describing these results has appeared in EMBO Journal (vol. 15, pp. 2020-2028, 1996).

In yet another article, coronavirus like particles were shown which were not devoid of a nucleo-capsid, assembly here did not take place independent of a nucleocapsid (Bos et al., Virology 218, 52-60, 1996). Furthermore, packaging of RNA was not very efficient. Furthermore, neither of these two publications provides sufficient targeting and delivery features which would make the VLP's suitable as therapeutic carrier, for example being equipped with specific targeting information and/or with a genetic or non-genetic message.

However, coronaviruses do have several distinct theoretical advantages for their use as vectors over other viral expression systems (see also WO98/49195): (i) coronaviruses are single-stranded RNA viruses that replicate within the cytoplasm without a DNA intermediary, making unlikely the integration of the virus genome into the host cell chromosome; (ii) these viruses have the largest RNA genome known having in principle room for the insertion of large foreign genes; (iii) since coronaviruses in general infect the mucosal surfaces, both respiratory and enteric, they may be used to induce a strong secretory immune response; (iv) the tropism of coronaviruses may be modified by the manipulation of the spike (S) protein allowing the engineering of the tropism and virulence of the vector; and, (v) non-pathogenic coronavirus strains infecting most species of interest are available to develop expression systems.

Two types of expression vectors have been developed based on coronavirus genomes., One requires two components (helper dependent) and the other a single genome that is modified either by targeted recombination or by engineering a cDNA encoding an infectious RNA. Helper dependent expression systems, also called minigenomes have been developed using members of the three groups of coronaviruses. Coronavirus derived minigenomes have a theoretical cloning capacity close to 25 kb, since minigenome RNAs of about 3 kb are efficiently amplified and packaged by the helper virus and the virus genome has about 30 kb. This is in principle the largest cloning capacity for a vector based on RNA virus genomes.

Reverse genetics has been possible by targeted recombination between a helper virus and either non-replicative or replicative coronavirus derived RNAs (9). Targeted recombination has been mediated by one or two cross-overs. Changes were introduced within the S gene that modified MHV pathogenicity. The gene encoding green fluorescent protein (GFP) was inserted into MHV between genes S and E by targeted recombination, resulting in the creation of a vector with the largest known RNA viral genome (1d). Mutations have also been created by targeted mutagenesis within the E and the M genes showing the crucial role of these genes in assembly.

The construction of a full-length genomic cDNA clone could considerably improve the genetic manipulation of coronaviruses. The construction of an infectious TGEV cDNA clone has recently been made possible (1c). To obtain an infectious cDNA three strategies have been combined (i) the construction of the full-length cDNA was started from a DI that was stably and efficiently replicated by the helper virus. Using this DI, the full-length genome was completed and the performance of the enlarged genome was checked after each step. This approach allowed for the identification of a cDNA fragment that was toxic to the bacterial host. This finding was used to advantage by reintroducing the toxic fragment into the viral cDNA in the last cloning step; (ii) in order to express the long coronavirus genome, and to add the 5' cap, a two-step amplification system that couples transcription in the nucleus from the CMV promoter, with a second amplification in the cytoplasm, driven by the viral replicase, was uses; and, (iii) to increase viral cDNA stability within bacteria, the cDNA was cloned as a bacterial artificial chromosome (BAC), that produces only one or two plasmid copies per cell. The full-length cDNA was divided into two plasmids because their fusion into one reduced the stability of the cDNA. One plasmid contained all virus sequences except for a fragment Cla I to Cla I of abouw 5 kb that was included within a second BAC. A fully functional infectious cDNA clone, leading to a virulent virus able to infect both the enteric and respiratory tracts, was engineered by inserting the Cla I fragment into the rest of the TGEV cDNA sequence.

As said, both helper-dependent expression systems, based on two components and single genomes constructed by targeted recombination or by using an infectious cDNA have been developed. The sequences that regulate transcription have been characterized. Expression of high amounts of heterologous antigens (1 to 8 µg/10⁶ cells) have been achieved, and the expression levels have been maintained for around 10 passages. These expression levels should be sufficient to elicite protective immune responses.

Single genome coronavirus vectors have been constructed efficiently expressing a foreign gene such as GFP. Thus, a new avenue has been opened for coronaviruses which have unique properties, such as a long genome size and enteric tropism, that makes them of high interest as expression vectors, be it that for vaccine development and gene therapy also other conditions need be met, notably host virulence of vector constructs, and viability of vector constructs in cell culture need to be within distinct limitations. One the one hand, the vector may not remain too virulent, but should have at least some attenuated properties rendering it useful for in vivo replication as gene delivery vehicle or vaccine for the host or subject undergoing therapy. On the other hand, the vector should replicate well in cell-culture, at least when commercial use is desired.

The disclosure provides replicative coronaviruses and replicative coronavirus-like particles (VLPs) from which large parts of their genome are (at least functionally) deleted and/or rearranged without abolishing their replicative capacities. Said deletion is preferably resulting in at least a functional deletion in that the corresponding gene is not or only partly expressed whereby the resulting gene product is absent, dysfunctional or at least functionally distinct from a corresponding wild-type gene product. One striking result seen with VLPs provided with deletions and/or rearrangements as provided herein is that said deleted or rearranged VLP, albeit capable of replication in vitro and in vivo, is in general well attenuated, in that it does not cause disease (or do so much less) in the target host, making it very suitable for therapeutic use, for example as a delivery vehicle for genes and other cargo (whereby specific targeting may be provided as well when desired), or for use as a vaccine, being attenuated while carrying important immunogenic determinants that help elicit an immune response. Such determinants may be derived from a (homologous or heterologous) coronavirus, but may also be derived from other pathogens. In other words, the disclosure provides the use of a replicative VLP with a partly deleted and/or rearranged genome as a vector. Into the vector a foreign nucleic acid sequence may be introduced. This foreign gene sequence encodes (part of) a protein. It is this protein, or part there of, encoded by the inserted sequence, that may serve as an immunogen or a "targetting means" ( ligand capable of binding to a receptor).

In a preferred example, said VLP as provided herein are further modified in one of various ways, genomically or in their protein composition, thereby exposing at their surface various biological or target molecules and/or carrying within the particles molecules with biological activity which need to be protected or shielded and/or containing genomes into which foreign genes or sequences have been incorporated. One of the major needs in present-day medicine is systems for the targeted delivery of therapeutic agents in the body. By consequence, the development of carriers that can direct cargo to specified groups of cells and introduce this cargo into these cells such that it can exert its biological activity, is a major challenge in biomedical research. Tremendous efforts have already been spent in the development and testing of systems based on liposomes, microspheres, antibodies etc. for delivery of drugs, genes, peptides and proteins. Though many of these approaches are promising, the actual successes so far are limited. The genome of a VLP as provided herein has been deleted and/or rearranged to such an extent without abolishing replication that it serves very well as delivery vehicle for said cargo. Providing said VLP with cargo or using the VLP as a vector is e.g. done by inserting a foreign gene sequence that encodes a desired molecule such as a ligand or binding molecule, whether this is an immunogen or receptor binding molecule or any other protein or peptide. In a preferred example said cargo comprises a nucleic acid into which foreign genes or sequences of interest have been incorporated. Foreign genes can be expressed by a VLP both by the additional insertion of such a gene in its genome or by using the genetic space created by deletion of nonessential genes.

In one embodiment the invention provides a replicative VLP according to the claims which is also modified at the ectodomain and/or the endodomain of a viral protein. For example, by modifying the ectodomain of the spike protein, the VLP is provided with modified biological molecules as targeting means that serve to direct the VLP to interact with other biological molecules that mirror or can interact with the target means, such as receptor proteins on cells, be it hormone receptors, specific immunoglobulines on B-cells, MHC and MHC associated molecules present on T-cells and other cells, transfer proteins or other receptor molecules known to the person skilled in the field of cell surface receptors. The targeting means can also be provided to interact with known binding sites of selected enzymes on proteins or other molecules that serve as substrate for the selected enzyme.

In a further example, replicative VLPs are provided exposing an immunogenic determinant, such as a bacterial toxin or a heterologous viral or bacterial protein comprising a relevant antigenic determinant specific for a pathogen. This is an example whereby the VLPs serve as immunogen or vaccine, here for example directed against the bacterial toxin. B-lymphocytes carrying the corresponding immunoglobuline at their surface are in this case the target cells for the VLPs, once recognized by the B-lymphocyte, this cell(s) will multiply and produce the appropriate antibody.

Preparation of VLPs or coronaviruses with modified spikes can be achieved genetically by modification of the viral genome such that it expresses the modified S protein in infected cells. Here we also provide the preparation of coronaviruses containing altered spikes in a different way by expressing modified S genes in cells which are in addition infected with coronavirus. The co-incorporation of the mutant spike provides the virus with new targeting means.

The viruses of group I, with the feline infectious peritonitis virus (FIPV) probably as the most complex member, have their typical genes located between genes S and E and downstream of the N-gene, i.e. between N-gene and 3' UTR (untranslated region). Group II viruses, to which the mouse hepatitis viruses (MHV) belong, have their particular genes between the polymerase and S genes and between the genes for the S and E proteins. One of the encoded proteins characteristic for this group is the hemagglutinin-esterase (HE) protein, which is incorporated into virions and of which the hemagglutinating and esterase activities have been demonstrated. HE activities are not essential and their significance remains to be elucidated. Finally, also the group III viruses, with the prototype coronavirus infectious bronchitis virus (IBV) as the representative, have sequences between the S and E genes but also between the M and N genes. While for some of the group-specific genes no expression products could be detected in infected cells while for others (e.g. the HE gene in MHV) naturally occurring viral mutants carrying deletions in these genes have been observed, the possibility exists that for some of these genes not the protein products but other gene products, such as the nucleotide sequences (DNA or RNA) *per se*, are important or essential.

In another embodiment, for groups I, II, or III, the invention provides a virus-like particle capable of replication, said particle derived from a coronavirus wherein the genes for the structural proteins do not occur in the order 5'-S-E-M-N-3'. Such a replicative VLP with rearranged gene order has two important features. One is safety, resulting from the fact that homologous recombination of the VLP genome with that of an accidental field virus is unlikely to generate viable new progeny. The other is attenuation due to the shuffling of the genes. Such a replicative VLP with rearranged gene order provides well attenuated VLP's for vaccine or gene delivery use, and is herein provided bearing the deletions from the non-essential genes as well. It is shown herein that changes in the so-called invariable order of the genes specifying the polymerase functions (ORF1a/1b) and the structural proteins S, E, M, and N in the coronaviral genome is surprisingly well tolerated, for example VLP's with gene order S, M, E, N, or E, S, M, N are easily obtained. Also here, foreign genes can be inserted at different positions in the viral genome, either as an additional gene or replacing deleted non-essential genes; these genes are expressed and are stably maintained during passage of the virus. Gene rearrangement had been shown for nonsegmented negative strand RNA viruses (Wertz, 1998).

In another example, the disclosure provides a recombinant VLP wherein nucleic acid encoding the S-protein has been modified or at least partly deleted. The S protein of these viruses is responsible for binding to the cell receptor and for subsequent fusion of viral and cellular membrane during entry. These two functions occur in separate regions of the molecule: receptor binding in the amino-terminal and fusion in the carboxy-terminal part. Therefore, by replacing (parts of) the receptor binding domain by biological molecules with different targeting specificities coronaviruses can be directed to interact with a wide variety of target molecules that are for instance expressed on the surface of cells. Doing so without affecting the fusion function of the S-protein, the VLP according to the invention can fuse with or penetrate into cells not normally infectable by the original virus.

Provided by the disclosure are virus-like particles (VLPs) derived from coronaviruses in which one or more copies of the viral membrane proteins have been modified so as to contain foreign protein moieties of viral (either coronaviral or noncoronaviral) or non-viral origin, which moieties either are replacing part(s) of the VLP membrane proteins or are incorporated within these membrane proteins thereby constituting an integral part of them. By this the VLP is provided with novel biological properties such as new targeting means, or immunological information, or proteins with specific biological activity contained within the virus-like particle, which biological properties are associated with the VLP next to or in place of the natural spike protein of the original coronavirus.

In one example, recombinant VLPs with deleted and/or rearranged genome are provided in which (a part of) the ectodomain (i.e. the part exposed at the outside of the viral particle) of the spike protein has been replaced by the corresponding domain (or part thereof) of the spike protein of another corona virus. Hereby the VLP has acquired another cell substrate specificity whereby the VPL is capable of entering cells otherwise not accessible or susceptible to the original corona virus. In a further example, VLPs are provided which are composed of the mouse hepatitis coronavirus (MHV) M and E proteins and which contain chimaeric spike molecules consisting of the transmembrane + carboxy-terminal domain of MHV S but the ectodomain of the spike protein of feline infectious peritonitis coronavirus (FIPV). These VLPs can now enter feline cells and deliver MHV-like particles. Particles with these chimaeric spikes are produced by making constructs of the corona virus MHV S gene in which the region encoding the amino-terminal domain is replaced by the corresponding domain of FIPV. These constructs are inserted into plasmids behind a bacteriophage T7 polymerase promoter. The constructs are then co-transfected with plasmids carrying the MHV M and E genes, both also behind the T7 promotor, in OST-7 cells which have been infected with a recombinant vaccina virus expressing the T7 polymerase. The resulting VLPs contain the chimaeric MHV/FIPV S protein. In another example, the VLP is provided by the methods used as above with ectodomains of the spike protein of infectious bronchitis coronavirus (IBV), or the ectodomain (or part thereof) of an envelope protein of any enveloped virus not belonging to the coronaviruses. For example, MHV-based VLPs are provided by the invention which carry at their surface the ectodomain of the pseudorabies virus (PRV) glycoprotein gD instead of the MHV spike ectodomain or the luminal (i.e. amino-terminal) domain (or part thereof) of any nonviral type I membrane protein. In this way VLPs are provided that have a cell specificity for chicken cells, or pig cells, or cells reactive with the type I membrane protein.

In yet another example, replicative VLPs are produced with modifications that are contained within the particles. This is achieved by the incorporation of modified constructs of any of the corona viral proteins S, M, E and HE. In corona virus particles these proteins have their carboxy-terminal domain enclosed within the interior of the viral envelope. Thus, foreign protein sequences incorporated within, appended to or replacing the carboxy-terminal domain are enclosed as well. In this way, VLPs can be provided that contain protein moieties, or fragments thereof, from another virus, or non-viral proteins such as hormones, such as erythropoietin. This allows the production of VLPs containing a biological active protein or fragments thereof, which is/are shielded by the viral envelope and can be released and/or retrieved later, when the viral membrane is degraded or fused with another membrane. This allows the *in vitro* production in cells, or the *in vivo* production in secretory glands such as milk glands of biologically active substance which are otherwise harmful or toxic to the producing cells, or which for other reasons need to be produced in a shielded form.

As another example, MHV-based VLPs are provided carrying on their surface or inside an enzymatically active molecule like furin, or a cytokine, or a hormone receptor, or another viral or nonviral polypeptide with biological activity. In these examples, VLPs are provided with (additional) targeting means that serve to direct the VLP to cells otherwise not accessible to the original corona virus. The disclosure provides recombinantly obtained replicative VLPs which are further modified at the ectodomain and/or the ectodomain of any of the viral proteins. By modifying the ectodomain of the spike protein, the VLPs are provided with modified biological molecules as targeting means that serve to direct the VLP to interact with other biological molecules that mirror or can interact with the target means, such as receptor proteins on cells, be it hormone receptors, specific immunoglobulines on B-cells, MHC and MHC associated molecules present on T-cells and other cells, transfer proteins or other receptor molecules known to the person skilled in the field of cell surface receptors. The targeting means can also be provided to interact with known binding sites of selected enzymes on proteins or other molecules that serve as substrate for the selected enzyme.

Preparation of VLPs or coronaviruses with modified spikes can be achieved genetically by modification of the viral genome such that it expresses the modified S protein in infected cells. Here we also provide the preparation of coronaviruses containing altered spikes in a different way by expressing modified S genes in cells which are in addition infected with coronavirus. The co-incorporation of the mutant spike provides the virus with new targeting means. As an example we demonstrate the production of MHV particles containing the chimaeric MHV/FIPV S protein. The chimaeric S gene construct is expressed in L cells which are subsequently infected with wild-type MHV strain A59 (MHV-A59) or a mutant thereof. The progeny virus released by the cells contains the modified S protein. To demonstrate the altered targeting the virus was used to infect feline cells which are naturally not susceptible to MHV. The cells are now infected as shown by immunofluorescence and produce normal MHV. As another example we demonstrate the production of MHV containing chimaeric S proteins in which part of the S ectodomain has been replaced by the corresponding part (i.e. the luminal or amino-terminal domain) of the human CD4 molecule, as an example of a nonviral protein. These modified coronaviruses have acquired the property to infect HIV-infected cells and cells expressing HIV envelope glycoprotein through the specific recognition of the CD4 and HIV gp 120 complex. As a result, the HIV-infected cells will undergo a lytic infection, effectively reducing the number of HIV-infected cells in the body and thereby reducing the severity of the disease or even terminating the infection.

As another example, we demonstrate the production of a VLP according to the invention containing spike molecules of which the amino-terminal part has been replaced by a single chain-antibody fragment recognizing a specific cell surface protein that is expressed on cells that can normally not be infected with the coronavirus laying at the basis of said VLP. The modified virus is able to infect these otherwise refractory cells. This example illustrates the principle that in this way, i.e. by inserting very specific targeting information into the viral spike, corona viruses can be directed to selected cells or tissues. The single chain-antibody fragment can for instance be selected in phage-display systems, or in other clonal selection systems of single-chain antibody fragments known in the field.

Another aspect of the present disclosure relates to the use of said replicative VLPs or coronaviruses as gene delivery vehicles. This can be achieved in different ways. One way is by incorporating foreign genes or sequences into the viral genome such that upon entry of the virus into cells these genes are expressed or that the inserted sequences become otherwise biologically active (as is the case with ribozymes or antisense RNAs generated by the virus within the cells). The other way uses VLPs to package foreign RNA into particles by making use of the coronaviral packaging signal(s). Incorporating foreign sequences into the coronaviral genome can be accomplished by genetic manipulation using an infectious (c-)DNA clone, a full-size DNA copy of the viral genome. It can also be achieved by RNA recombination in which case RNA representing part of the viral genome and containing the foreign sequences is introduced in infected cells allowing the foreign sequences to be incorporated through homologous recombination. Because coronaviruses will usually kill the cells they infect, it is important for most purposes to attenuate them so that they will not kill the cells with which they interact. Attenuation is here accomplished by genomically altering the virus through deletion or rearrangement.

As an example attenuation is provided by the preparation of an MHV mutant from which an essential gene has been deleted by recombination. A mouse cell line is provided in which the MHV E gene has been chromosomally integrated allowing the E protein to be produced by the expression of the gene. MHV lacking an E gene has been produced in normal mouse cells by recombination using a synthetic RNA containing a perfect copy of the MHV genomic 3'-end except for the lack of an intact E gene. The E-defective virus is able to grow only in the cells complementing the defect. The virus produced is attenuated such that it can infect other mouse cells, but non-productively: the lack of an E protein prevents the assembly of progeny. As an example of the principle of incorporating foreign genetic sequences into attenuated or not-attenuated VLPs or coronaviruses and of their expression is the following provided by the invention. An MHV derived VLP is provided into which a reporter gene such as LacZ or green fluorescent protein has been recombined and one in which the chimaeric MHV/FIPV S gene has been incorporated. The expression of the genes is shown by blue or green-fluorescent staining of VLP infected cells and by the acquired ability to infect feline cells, respectively. The other way to obtain coronavirus-based delivery vehicles uses VLPs comprising foreign RNA sequences. Incorporation of foreign RNA sequences into these particles requires their packaging into nucleocapsids. Viral RNA-packaging by nucleocapsid (N) protein molecules occurs by the recognition of specific sequences, packaging signal(s) by the N protein. In MHV the packaging signal includes a 69 nucleotides long region in gene 1B. Foreign (noncoronaviral) RNAs containing the coronavirus packaging signal(s), or defective coronaviral genomes in which these signal(s) have been retained but into which foreign sequences have been incorporated, are assembled into VLPs when introduced into cells expressing the N, M and E (±S) genes. The VLP can introduce into a target cell a defined RNA that may have one of several functions. An example provided is a RNA acting as mRNA and specifying a particular protein such as a toxin or an inducer of apoptosis or an antibody fragment. Another example is an antisense RNA or an RNA with ribozyme activity. For most purposes it is essential to acquire multiple copies of the RNA in each cell to obtain the desired effect. This may not be feasible with VLPs which will only carry one or a few pseudo-NC. The invention thus provides the RNAs with amplification signals such that they will be multiplied in the target cell. To achieve this goal, Semliki Forest virus (SFV) replication sequences are used as the basis of the RNA construct. SFV-derived mRNA further comprising the coronavirus encapsidation sequences and specifying a reporter protein are assembled into VLPs. The SFV-driven amplification allows synthesis of the reporter protein in cells; in animals the appearance of antibodies to the reporter protein testifies to the productive delivery of the VLPs' content. The disclosure also provides a VLP which is an antigen or epitope delivery vehicle meant for the induction of specific immune responses, cellular and/or humoral, systemic and/or local, including the induction and production of specific antibodies against proteins, to achieve protection against infection by pathogens, of viral and nonviral origin. As an example the disclosure provides the induction of antibodies against the reporter protein derived from SFV-derived mRNA further comprising the coronavirus encapsidation sequences and specifying a reporter protein, as described above. As another example the induction of antibodies is demonstrated in mice to the FIPV spike and to PRV gD by immunization with the VLPs, also described above. Thus immune responses can be elicited both against proteins which are encoded by the altered genome of the VLP and/or against proteins which have been incorporated as targeting means in the VLP, thereby partly or wholly replacing the original spike protein. The examples illustrate the applicability of the approach for the induction of immune responses against proteins as diverse as for instance viral, bacterial, parasitic, cellular and hormonal origins.

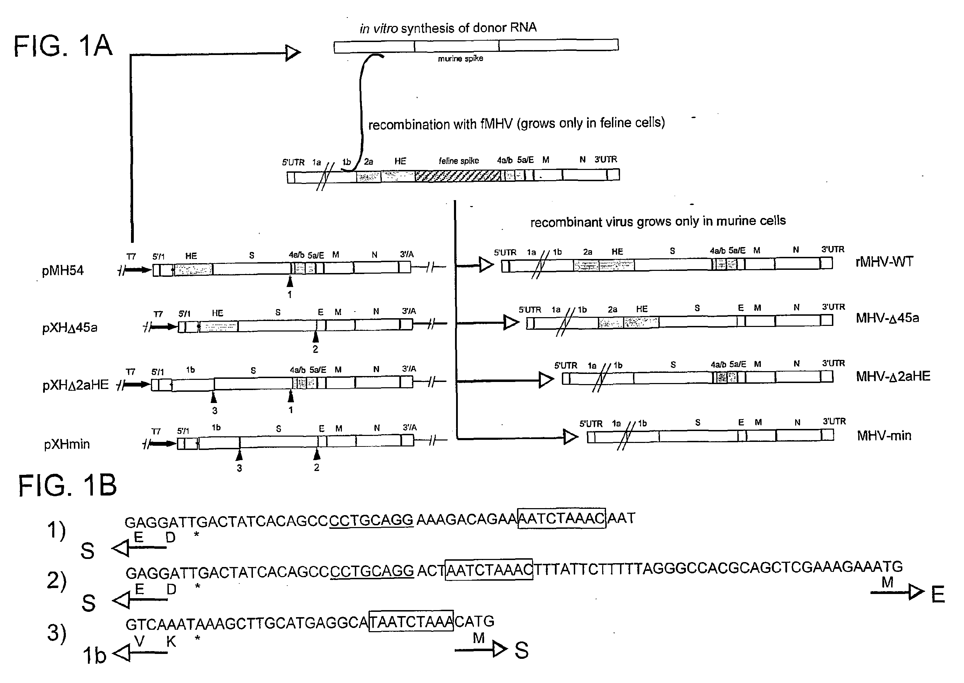

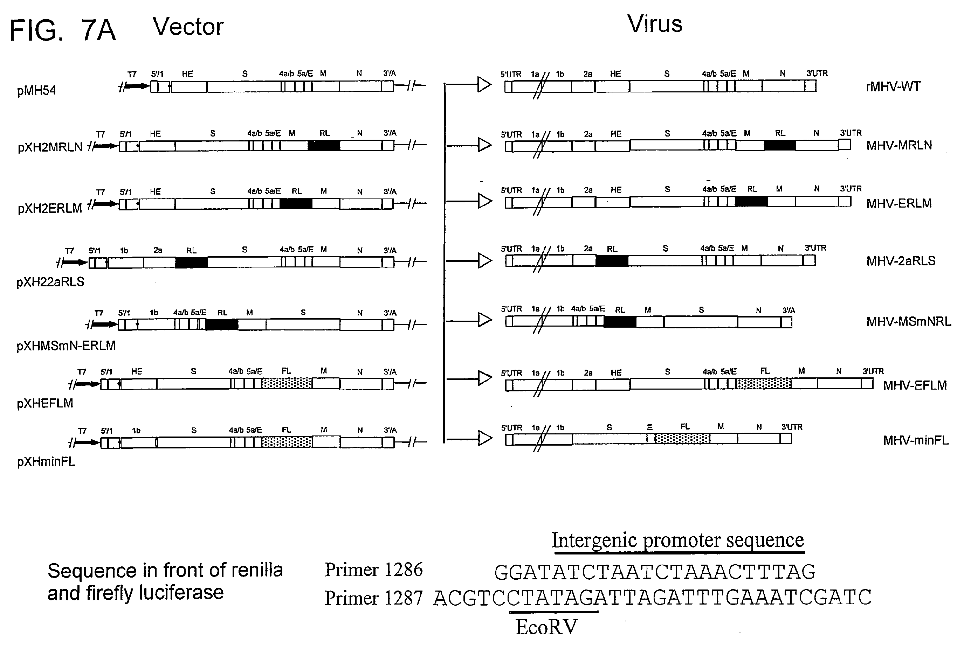

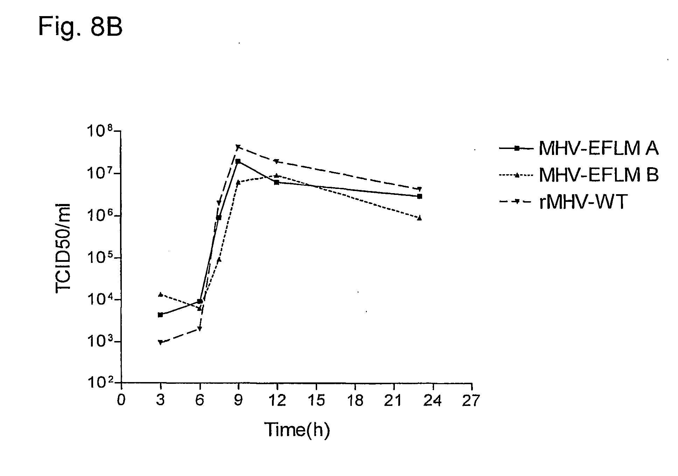

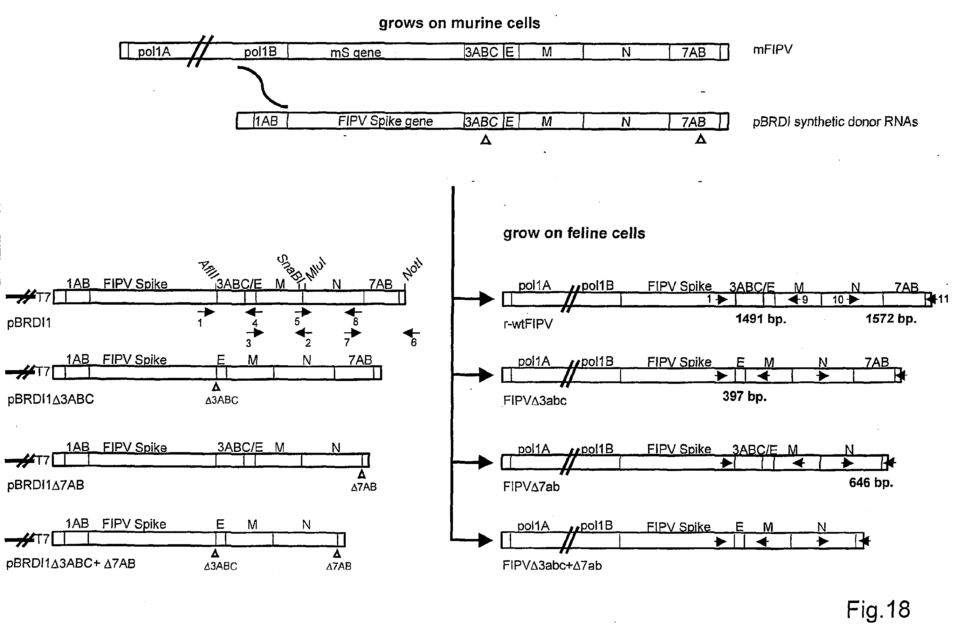

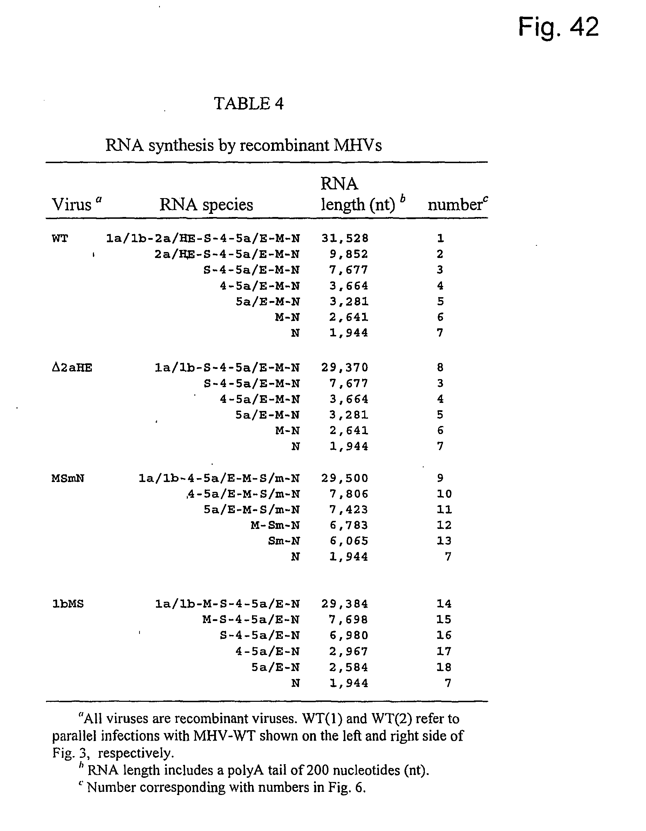

The disclosure also provides VLPs which have fully maintained the original spike protein but which are altered genomically to attenuate the VLP and/or to encode nucleotide sequences that need to be delivered at the cells to which the original coronavirus was targeted. For example, in this way, intestinal epithelial cells, or respiratory epithelial cells, that are normally infected by TGEV, or PRCV, respectively, can now interact with VLPs derived from TGEV or PRCV, or other cell-specific coronaviruses if needed, to express proteins normally not expressed by said viruses. In this way, respiratory epithelial cells of cystic fibrosis patients can for instance be induced to express lung surfactant molecules that are encoded by the altered genome of the VLP. Various examples are provided in the detailed description.
Fig. 1:
   A. The top part shows the principle of the RNA recombination technology. Feline cells are infected with fMHV (the MHV-derivative carrying a chimeric S protein with an ectodomain from the FIPV S protein allowing the virus to grow in feline cells) and transfected with synthetic donor RNA that carries, among others, the intact MHV S gene. Proper RNA recombination leads to viruses that have regained the ability to grow in murine cells by the acquisition of the cognate spikes. The lower part shows, on the left, the schematic representation of the relevant parts of the plasmid constructs from which donor RNAs were generated. On the right the genomic organization of the recombinant viruses generated with these donor RNAs are depicted.
   B. The nucleotide sequences are shown of the new junctions created in the plasmid constructs (and the resulting viruses), the positions and numbers of which are indicated in part A (left side).
Fig. 2:
   RT-PCR analysis of recombinant viruses with genetic deletions. Genomic RNA was isolated from cloned virus, cDNA was prepared by RT with proper primers (1092 and 1127) and PCR was done with primers 1261 + 990 or with primers 1173 + 1260 to analyze the region of genes 4a/b/5a or of genes 2a + HE, respectively. The outline of the analyses is shown at the right, the results of the agarose gel analyses of the PCR products is shown at the left. The viruses analyzed are shown above the gel. In the right lane marker DNAs were run.
Fig. 3:
   Viral RNAs synthesized in infected cells by the different MHV deletion mutants were analyzed by extraction of total cytoplasmic RNA, metabolically labeled with [³³P]orthophosphate in the presence of actinomycin D, followed by electrophoresis in 1% agarose gel. The genetic make-up of the deletion viruses is shown at the top while the subgenomic RNA species are designated at the right also according to their genetic composition.
Fig. 4:
   One-step growth curves of the recombinant deletion viruses. After high-m.o.i. infection of LR7 cells with the deletion viruses and MHV-WT samples were taken from each culture medium at different times and the infectivity in these samples was analyzed by titration.
Fig. 5:
   As in Fig. 1 the construction of viruses with rearranged gene order is depicted. On the left the relevant parts of plasmid constructs: the genome organization of their MHV cDNA sequences and the designations of the plasmids. On the right the respective viruses with their genome structure and their names.
Fig. 6:
   One-step growth curves of the recombinant viruses with rearranged genome. After high-m.o.i. infection of LR7 cells with the viruses MHV-Ä2aHE (DELTA2aHE), MHV-1bMS, MHV-MSmN, or MHV-WT samples were taken from each culture medium at different times and the infectivity in these samples was analyzed by titration.
Fig 7:
   A. As in Fig. 1 the construction of viruses with foreign gene insertions is depicted. At the left the various plasmid (vector) constructs , with their names, are depicted. At the right, the genetic make-up of viruses obtained with these plasmids by RNA recombination in feline cells are shown together with their names. Below: the primer sequences (and numbers) used for the introduction of an intergenic promoter sequence (IGS) in front of the renilla (RL) and firefly luciferase (FL) gene.
   B. RT-PCR analysis of recombinant viruses carrying the RL gene. cDNA was prepared using primer 1412 by RT on viral RNA; for each virus two independently derived viruses (designated by the extensions A1A and B1A; A2 and B1; A7A and B2D) were analyzed in parallel. Subsequently, PCR was carried out on the resulting cDNA as well as on the plasmids used to generate the viruses (see Fig. 7A) with the primer pairs indicated at the bottom of the figure. For each set a negative control sample (H₂O) was also included. The agarose gel analysis of the PCR fragments together with DNA markers (flanking lanes) are shown and the sizes of the products are indicated at the right.
Fig. 8:
   One-step growth curves of the recombinant viruses carrying foreign genes. After high-m.o.i. infection of LR7 cells with viruses, samples were taken from each culture medium at different times and the infectivity in these samples was analyzed by titration.
   A. Growth curves of different viruses having the renilla luciferase gene as compared to MHV-WT.
   B. The growth of two independently obtained clones of MHV-EFLM virus carrying the firefly luciferase gene is shown as compared to MHV-WT.
Fig. 9:
   Expression of luciferase by recombinant viruses. LR7 cells were infected with viruses expressing the renilla (A) or firefly (B) luciferase and with MHV-WT, and the luciferase activity generated in the cells was monitored over time. In B the two independently obtained clones of MHV-EFLM virus (see Fig. 8B) are compared to MHV-WT.
Fig. 10:
   Stability of viruses carrying foreign genes. The recombinant viruses MHV-ERLM and MHV-EFLM (two independently obtained clones in each case) were passaged 8 times over LR7 cells at low m.o.i. After each passage the viral infectivity (TCID50) in the harvested culture medium was determined. The collection of viruses thus obtained was inoculated in parallel into LR7 cells at m.o.i. of 5 and the luciferase expression in the cells at 8 hr post-infection was quantified. Results are plotted as a function of passage number.
Fig. 11:
   Inhibition of MHV infection by the HR2 peptide. LR7 cells were inoculated with the virus MHV-EFLM in the presence of different concentrations of HR2 peptide or, as a control, a peptide corresponding to amino acids 1003-1048 of the viral S protein. After one hour of inoculation cells were washed and incubated further in the absence of peptide. To evaluate the success of the infection cells were analyzed at 4 h p.i. for luciferase expression. In the figure luciferase activity is plotted against the different peptide concentrations used.
Fig 12:
   Inhibition of cell-cell fusion by HR2 peptide. After inoculation of parallel cultures of LR7 cells with MHV-A59 cells were overlaid with agar medium containing different concentrations of HR2 peptide and plaques were counted the following day.
Fig. 13:
   A. The genetic structure of the primary plasmid vector pBRDI1 as compared to that of the parental virus from which it was derived. The top part shows the genome organization of FIPV strain 79-1146. The dotted parts (i.e. the very 5'-most 702 bases and the 3-derived 9,2,62 bases) were assembled into the cDNA construct of pBRDI1; in this plasmid the viral cDNA is preceded by a phage T7 promoter sequence and the insert is flanked by *XhoI* (5') and *NotI* (3') restriction sites.
   B. Plasmid pBRDI2 is a derivative of pBRDI1 in which the FIPV S gene has been replaced by a chimaeric S gene (designated mS) which encodes the MHV-A59 S ectodomain while having retained the sequences for the FIPV S protein transmembrane and endodomain. Plasmid pBRDI2 was obtained by substituting in pBRDI1 the *SacI-AflII* segment by the corresponding fragment of pTMFS1. The latter plasmid is a derivative of plasmid pTMFS (12) which contains the chimaeric gene sequence and into which a *SacI-StuI* fragment was cloned to extend the MHV S sequence at its 5' end with sequences corresponding to the FIPV pol1B 3' end.
Fig. 14:
   Sequence details of the pBRDI constructs.
   A. The nucleotide sequence of pBRDI1 and pBRDI2 around the very 5' end of the FIPV genome sequence: the *XhoI* site and the phage T7 sequence are followed by a G-triplet and subsequently by the FIPV sequence.
   B. The sequence at the pol1A/pol1B junction.
   C. The nucleotide sequence at the very 3' end of the cDNA construct. The 3' untranslated region (3'UTR) is followed by a stretch of adenine nucleotides and a *NotI* sequence.
   D. Nucleotide sequence at the FIPV pol1B- MHV S transition in pTMFS1 and pBRDI2.
Fig. 15:
   Schematic picture of the generation of mFIPV by the recombination of FIPV genomic RNA and pBRDI2 derived synthetic RNA in feline cells. The genetic organisation in each RNA is indicated as well as the selection for recombinant (i.e. chimaeric mS containing) virus by growth on murine cells.
Fig. 16:
   Analysis of the mFIPV structural proteins. Murine LR7 cells were infected with mFIPV and, for comparison, with MHV-A59; feline FCWF cells were infected with FIPV strain 79-1146. Infected cells were labeled with ³⁵S-amino acids from 5-7h p.i., cell lysates were prepared and immunoprecipitations were carried out with different antibodies: polyclonal antibodies against FIPV (lanes 1, 6, and 10) and MHV (lanes 3, 4, 8 and 12) and monoclonal antibodies against the feline S protein (S_{f}; lanes 2, 7, and 11) and the murine S protein (Sm; lanes 5, 9, and 13). The proteins were analyzed by electrophoresis in SDS-12% polyacrylamide gel. The position in the gel of the MHV and FIPV proteins are indicated at the left and right side, respectively. The mFIPV S protein is precipitated by the MHV S specific sera, not by those precipitating the FIPV S protein, and its migration in gel is similar to that of the MHV S protein.
Fig. 17:
   One-step growth curves of mFIPV as compared to MHV. After high-m.o.i. infection of LR7 cells with the two viruses samples were taken from each culture medium at different times and the infectivity in these samples was analyzed by titration.
Fig. 18:
   Generation of FIPV deletion mutants. At the top the principle of the method is shown: recombination of the mFIPV RNA with synthetic pBRDI derived donor RNA carrying the intact FIPV S gene. Below this are depicted the genetic make-up of the constructed pBRDI1 plasmids (left) and of the generated viruses (right). Arrows indicate the position (and number) of the primers used, open triangles indicate deletions. At the right, numbers of base pairs (bp) indicate the sizes of PCR fragments predicted to be obtained with the indicated primer pairs.
Fig. 19:
   Genetic analysis of the recombinant FIPV deletion viruses. Genomic RNA was isolated from the deletion viruses as well as from recombinant wild-type FIPV. RT and PCR reactions were carried out using the primers 1 and 9 for the analysis of the genes 3ABC region (panel A) and primers 10 and 11 for the genes 7AB (panel B). The positions of the DNA fragments in the agarose gels are indicated alongside the gel together with their predicted size (c.f. Fig. 18, right). The viral RNAs analyzed in the different lanes are indicated at the right.
Fig. 20:
   Heptad repeat (HR) regions and their amino acid sequences in coronavirus spike proteins.
   A. Schematic representation of the coronavirus MHV-A59 spike structure. The spike (S) glycoprotein contains an N-terminal signal sequence (SS) and a transmembrane domain (TM) close to its C-terminus. S is proteolytically cleaved (arrow) in an S1 and S2 subunit, which are non-covalently linked. S2 contains two conserved heptad repeat regions, HR1 and HR2, as indicated.
   B. Sequence alignment of HR1 and HR2 domains of MHV-A59, HCV-OC43 (human coronavirus strain OC43), HCV-229E (human coronavirus strain 229E), FIPV and IBV (infectious bronchitis virus strain Beaudette). The alignment shows a remarkable insertion of exactly 2 heptad repeats (14 aa) in both HR1 and HR2 of HCV-229E and FIPV, which is present in S proteins of all group 1 viruses. The predicted hydrophobic heptad repeat a and d residues are indicated above the sequence. The frame shift of predicted heptad repeats in HR1 is caused by a stutter. Asteriks denote conserved residues. The amino acid sequences of the peptides HR1, HR1a, HR1b, HR1c and HR2 used in this study are presented in italics below the alignments. N-terminal residues derived from proteolytic cleavage site of the GST-fusion protein are between brackets.

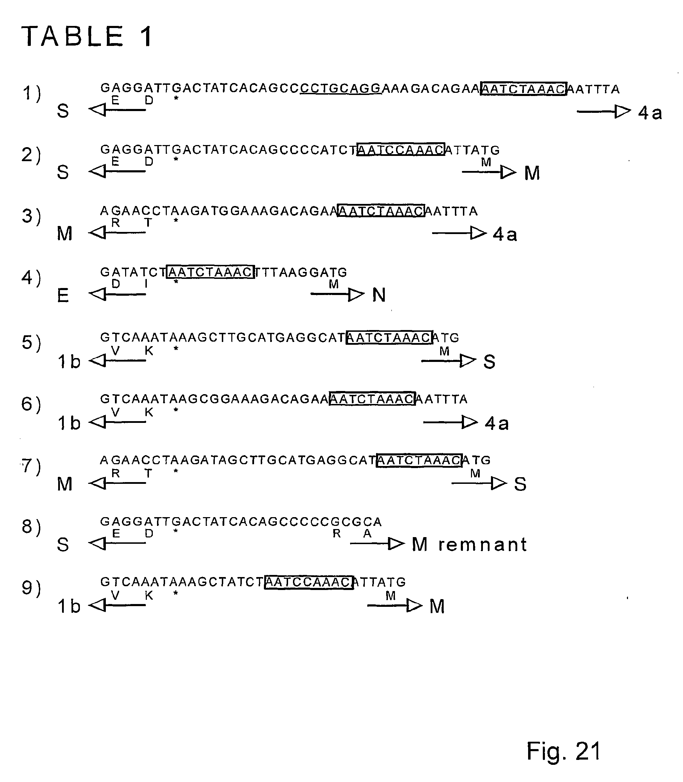

Fig. 21 = Table 1:
   The sequences are shown of the junctions that were generated in the plasmids depicted in Fig. 5 and in the viruses obtained with these plasmids. The numbers correspond to the numbering of the junctions as indicated by arrowheads in the plasmids (Fig. 5, left).

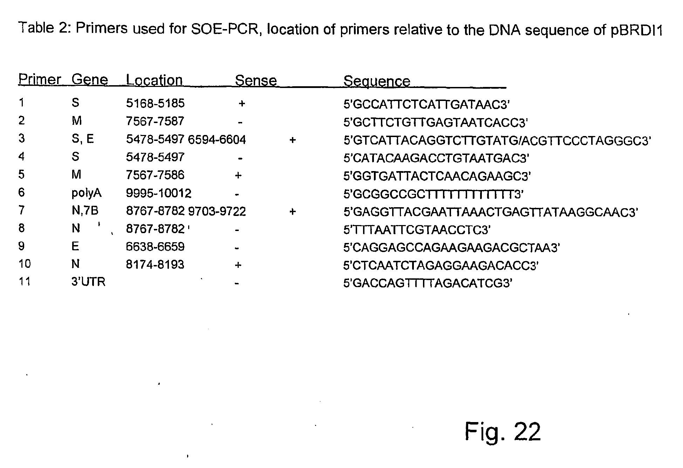

Fig. 22 = Table 2:
   Primers used for splicing overlap extension (SOE)-PCR and for RT-PCR in the construction and analysis of recombinant FIPVs. For their position on the FIPV genome: see Fig. 18, in which their numbers and sense are indicated by arrows. The numbering of the primers refers to that in the pBRDI1 sequence.

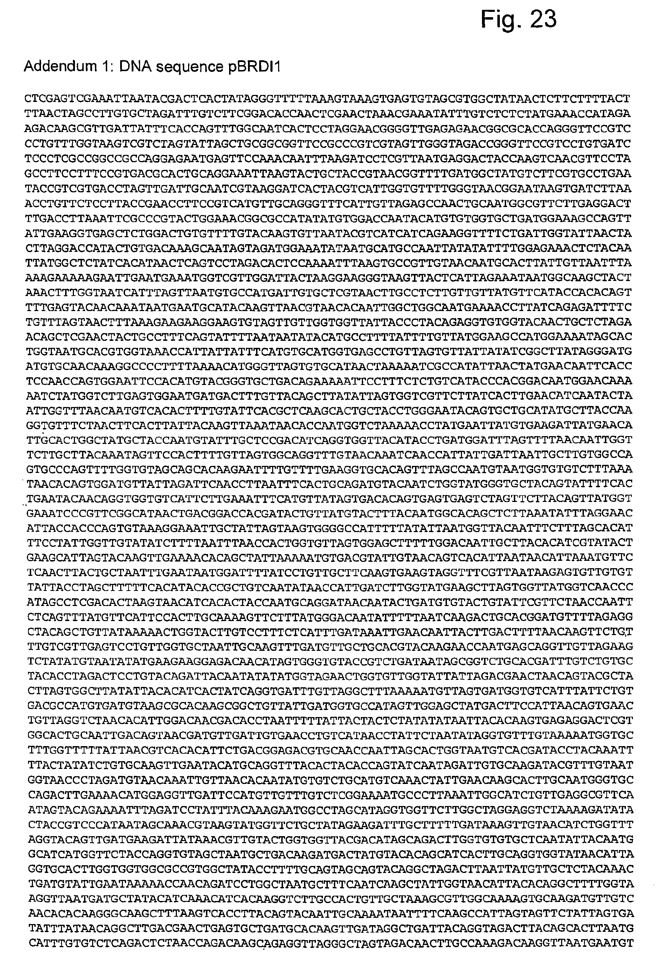

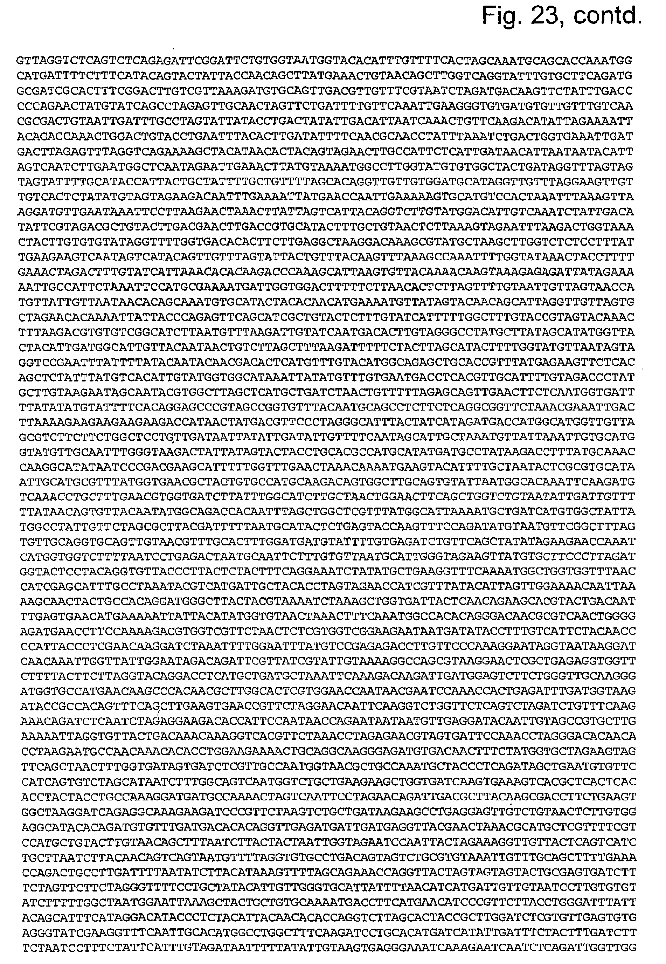

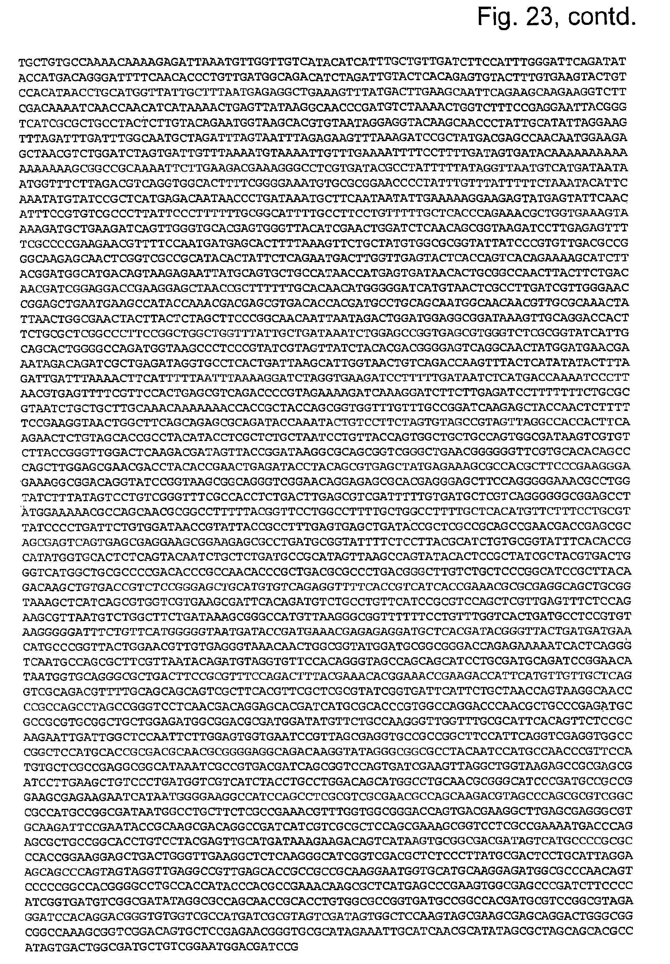

Fig. 23 = Addendum 1:
   Nucleotide sequence of plasmid pBRDI1. The sequence starts with the *XhoI* site (ctcgag) followed by the phage T7 polymerase promoter sequence and the triple G sequence, after which it proceeds with the 5' FIPV cDNA sequence.

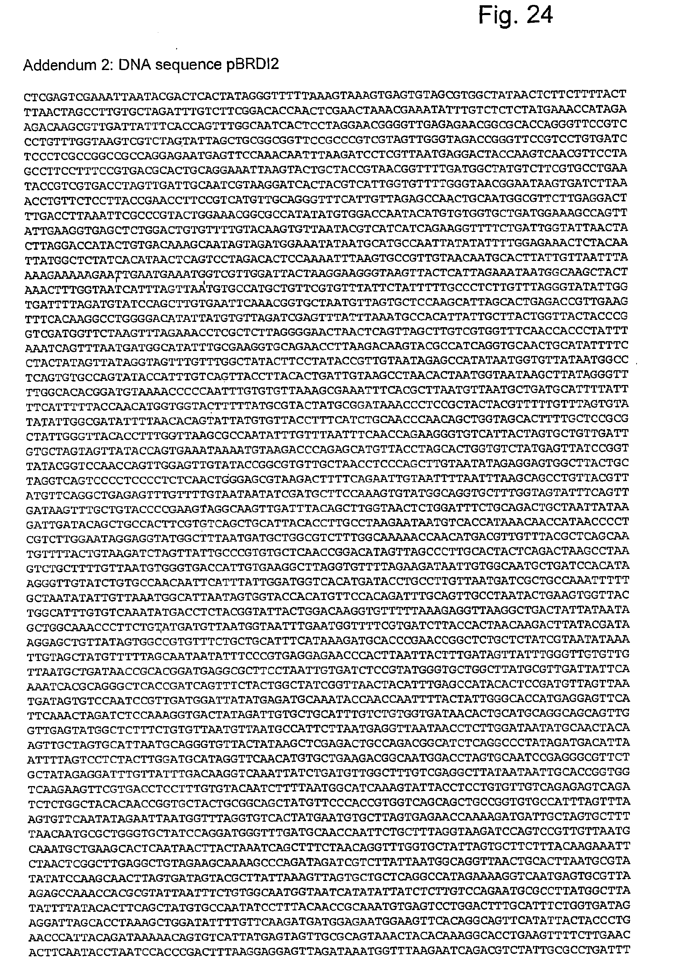

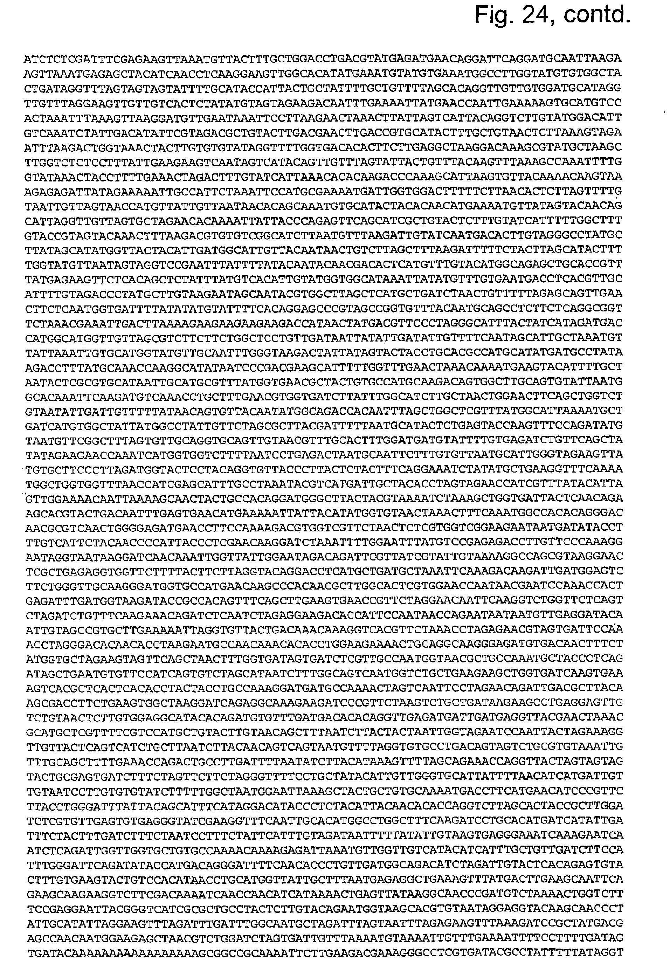

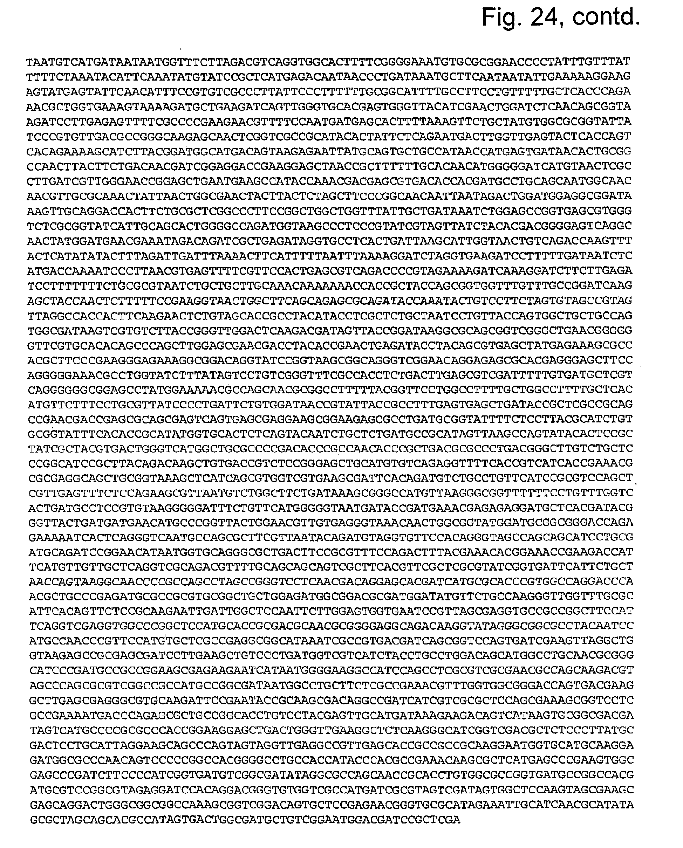

Fig. 24 = Addendum 2:
   Nucleotide sequence of plasmid pBRDI2. The sequence starts with the *XhoI* site (ctcgag) followed by the phage T7 polymerase promoter sequence and the triple G sequence, after which it proceeds with the 5' FIPV cDNA sequence.
Fig. 25:
   Survival of C57B1/6 mice infected with MHV recombinants. Four-week-old mice were inoculated intracranially with various dilutions of recombinant wild type and deletion viruses (n=5 per virus) and survival was monitored. The data for mice infected with 2.5x10⁵ PFU are shown. While the animals infected with MHV-WT had all succumbed by day 7 post-infection, all mice inoculated with the deletion mutant viruses survived until 21 days post-infection.
Fig. 26A:
   Infected 17Cl1 cells were metabolically labeled with [³³P]orthophosphate in the presence of actinomycin D essentially as described (4, 10). Samples of total cytoplasmic RNA, purified using Ultraspec reagent (Biotecx), were denatured with formaldehyde and formamide, separated by electrophoresis through 1% agarose containing formaldehyde, and visualized by fluorography. The different RNA species are indicated by numbers corresponding with Table 4 (=Fig 42).
Fig. 26B:
   Mice inoculated intraperitoneally with 1x10⁶TCID⁵⁰ of each MHV recombinant were euthanized at day 4 post-infection and the viral replication in the liver was determined by quantitative plaque assays.
Fig. 27:
   RT-PCR analysis of recombinant viruses MHV-EFLM and MHV-minFL.cDNA was prepared using primer 1475 by RT on viral RNA; for each virus two independently derived viruses (designated by the extensions A1A and B1A, A6F and B4A) were analyzed in parallel. Subsequently, PCR was carried out on the resulting cDNA as well as on the plasmids used to generate the viruses (see Fig. 7A) with the primer pair 935 and 1474. The agarose gel analysis of the PCR fragments together with a DNA marker is shown.
Fig. 28:
   RT-PCR analysis of recombinant viruses MHV-EFLM and MHVminFL. CDNA was prepared using primer 1412 by RT on viral RNA; for each virus two independently derived viruses (designated by the extensions A2E and B4D) were analyzed in parallel. Subsequently, PCR was carried out on the resulting cDNA as well as on the plasmids used to generate the viruses (see Fig. 7A) with the primer pairs indicated at the bottom of the figure. The agarose gel analysis of the PCR fragments together with a DNA marker is shown.
Fig. 29-31:
   RNA synthesis by recombinant MHVs. The genomes of the recombinant viruses are depicted at the top, their observed RNA expression patterns are shown below. Infected 17Cl1 cells were metabolically labeled with [³³P]orthophosphate in the presence of actinomycin D essentially as described (4, 10). Samples of total cytoplasmic RNA, purified using Ultraspec reagent (Biotecx), were denatured with formaldehyde and formamide, separated by electrophoresis through 1% agarose containing formaldehyde, and visualized by fluorography. Note that the subgenomic RNA species in this figure are designated by their composition, rather than as RNA2 through RNA7, since the numerical designations would be ambiguous for the mutants.

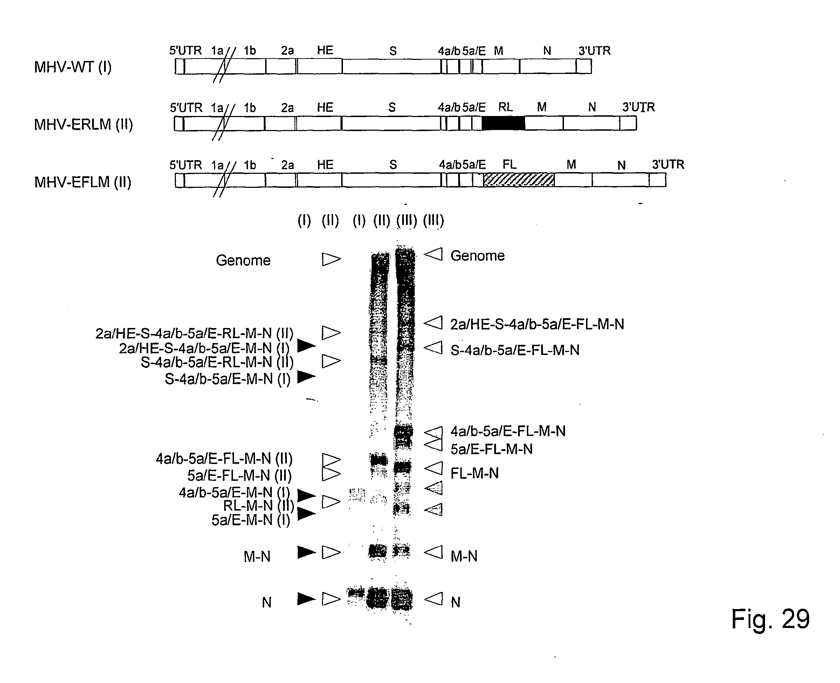

   Fig. 29: MHV-WT, MHV-ERLM and MHV-EFLM

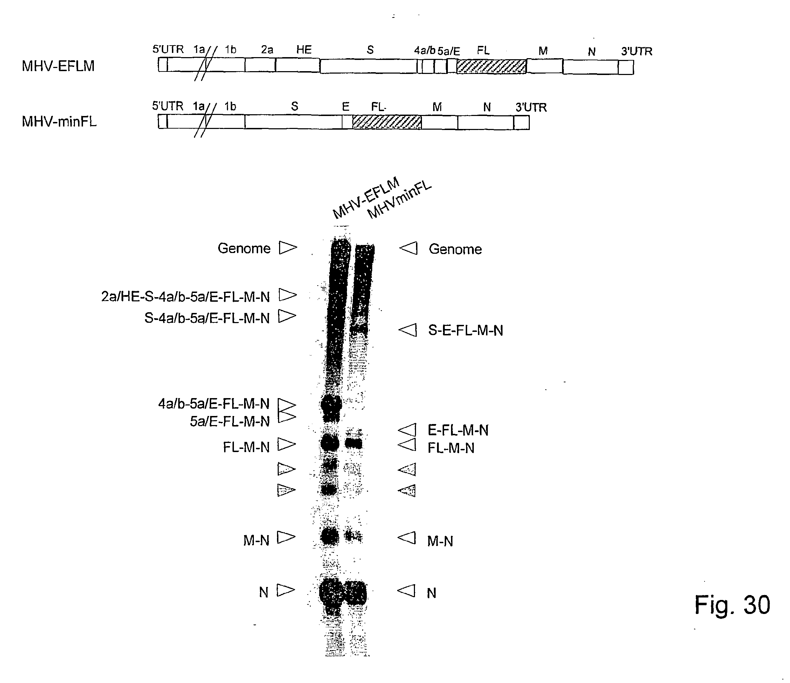

   Fig. 30: MHV-EFLM and MHV-minFL

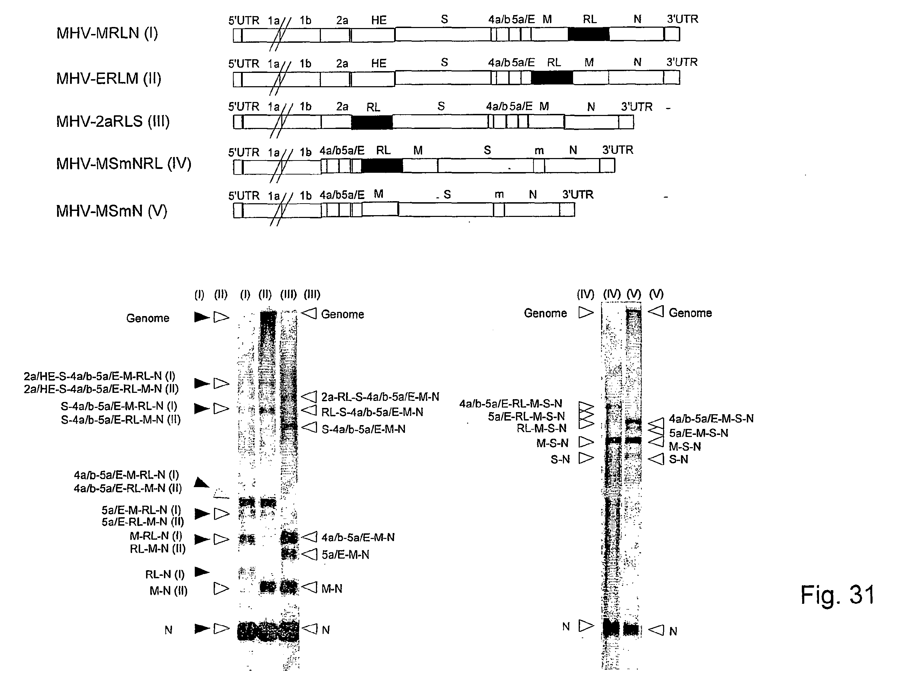

   Fig. 31: MHV-MRLN, MHV-ERLM, MHV-2aRLS, MHV-MSmNRL, and MHV-MSmN
Fig. 32:
   *In vitro* replication of the recombinant viruses carrying foreign genes. After high-m.o.i. infection of LR7 cells with viruses (top: MHV-MRLN, MHV-ERLM, MHV-2aRLS; middle: MHV-EFLM, MHV-minFL; bottom, MHV-ERLM, MHV-MSmNRL), samples were taken from each culture medium at 9hr post-infection and the infectivity in these samples was analyzed by titration.
Fig. 33:
   Expression of luciferase by recombinant viruses. Intracellular expression of firefly and renilla luciferase of several recombinant viruses was determined according to the manufacturer's instructions (Promega) at 9 hr post-infection. Top: MHV-EFLM and MHV-minFL; bottom: MHV-ERLM and MHV-MSmNRL.

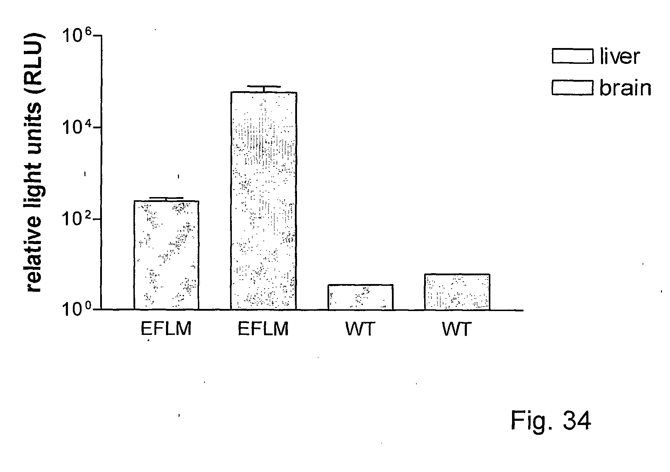

Fig. 34: Expression of firefly luciferase *in vivo.* Eight-week-old, MHV-negative, female BALB/c mice were used in the experiment. Mice were inoculated intranasally with MHV-EFLM. Four animals per dose (10⁶ TCID⁵⁰) were used. Mice were sacrificed and the livers and brains were removed at day 4 post-infection. Organs were quick-frozen in liquid nitrogen and homogenized in Cell Culture Lysis Reagent provided with the Luciferase Assay System (Promega). FL activity was measured according to the manufacturer's instructions using a luminometer (Lumac Biocounter M2500).
Fig. 35:
   Survival rates following inoculation of cats with various deletion mutants: FIPW 79-1146; r-wtFIPV; FIPVΔ3abc ; FIPVΔ7a; and FIPVΔ3abc+7ab (100 pfu).
Fig. 36:
   FIPV neutralizing antibody titers raised at different time points after infection of cats with FIPV deletion variants.
Fig. 37:
   Survival rates following challenge of cats with FIPV 79-1146 (100 pfu). Cats (N=4) not previously vaccinated (□); cats (N=5) previously vaccinated with FIPVΔ3abc (◆); cats (N=5) previously vaccinated with FIPVΔ7ab (□); and cats (N=5) previously vaccinated with FIPVΔ3abc+7ab (□).
Fig. 38:
   One-step growth curves of the recombinant FIPV viruses carrying foreign genes. After high-m.o.i. infection of FCWF cells with viruses, samples were taken from each culture medium at different times and the infectivity in these samples was analyzed by titration. Recombinant nr. 1 (0) and nr. 9 (◆).
Fig. 39:
   Expression of luciferase by recombinant viruses. FCWF cells were infected with viruses expressing the renilla luciferase (nr. 1 □; and nr. 9 ◆) and with wt-rFIPV (□), and the luciferase activity generated in the cells was monitored over time.
Fig. 40:
   One-step growth curves of the recombinant FIPV viruses with deletion of non-essential genes. After high-m.o.i. infection of FCWF cells with viruses, samples were taken from each culture medium at different times and the infectivity in these samples was analyzed by titration.

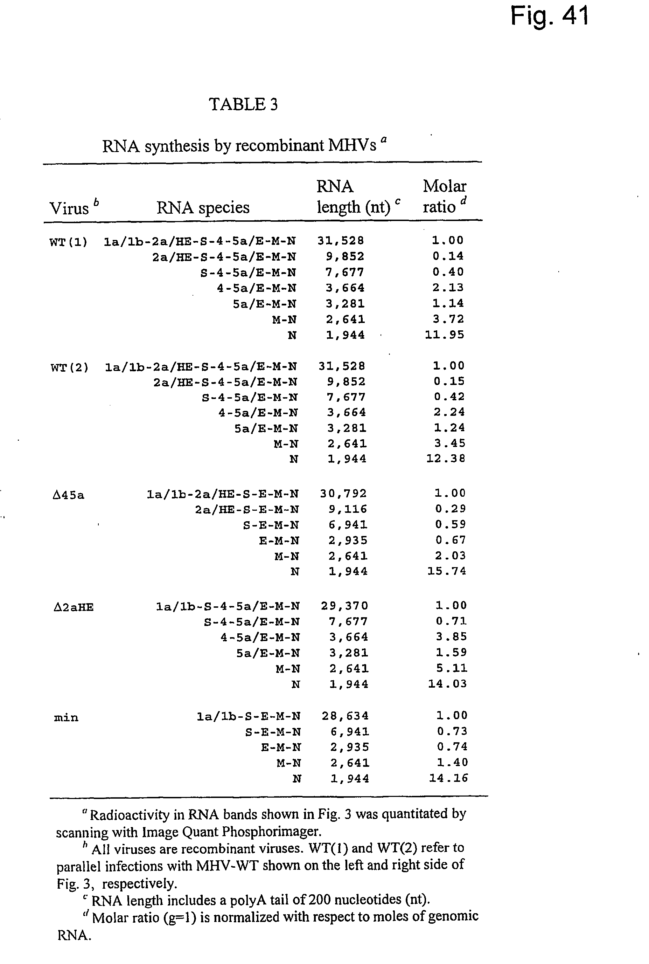

Fig. 41 = Table 3
   Quantitations of RNA synthesis by recombinant MHVs carrying deletions of non-essential genes.
Fig. 42 = Table 4
   Quantitations of RNA synthesis by recombinant MHVs with rearranged genomes.

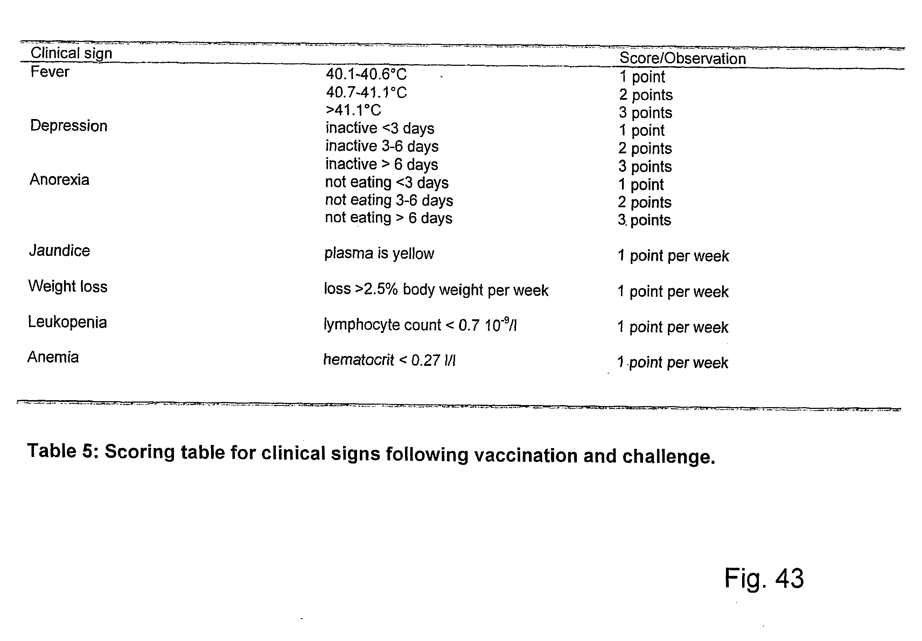

Fig. 43 = Table 5
   Scoring table for clinical signs following vaccination and challenge.

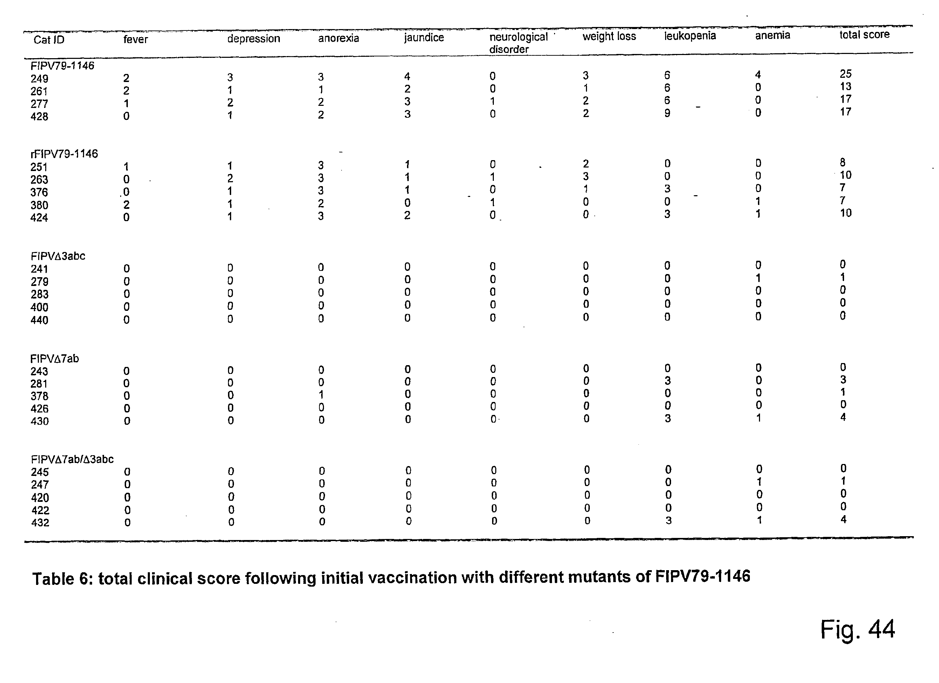

Fig. 44 = Table 6
   Total clinical score following initial vaccination with different mutants of FIPV79-1146.

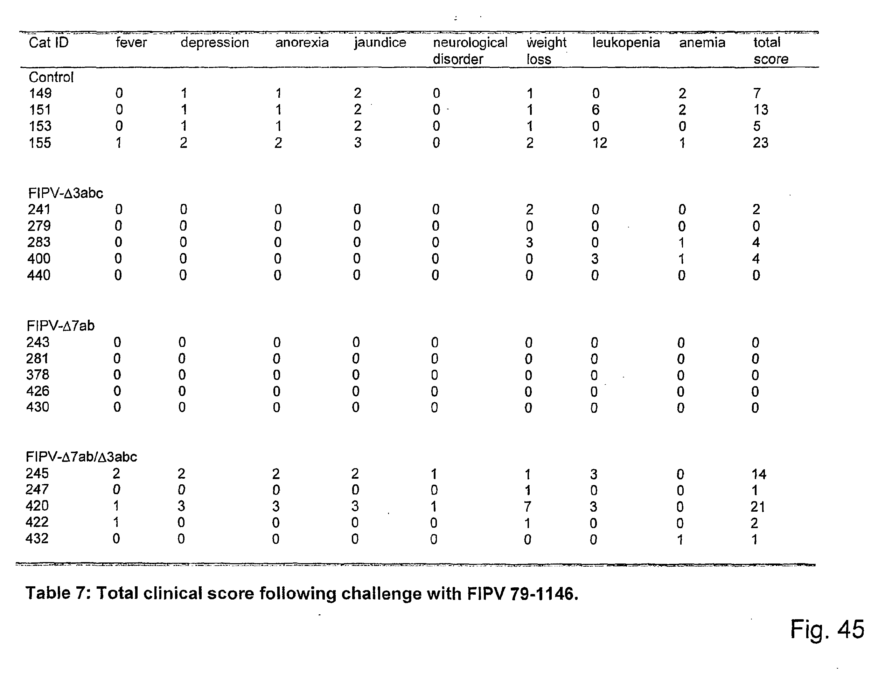

Fig. 45 = Table 7
   Total clinical score following challenge with FIPV 79-1146.
Fig. 46A:
   Replication of the recombinant virus carrying 2 foreign genes. After high-m.o.i. infection of LR7 cells with viruses (MHV-RLFL, MHV-2aRLS, and MHV-EFLM) samples were taken from each culture medium at 9hr post-infection and the infectivity in these samples was analyzed by titration.
Fig. 46B:
   Expression of luciferase by recombinant viruses. Intracellular expression of firefly and renilla luciferase of several recombinant viruses was determined according to the manufacturer's instructions (Promega) at 9 hr post-infection (MHV-RLFL, MHV-2aRLS, and MHV-EFLM).

Experimental data related to the patent:
I. Mouse Hepatitis Virus (MHV), strain A59 (MHV-A59)
   1. Generation of live attenuated viruses
      a. Construction of recombinant MHVs lacking genes
      b. Confirmation of the recombinant genotypes
      c. RNA synthesis by MHV deletion mutants
      d. Tissue culture growth phenotype
      e. Virulence of recombinant viruses in mice
   2. Generation,of recombinant viruses with rearranged gene order
      a. Construction of recombinant viruses with rearranged gene order
      b. RNA synthesis by MHV mutants with rearranged gene order
      c. Tissue culture growth phenotype
      d. Replication of recombinant viruses in mice
   3. Generation of recombinant viruses expressing foreign genes
      a. Construction of recombinant viruses carrying reporter genes
      b. RNA synthesis by recombinant viruses
      c. Replication of viruses expressing renilla or firefly luciferase
      d. Expression of renilla and firefly luciferase in cell culture
      e. Maintenance of foreign genes during viral passage
      f. Expression of firefly luciferase in mice
      g. Generation of MHV expressing two foreign genes from one genome
      h. Generation of MHV expressing a chimaeric Spike-GFP gene
   4. Inhibition of infection and of cell fusion by spike protein derived peptide
II. Feline Infectious Peritonitis Virus (FIPV), strain 79-1146
   1. Generation of mFIPV, a feline coronavirus growing on murine cells
      a. Construction of a synthetic RNA transcription vector
      b. Generation of mFIPV by RNA recombination
      c. mFIPV protein analysis
      d. mFIPV growth characteristics
   2. Generation of live attenuated FIPV vaccine by gene deletions
      a. Construction of synthetic RNA transcription vectors
      b. Generations of recombinant FIPVs lacking genes
      c. Genetic analysis of recombinant FIPVs lacking genes
      d. Growth characteristics of FIPVs lacking genes
      e. Virulence of recombinant viruses in cats
      f. Immune-response induced by recombinant viruses
      g. FIPV deletion viruses serve as attenuated, live vaccines
   3. Insertion and expression of foreign genes
      a. Construction of recombinant viruses carrying reporter genes
      b. One-step growth of viruses
      c. Expression of renilla luciferase
   4. Generation of multivalent FIPV-based vaccines
      a. Construction of a multivalent Feline leukemia virus (FeLV) vaccine based on FIPV vector
      b. Construction of a multivalent Feline immunodeficiency virus (FIV) vaccine based on a FIPV vector
      c. Construction of a multivalent Feline calicivirus (FCV) vaccine based on a FIPV vector
      d. Construction of a multivalent Feline panleucopenia virus (FPV) vaccine based on a FIPV vector
      e. Construction of a multivalent Feline herpes virus (FHV) vaccine based on a FIPV vector
      f. Construction of a multivalent FIPV serotype I and II vaccine based on a FIPV serotype II vector
   5. Generation of recombinant viruses with rearranged gene order
   6. Generation of FIPV based vaccines against canine pathogens
      a. Generation of FIPV based vaccine against canine distemper
      b. Generation of FIPV based vaccine against canine parvo disease
      c. Generation of FIPV based vaccine against infectious canine hepatitis
      d. Generation of FIPV based vaccine against hemorrhagic disease of pups
III. Transmissible gastro-enteritis virus (TGEV)
   1. Generation of a live attenuated vaccine against TGEV
   2. Generation of multivalent TGEV-based vaccines
      a. Construction of a multivalent Porcine Parvovirus (PPV) vaccine based on a TGEV vector
      b. Construction of a multivalent swine influenza virus vaccine based on a TGEV vector
      c. Construction of a multivalent African swine fever virus vaccine based on a TGEV vector
      d. Construction of a multivalent Porcine circovirus type 2 vaccine based on a TGEV vector
      e. Construction of a multivalent Porcine respiratory and reproductive syndrome virus vaccine based on a TGEV vector
   3. Generation pf recombinant viruses with rearranged gene order
IV. Avian Infectious Bronchitis Virus (IBV)
   1. Generation of a live vaccine based on attenuated IBV
   2. Generation of multivalent IBV-based vaccines
      aI. Construction of a multivalent vaccine based on an IBV vector that protects against more than one IBV serotype.
      aII. Construction of a multivalent vaccine based on an IBV vector that protects against Newcastle Disease.
      b. Construction of a multivalent vaccine based on an IBV vector that protects against Avian Influenza.
      c. Construction of a multivalent vaccine based on an IBV vector that protects against Chicken Anemia Virus (CAV) disease.
      d. Construction of a multivalent vaccine based on an IBV vector that protects against Avian reovirus disease.
      e. Construction of a multivalent vaccine based on an IBV vector that protects against Infectious Bursal Disease.
      f. Construction of a multivalent vaccine based on an IBV vector that protects against Marek's disease.
      g. Construction of a multivalent vaccine based on an IBV vector that protects against Infectious laryngotracheitis.
   3. Generation of recombinant viruses with rearranged gene order
V. Human Coronavirus (HCoV) strain 229E (HCoV-229E)
   1. Generation of a live vaccine based on attenuated HCoV-229E
   2. Generation of a live attenuated vaccine against HCoV strain OC43
   3. Generation of multivalent HCoV -based vaccines
      a. Construction of a multivalent vaccine based on an HCoV vector that protects against Respiratory Syncytial Virus (REV)
      b. Construction of a multivalent vaccine based on an HCoV vector that protects against rotavirus
      c. Construction of a multivalent vaccine based on an HCoV vector that protects against Norwalk-like viruses
      d. Construction of a multivalent vaccine based on an HCoV vector that protects against influenza virus
   4. Generation of recombinant viruses with rearranged gene order

### I. Mouse Hepatitis Virus (MHV), strain A59 (MHV-A59)

### I.1. Generation of live attenuated viruses

General aim: establish whether deletion from the coronaviral (i.c. MHV-A59) genome of genes or gene clusters not belonging to the genes specifying the polymerase functions (ORF1a/1b) or the structural proteins N, M, E, and S, is tolerated and yields viable viruses even if these gene sequences are removed altogether; establish whether such deletions have an attenuating effect on the virus when inoculated into mice.

### I.1.a. Construction of recombinant MHVs lacking genes

### Specific aim: generate MHV-A59 deletion mutants lacking genes 2a + HE (MHV-□Δ2aHE), genes 4a + 4b + 5a (MHV-□Δ45a), and genes 2a + HE + 4a + 4b + 5a (MHV-min).

Approach: targeted RNA recombination (3, 9, 10) using fMHV, the MHV-A59 derivative infecting feline (FCWF) cells not murine (LR7) cells (7), and synthetic donor RNAs carrying the intended deletions (Fig. 1A, top).

Procedure: Transcription vectors for the production of synthetic donor RNAs were constructed from the plasmid pMH54 (7), which encodes a run-off transcript consisting of the 5' end of the MHV-A59 genome (467 nt) fused to codon 28 of the HE gene and running to the 3' end of the genome (Fig. 1). Plasmid pMH54 was used to reconstruct the recombinant WT-MHV, an fMHV derivative again infecting murine cells. Transcription vector pXH□Δ45a lacks the ORFs 4a, 4b, and 5a. For the construction of this plasmid a PCR product was obtained from plasmid pB59 (2) by using primer 1089 (5'-ACCTGCAGGACTAATCTAAACTTTATTCTTTTTAGGGCCACGC-3'), which encodes a PstI/Sse8387I restriction site, an intergenic sequence (IGS) and which is complementary to the sequence just upstream of the E or 5b gene, and primer 1092 (5'-CCTTAAGGAATTGAACTGC-3'), which is complementary to the 5' end of the M protein coding region. The PCR product was cloned into pGEM-T Easy (Promega) according to the manufacturer's instructions, yielding pXH0803. The PCR product was subsequently excised with PstI and EcoRV and cloned into pMH54 treated with Sse8387I and EcoRV, resulting in pXH□Δ45a. Transcription vector pXH□Δ2aHE lacks ORFs 2a and HE and contains approximately 1200 bp of the 3' end of the polymerase gene fused to the S gene. To construct this plasmid a PCR product was obtained by splicing overlap extension PCR. One PCR product was obtained from plasmid p96 (1) using primer 1128 (5'-ACGGTCCGACTGCGCGCTTGAACACGTTG-3'), which encodes a RsrII restriction site and is complementary to the region 1200 bp upstream of the ORF1b stop codon, and primer 1130 (5'-CATGCAAGCTTTATTTGACATTTACTAGGCT-3'), which is complementary to the 3' end of the polymerase coding region and the IGS region upstream of the S gene. The other PCR product was obtained from pMH54 using primer 1129 (5'-GTCAAATAAAGCTTGCATGAGGCATAATCTAAAC-3'), which is complementary to primer 1130, and primer 1127 (5'-CCAGTAAGCAATAATGTGG-3'), which is complementary to the 5' end of the S gene. The PCR products were purified and mixed and then amplified with primers 1128 and 1127. The PCR product obtained in the second round of PCR was cloned into pGEM-T Easy, yielding pXH1802. The PCR product was excised with RsrII and AvrII and cloned into pMH54 treated with the same enzymes, resulting in pXH□Δ2aHE. Transcription vector pXHmin has the ORF1b 3' end fused to the S gene and the deletion of ORF4a, 4b and 5a. This vector was constructed by cloning the fragment excised with PstI and EcoRV from pXH0803 into pXH□2aHE treated with Sse8387I and EcoRV. The composition of all PCR-generated segments was confirmed by DNA sequencing.

To generate the deletion mutant viruses, donor RNAs were transcribed from the (PacI-linearized) pMH54-derived plasmids and transfected by electroporation into feline FCWF cells that had been infected with fMHV. These infected and transfected cells were then plated onto a monolayer of mouse LR7 (7) cells. After 24 h of incubation at 37°C, progeny viruses were harvested by taking off the cell culture supernatant and candidate recombinants were selected by two rounds of plaque purification on LR7 cells.

Result: With each of the synthetic donor RNAs used, clear plaques were obtained. Conclusion: Recombinant viruses had been obtained that had regained the ability to grow on murine cells.

### I.1.b. Confirmation of the recombinant genotypes

Aim: Confirming by RT-PCR the genetic make-up of the recombinant viruses obtained.

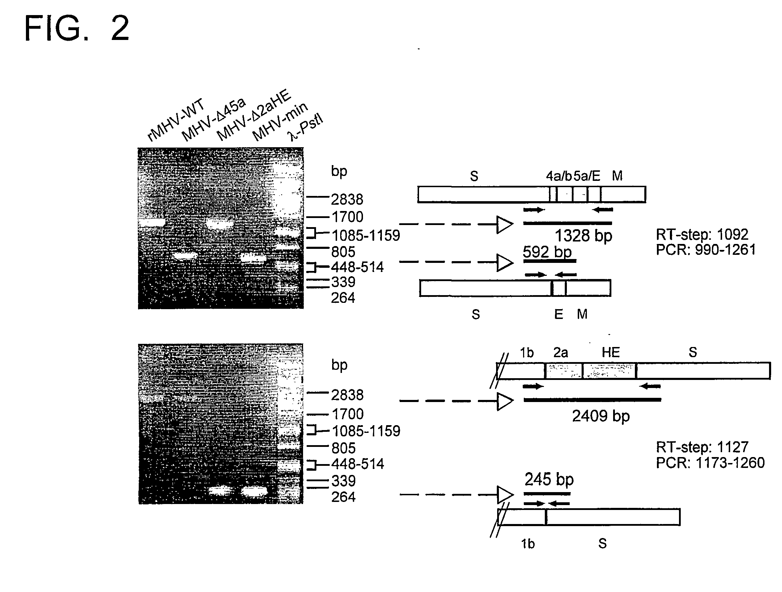

Procedure and Results: Cloned recombinant viruses, one from each recombination experiment, were produced on LR7 cells, viral RNA was isolated and RT-PCR was done using standard methods on genomic RNA as shown in Fig. 2. To confirm the deletion of the ORFs 4 and 5a, the RT step was performed with primer 1092 (5'-CCTTAAGGAATTGAACTGC-3'), which is complementary to the 5' end of the M gene, while the PCR was performed with primer 1261 (5'-GCTGCTTACTCCTATCATAC-3') and primer 990 (5'-CCTGATTTATCTCTCGATTTC-3'), which are complementary with the 3' end of the E and S gene, respectively. In the case of the recombinant MHV-WT an RT-PCR product corresponding in size with the expected 1328 bp was observed (Fig. 2, top). As expected, a PCR product of the same length was observed for MHV-Δ□2aHE. In contrast, both for MHV-□Δ45a and MHV-min much smaller RT-PCR products were detected. The smaller size of the RT-PCR products corresponded with the deletion of 736 bp. The deletion of ORFs 2a and HE was analyzed in a similar way. The RT step was performed with primer 1127 (5'-CCAGTAAGCAATAATGTGG-3'), which is complementary to the 5' end of the S gene. The PCR was performed with primer 1173 (5'-GACTTAGTCCTCTCCTTGA-3') and primer 1260 (5'-CTTCAACGGTCTCAGTGC-3'), which are complementary to the 3' end of the 1b gene and the 5' end of the S gene, respectively. Both for MHV-WT and MHV-□Δ45a PCR products were detected, which were much bigger than the PCR products detected for MHV-Δ2aHE and MHV-min (Fig. 2, bottom). The difference in size corresponded with the deletion of 2164 bp. Finally, the newly generated junctions, present in the genomes of the deletion mutant viruses (Fig. 1A [triangles] and 1B [sequence]), were analyzed by sequencing of the RT-PCR products. To this end the PCR products were cloned into the pGEM-T easy vector (Promega). The sequences obtained were in perfect agreement with the predictions.

Conclusion: The constructed viral mutants had the intended genetic deletions.

### I.1.c. RNA synthesis by MHV deletion mutants

Aim: Confirming the patterns of RNAs synthesized in cells infected by the mutant viruses.

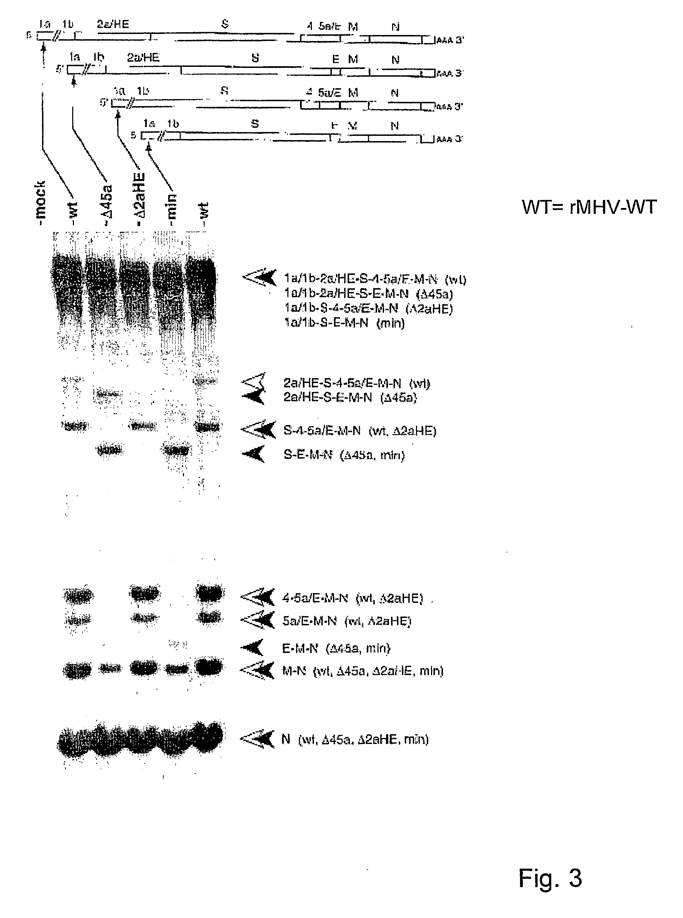

Procedure: Infected 17Cl1 cells were metabolically labeled with [³³P]orthophosphate in the presence of actinomycin D essentially as described (4, 10). Samples of total cytoplasmic RNA, purified using Ultraspec reagent (Biotecx), were denatured with formaldehyde and formamide, separated by electrophoresis through 1% agarose containing formaldehyde, and visualized by fluorography (Fig. 3; note that the subgenomic RNA species in this figure are designated by their composition, rather than as RNA2 through RNA7, since the numerical designations would be ambiguous for the deletion mutants).

Result: For the recombinant MHV-WT the RNA pattern and the relative molar amounts of the six subgenomic (sg) RNA species and the genomic (g) RNA were very similar to those reported previously for MHV (10)(4)(5)(8), with one notable exception. The 4-5a/E-M-N sgRNA, which is usually denoted RNA4, was far more abundant than previously observed for this species in wild-type MHV (10)(4)(5)(8). This was presumably due to the three nucleotide changes at positions 13, 15, and 18 upstream of the consensus transcription regulatory signal, (5'AAUCUAAAC3') that precedes gene 4 (Fig. 1) which were introduced into the transcription vector pMH54 to create the Sse8387I site downstream of the S gene (7). For the deletion mutants, all variant sgRNAs had mobilities that corresponded to their predicted sizes (Fig. 3 and Table 3), and no prominent extra species were observed. The relative molar amounts of the mutant sgRNA species were quite similar to those of their wild-type counterparts originating from the corresponding transcription regulatory signals. Conclusion: The results confirm the genotypes of the recombinant viruses and demonstrate their expected phenotypes at the RNA level.

### I.1.d. Tissue culture growth phenotype

Aim: Comparing the in vitro growth phenotypes.

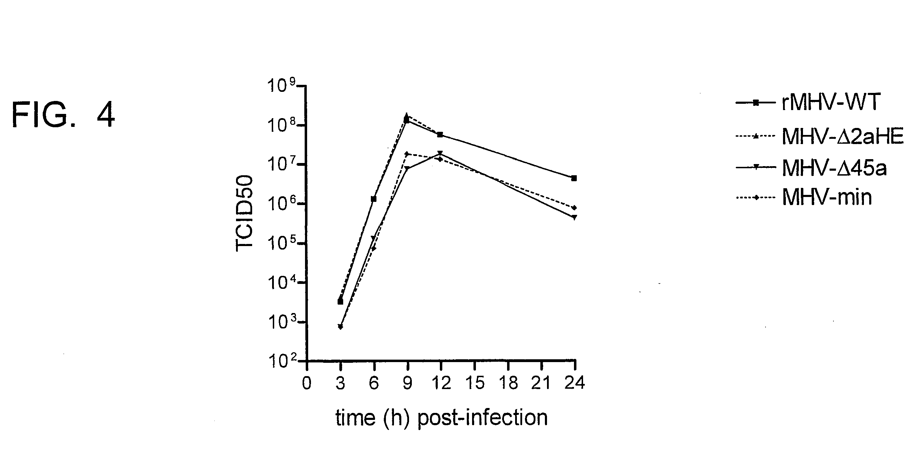

Procedure and Results: Confluent LR7 cell monolayers grown in 35-mm dishes were infected with each recombinant virus (8 PFU/cell) and viral infectivity in culture media at different times post-infection (p.i.) was determined by titration on LR7 cells. TCID⁵⁰ (50% tissue culture infective doses) values were calculated and plotted (Fig. 4). The recombinant viruses did not differ appreciably with respect to the induction of extensive syncytia or cytopathic effects or in their plaque size. However, MHV-□Δ45a and MHV-min differed from MHV-WT and MHV-□Δ2aHE in their one-step growth kinetics (Fig. 4). The two viruses displayed approximately 10-fold lower titers at all time points.

Conclusion: All deletion viruses multiply well *in vitro* although the deletion of genes 4 and 5a had a slightly negative effect.

### I.1.e. Virulence of recombinant viruses in mice

Aim: Establishing whether the genetic deletions affect viral virulence.

Procedure and Results: The recombinant viruses were characterized in their natural host, the mouse. As a first step, we determined the virulence of the recombinant viruses. An LDso (50% lethal dose) assay was carried out by inoculating MHV-negative, C57Bl/6 mice mice intracranially with four 10-fold serial dilutions (5x10⁵ - 5x10²) of recombinant viruses. Viruses were diluted using PBS containing 0.75% bovine serum albumin. A volume of 25 µl was used for injection into the left cerebral hemisphere. Five animals per dilution per virus were analyzed. LD₅₀ values were calculated by the Reed-Muench method based on death by 21 days post-infection. Clearly, deletion mutant viruses were attenuated when compared to the recombinant MHV-WT. While the MHV-WT virus had an LD50 of 1.8x10⁴ no LD₅₀ could be derived for the deletion mutants. Although the animals inoculated with the higher doses showed some signs of illness, none of the animals infected with any of the deletion mutant viruses died up to input of 50,000 PFU/mouse. This implies that the LD₅₀ for these viruses exceeds a value of 50,000 and may well be above 100,000.

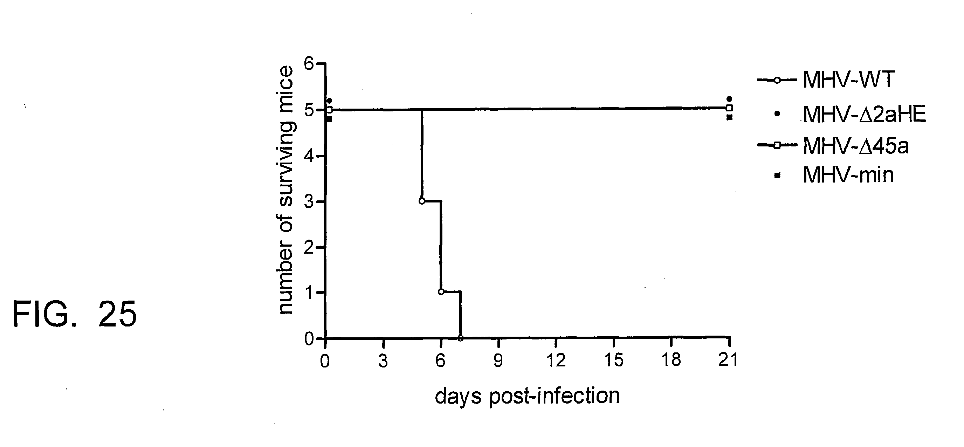

Figure 25 illustrates the kinetics of mortality of mice infected with the highest inoculation dose, 2.5x10⁵ plaque forming units (PFU), of WT and deletion viruses. While all the animals inoculated with wild type virus had died by seven days post infection, the deletion viruses were highly attenuated, displaying no death and less severe clinical symptoms. Despite the observation of no mortality, all mice infected with the □45a and □2aHE viruses showed clinical signs of hunched posture, disheveled appearance and waddling gait during the first week post infection; these symptoms were less severe and observed in fewer mice infected with MHV-min. Conclusion: Viruses with deletions of the sequences specifying the genes 2a + HE or genes 4a + 4b + 5a or of the combination of all these genes exhibit a significantly attenuated phenotype in mice. In other words, the non-essential genes of coronaviruses are not crucial for in vitro growth but determine viral virulence. The attenuation acquired by their deletion thus provides excellent viral vaccines and therapeutic vectors.

### I.2. Generation of recombinant viruses with rearranged gene order

General aim: establish whether the invariable order of the genes specifying the polymerase functions (ORF1a/1b) and the structural proteins S, E, M, and N in the coronaviral genome is essential for the viability of these viruses or whether rearrangement of this order is tolerated.

### I.2.a. Construction of recombinant viruses with rearranged gene order

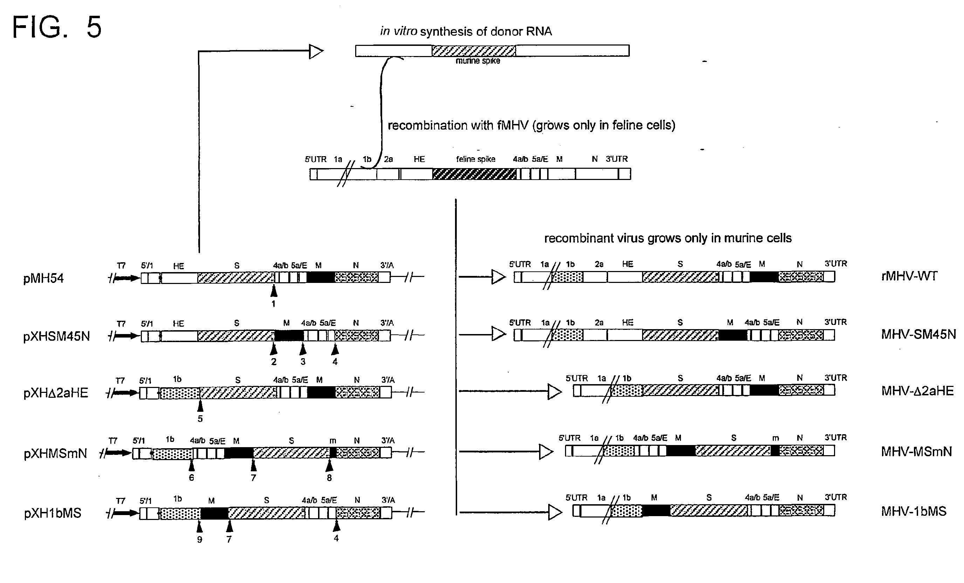

Specific aim: generate MHV-A59 mutants in which the relative positions of structural protein genes in the MHV-A59 genome are changed by moving the M and/or E gene. Approach: targeted RNA recombination using fMHV and synthetic donor RNAs carrying the intended rearrangements (Fig. 5, top).

Procedure: Transcription vectors for the production of donor RNA for targeted recombination were constructed from plasmids pMH54 and pXH□2aHE (described above). In order to generate transcription vector pXHSM45N (Fig. 5, lower left part), a PCR product was generated by splicing overlap extension (SOE)-PCR that contained the 3' end of the M gene and the 5' end of the N gene and in which a EcoRV restriction site was introduced between the M gene and IGS just upstream of the N gene. To generate this PCR fragment, outside primer 1C (5'-GTGTATAGATATGAAAGGTACCGTG-3'), corresponding to the region of the M gene that contains the unique KpnI site, and outside primer 1097 (5'-CGAACCAGATCGGCTAGCAG-3'), corresponding to the region of the N gene that contains the unique NheI site, were used. Primer 1095 (5'-AGATTAGATATCTTAGGTTCTCAACAATGCGG-3') and primer 1096 (5'-GAACCTAAGATATCTAATCTAAACTTTAAGGATG-3') were used as inside primers. They correspond to the sequence between the M and the N gene and introduce the EcoRV restriction site. The resulting PCR product was cloned into pGEM-T easy (Promega) yielding vector pXH0302. As a next step in the construction of pXHSM45N, pMH54 was treated with the restriction enzymes Sse8387I and EcoRV and the resulting fragment was cloned into the EcoRV site of pXH0302 after being blunted by T4 DNA polymerase treatment, yielding vector pXH0902. After excision of the Sse8387I-EcoRV fragment of pMH54, the remaining vector was also blunted by T4 DNA polymerase treatment and religated resulting in plasmid pXH1401. Finally, plasmid pXH0902 was treated with restriction enzymes NheI and BssHII and the resulting fragment was cloned into pXH1401 treated with the same enzymes, yielding pXHSM45N.

For the construction of pXHMSmN, first the ORFs 4, 5 and M were removed from pMH54 by restriction of this vector with enzymes Sse8387I and BssHII, followed by treatment with T4 DNA polymerase and religation of the remaining vector, which yielded pXH□45M5'. Next, the fragment resulting from treatment of pXH0302 with enzymes NheI and BssHII was cloned into pMH54 treated with the same enzymes, resulting in pXHMeN. Subsequently, the fragment obtained after restriction of pXHMeN with EcoRV was cloned into pXH1802 (described above), which was digested with HindIII and treated with Klenow fragment of DNA polymerase I, yielding pXH0305B. The fragment obtained by restriction of pMH54 with enzymes MluI and EcoRV was cloned into pB59 (2) treated with the same enzymes, which resulted in vector pXH2801. Next, the fragment resulting from the treatment of pXH2801 with restriction enzymes KpnI and PstI was treated with T4 DNA polymerase and cloned into pXH1802 treated with restriction enzyme HindIII and with Klenow fragment of DNA polymerase I, yielding pXH0806. Subsequently, the fragment obtained by digestion of pXH0305B with SpeI and AflII was cloned into pXH0806 treated with the same enzymes, resulting in pXH1506. Finally, pXHSmN was obtained by cloning the fragment resulting from restriction of pXH1506 with RsrII and AvrII into pXH□45M5' treated with the same enzymes.

For the construction of transcription vector pXH1bMS, vector pXHMeN was restricted with EcoRV. The resulting fragment was removed and the vector was religated yielding pXH□M. Next, the fragment obtained by digestion of pXH0305B with RsrII and AvrII was cloned into pXH□M treated with the same enzymes, yielding pXH1bMS.

All constructs were confirmed by restriction and/or sequence analysis. They are depicted schematically in Fig. 5 (left). All new junctions generated, including the introduction of the Sse8387I site downstream of the S gene in pMH54 (7), are indicated with arrowheads, while their sequences are shown in Table 1. Recombinant viruses were generated by RNA-RNA recombination between transcription vector run-off transcripts and the fMHV genome as described above. After 2 rounds of plaque purification on LR7 cells the viruses were analyzed by reverse transcriptase-PCR on genomic RNA and found to contain the genomes with the expected organization.

Conclusion: The strict gene order of the coronaviruses is not an essential prerequisite for viability.

### I.2.b. RNA synthesis by MHV mutants with rearranged gene order

Aim: Confirming the patterns of RNAs synthesized in cells infected by the mutant viruses.

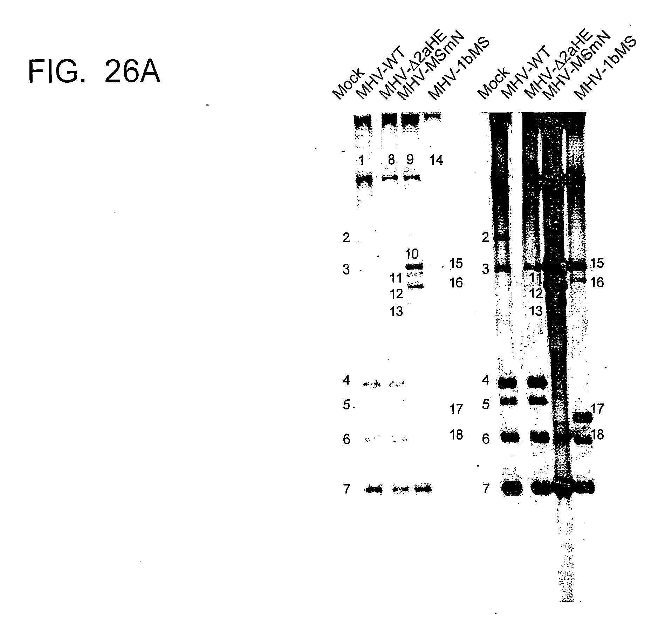

Procedure: Infected 17Cl1 cells were metabolically labeled with [³³P]orthophosphate in the presence of actinomycin D essentially as described (4, 10). Samples of total cytoplasmic RNA, purified using Ultraspec reagent (Biotecx), were denatured with formaldehyde and formamide, separated by electrophoresis through 1% agarose containing formaldehyde, and visualized by fluorography (Fig. 26A).

Result: Coronaviruses express their genome via the generation of a 3' co-terminal nested set of sg RNAs. Recombinant viruses with a rearranged genome organization are therefore predicted to synthesize patterns of viral RNAs that are distinctly different from that of the parent virus. For the reconstructed wild-type virus (MHV-WT) and for MHV-□2aHE, the RNA patterns and the amounts of the genomic (g) and sg RNA species appeared to be similar to those observed previously (Fig. 3). Note that MHV-□2aHE - as well as its derivatives MHV-MSmN and MHV-1bMS - does not synthesize the sg RNA species encoding the 2a protein. For the MHV mutants with the rearranged genomes, all variant sg RNAs had mobilities that corresponded to their predicted sizes (Fig. 26A and Table 4), and no obvious additional species were observed. MHV-SM45N grew very poorly, and was therefore labeled only weakly. All sg RNAs could be detected except the one from which the M protein should be translated. The low abundance of this sg RNA, the reason of which is unknown, is likely to be the cause of the impaired growth of this virus. Overall, the patterns of viral RNAs synthesized by the cells infected with the recombinant viruses nicely reflect the changes made to the coronavirus genome organization.

Conclusion: The results confirm the genotypes of the recombinant viruses and demonstrate their expected phenotypes at the RNA level.

### I.2.c. Tissue culture growth phenotype

### Aim: Comparing the in vitro growth phenotypes of the mutant viruses.

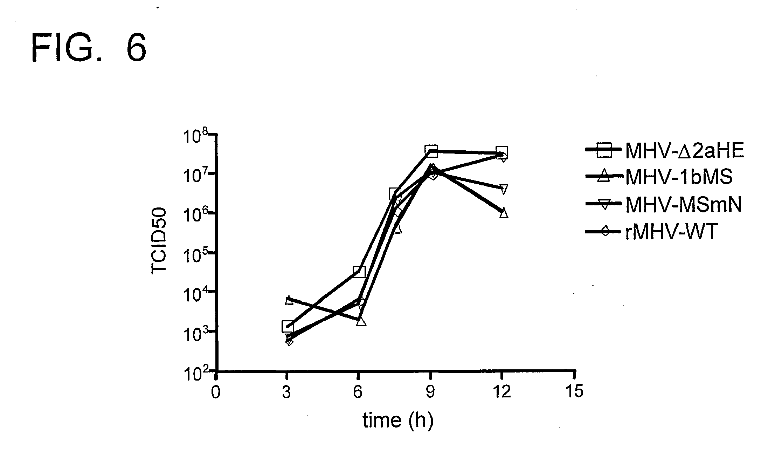

Procedure and Results: Confluent LR7 cell monolayers grown in 35-mm dishes were infected with each recombinant virus (8 PFU/cell) and viral infectivity in culture media at different times post-infection was determined by titration on LR7 cells. TCID⁵⁰ values were calculated and plotted (Fig. 6). All viruses except the mutant MHV-SM45N were analyzed in this way. The infectious titer of this latter virus was too low (approximately 1000 times lower than the WT recombinant MHV) to perform a one-step growth curve. Although MHV-1bMs appeared to induce syncytia somewhat slower than the WT recombinant, all viruses replicated approximately to the same extent in the one-step growth curve.

Conclusion: Except for mutant virus MHV-SM45N, the gene rearrangement had no dramatic effect on their *in vitro* growth characteristics.

### I.2.d. Replication of recombinant viruses in mice

Aim: Establishing whether viruses with rearranged gene order are able to replicate in mice.

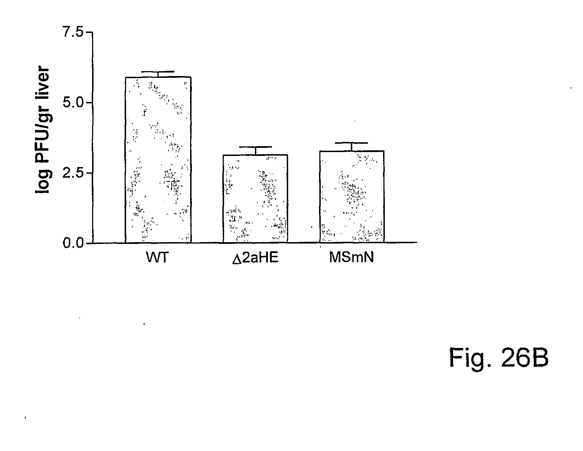

Procedure and Results: Eight weeks old, MHV-negative, female BALB/c mice were used in the experiment. Viruses were diluted in PBS and a total volume 100 µl (10⁶ TCID⁵⁰) was used for injection in the peritoneal cavity. Four animals per virus were inoculated. Mice were sacrificed and the livers were removed at day 4 post-infection. The livers were placed in 1.5 ml DMEM, weighed and then frozen at -80□C until titered for virus. Virus titers were determined by plaque-assay on LR7 cell monolayers following homogenization of the organs. The replication of MHV-WT, MHV-□2aHE and MHV-MSmN was studied in their natural host, the mouse. While the 50% lethal dose of MHV-WT in mice was previously determined at 2.7x10⁴ PFU, MHV-□2aHE was not virulent enough for a 50% lethal dose value determination (section I.1.e.). Therefore, we now decided to analyze the *in vivo* replication of the recombinant viruses. Mice inoculated intraperitoneally with 1x10⁶TCID⁵⁰ were euthanized at day 4 post-infection and the viral replication in the liver was determined. The results are shown in Fig. 26B. MHV-□2aHE and MHV-MSmN replicated in the liver to a similar extent albeit much lower than MHV-WT. Deletion of ORFs 2a and HE generated a recombinant virus (MHV-□2aHE) that was attenuated in the natural host, as shown in section I.1.e., while additional rearrangement of the coronavirus gene order (MHV-MSmN) did not result in a more attenuated phenotype in this assay.

Conclusion: Viruses with a rearranged gene order, which lack the typical coronavirus genome organization, and viruses that lack ORFs 2a and HE are able to replicate in their natural host, the mouse.

### I.3. Generation of recombinant viruses expressing foreign genes

Aim: Establish whether foreign genes can be inserted at different positions in the viral genome, either as an additional gene or replacing deleted non-essential genes or in combination with a rearranged gene order; establish whether these genes are expressed and whether they are stably maintained during *in vitro* passage of the virus.

### I.3.a. Construction of recombinant viruses carrying reporter genes

Aim: Generate MHV-A59 viruses with foreign gene insertions.

Procedure: Several viruses were constructed containing a foreign reporter gene in their genome at different positions (see Fig. 7A). Two reporter genes were used, encoding renilla luciferase (RL) and firefly luciferase (FL). For both genes, a plasmid was constructed in which the gene was preceded by the MHV intergenic sequence (IGS). From this construct the expression cassette (gene plus IGS) could then be transferred into the different transcription vectors.

As a first step, the MHV IGS was cloned in front of the RL gene. To this end, primer 1286 (5'-GGATACTAATCTAAACTTTAG -3') and 1287 (5'-CTAGCTAAAGTTTAGATTAGATATCCTGCA -3') were annealed to each other and cloned into pRL-null (Promega) treated with NheI and PstI, resulting in pXH1909. For the construction of the transcription vector containing the RL gene between genes 2a and S (pXH22aRLS) the following steps were taken. Vector p96 (1) was restricted with HindIII and MluI and the resulting fragment was cloned into pXH1802 (described above) treated with HindIII and BssHII, yielding pXH2103. Next, the RL expression cassette was removed from pXH1909 by restriction with EcoRV and XbaI, treated with Klenow fragment of DNA polymerase I and cloned into pXH2103 digested with HindIII and treated with Klenow fragment, resulting in pXH2509A. Finally pXH22aRLS was constructed by cloning the fragment resulting from digestion of pXH2509A with RsrII and AvrII into pMH54 treated with the same enzymes.

For the construction of pXH2ERLM the same expression cassette, that was used to make pXH2509A, was cloned into pMH54 digested with EcoRV.

This same expression cassette was also cloned into pXHMeN (described above) treated with EcoRV, yielding pXH2ERLN. Subsequently, pXH2MRLN was constructed by cloning the fragment resulting from restriction of pXHMeN with EcoRV into pXH2ERLN treated with the same enzyme. pXHMSmNRL was constructed by cloning the renilla expression cassette into pXHMSmN (described above) treated with EcoRV.

For the construction of pXHEFLM, the FL gene was first cloned behind the same IGS as was used for the RL expression cassette. To this end, the luciferase gene was removed from pSP-Luc+ (Promega) by restriction with AvrII and XbaI and cloned into pXH1909 treated with NheI and XbaI, yielding pXH2711. Subsequently, the FL expresssion cassette was cut out of pXH2711by restriction with EcoRV and XbaI, treated with Klenow fragment of DNA polymerase I, and cloned into pMH54 restricted by EcoRV, resulting in pXHEFLM.

Plasmid pXHminFL was constructed by cloning the FL expression cassette into pXHmin (described above) digested with EcoRV. This plasmid lacks ORFs 2a/HE/4a/4b/5a and contains the FL gene between genes E and M.

After confirmation of all constructs by restriction and sequence analysis, recombinant viruses were generated by RNA-RNA recombination between transcription vector run-off transcripts and the fMHV genome as described above. The resulting viruses were genetically confirmed by RT-PCR analysis.

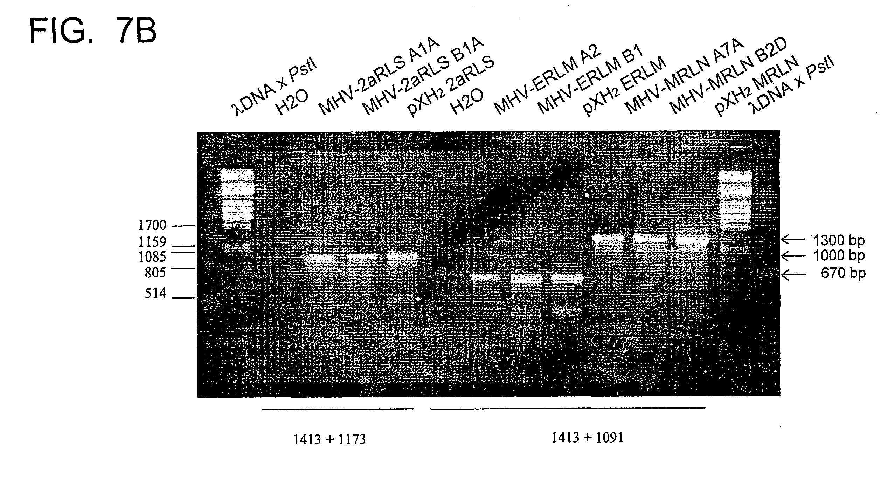

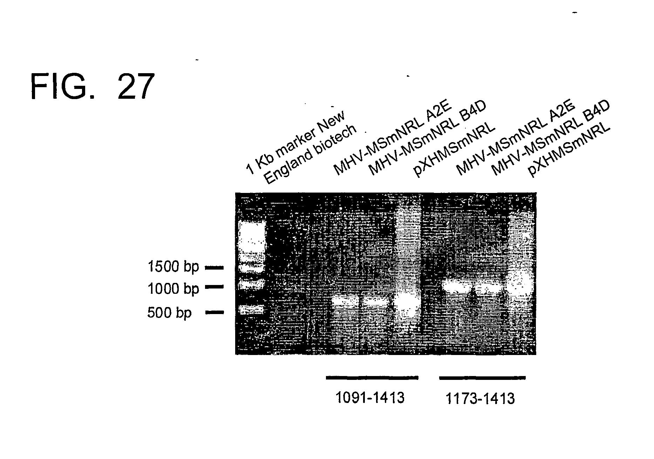

Results of recombinant viruses containing the RL gene are shown in Fig. 7B and 27. To confirm the insertion of this gene an RT step was performed on genomic RNA with primer 1412 (5'-CTGCGGACCAGTTATCATC-3'), which is complementary to the 5' end of the RL gene. Subsequently, a PCR was performed with primer 1091(5'-GTTACAAACCTGAATCTCATCTTAATTCTGGTCG-3') and primer 1413 (5'-CATCCGTTTCCTTTGTTCTGG-3') for MHV-ERLM and MHV-MRLN or with primer 1173 (5'-GACTTAGTCCTCTCCTTGATTG-3') and primer 1413 for MHV-2aRLS. Primer 1091 and primer 1173 correspond with the 3' end of gene 4b and gene 1b, respectively, while primer 1413 corresponds with the 5' end of the RL gene. As positive controls, the appropriate transcription vectors were taken along. In all cases, PCR fragments were obtained of the same size as the positive controls, while the water control was negative. The observed (and predicted) fragment sizes were approx. 1,000 bp for MHV-2aRLS, approx. 670 bp for MHV-ERLM, and approx. 1,300 bp for MHV-MRLN (7B). For MHV-MSmNRL a PCR was performed with primer 1091 and primer 1413 and with primer 1173 and primer 1413. As positive controls, the appropriate transcription vector was taken along. In all cases, PCR fragments were obtained of the same size as the positive controls. The observed (and predicted) fragment sizes were approx. 670 bp for the PCR reaction with primers 1091 and 1413, and approx. 1,200 bp for the PCR reaction with primers 1173 and 1413 (7B) (note that in Fig. 7B and 27 for each type of virus 2 independently obtained viral clones were analyzed and included). The results confirmed the insertion of the RL gene into the MHV genome at the correct position.

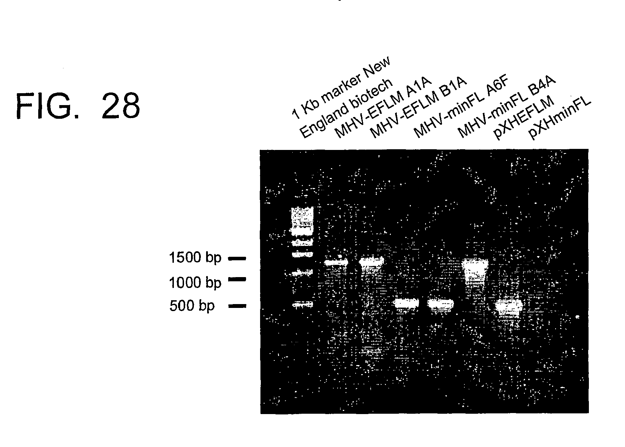

Results of recombinant viruses containing the FL gene are shown in Fig. 28. To confirm the insertion of this gene an RT step was performed on genomic RNA with primer 1475 (5'-GCCTAATGCAGTTGCTCTCC-3'), which is complementary to the 5'end of the FL gene. Subsequently, a PCR was performed with primer 935 (5'-GTTTTAGCACAGGGTGTGGCTCATG-3'), which corresponds with the 3'end of the S gene, and primer1474 (5'-CCATCTTCCAGCGGATAG-3'), which corresponds with the 5'end of the FL gene. As positive controls, the appropriate transcription vectors were taken along. In all cases, PCR fragments were obtained of the same size as the positive controls, while the water control was negative. The observed (and predicted) fragment sizes were approx. 1,200 bp for MHV-EFLM, and approx. 500 bp for MHV-ininFL (note that in Fig. 28 for each type of virus 2 independently obtained viral clones were analyzed and included).

Conclusion: Insertion of genetic modules into the coronaviral genome is tolerated at all positions tested; all the intended viruses were viable.

### I.3.b. RNA synthesis by recombinant viruses

Aim: Confirming the patterns of RNAs synthesized in cells infected by the mutant viruses.

Procedure: Infected 17C11 cells were metabolically labeled with [³³P]orthophosphate in the presence of actinomycin D essentially as described (4, 10). Samples of total cytoplasmic RNA, purified using Ultraspec reagent (Biotecx), were denatured with formaldehyde and formamide, separated by electrophoresis through 1% agarose containing formaldehyde, and visualized by fluorography (Fig. 29-31; note that the subgenomic RNA species in this figure are designated by their composition, rather than as RNA2 through RNA7, since the numerical designations would be ambiguous for the mutants).

Result: For all the MHV mutants with the luciferase genes, all variant sg RNAs had mobilities that corresponded to their predicted sizes (Fig. 29-31). For the viruses containing the renilla luciferase gene no obvious additional species were observed. For the viruses containing the firefly luciferase gene two additional RNA species were observed, which correspond in size with transcription of sgRNAs from sequences in the firefly luciferase gene that are similar to the MHV IGS. Overall, the patterns of viral RNAs synthesized by the cells infected with the recombinant viruses nicely reflect the insertion of the luciferase expression cassettes into the coronavirus genome.

Conclusion: The results confirm the genotypes of the recombinant viruses and demonstrate their expected phenotypes at the RNA level.

### I.3.c. Replication of viruses expressing renilla or firefly luciferase

Aim: Compere the growth characteristics of the viruses with wild-type virus and with each other.

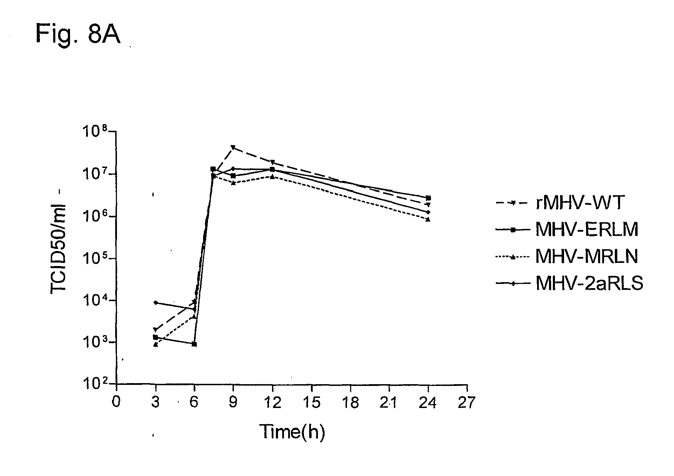

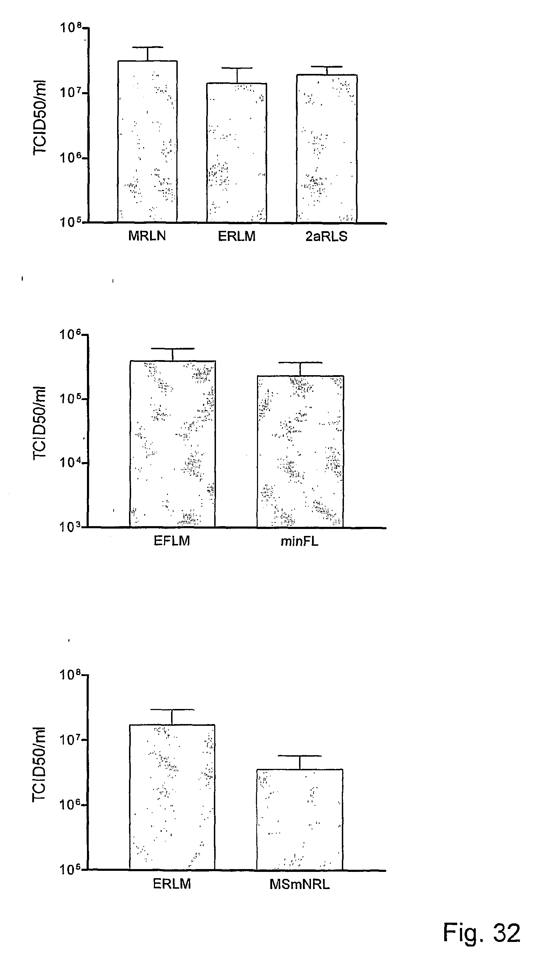

Procedure and Results: After two rounds of plaque purification virus stocks were prepared, titrated and used for high m.o.i (m.o.i. of 8) infection of LR7 cells after which the viral infectivities in the culture media were monitored. The results are represented by the growth curves shown in Fig. 8A and 8B. and by the results shown in Fig 32. Of MHV-EFLM two viral clones, independently obtained from the recombination experiment described under I.3.a, were analyzed. In Fig. 32, the TCID50 values obtained at 8-9 hours post-infection are shown. Obviously, the growth characteristics of the recombinant viruses shown in Fig 8A and 8B are essentially indistinguishable; all viruses grew to titers that were comparable to that of recombinant wild-type virus. MHV-MSmNRL reached somewhat lower titers (Fig. 32).

Conclusion: The inserted expression cassettes hardly affected the in vitro growth characteristics of the recombinant viruses.

### I.3.d. Expression of renilla and firefly luciferase in cell culture

Aim: Establish whether the inserted expression cassettes were functional.

Procedure and Results: Confluent monolayer cultures of LR7 cells were infected at a m.o.i. of 8 and the production of luciferase activity in the cells was monitored over time. RL expression in cells was measured by using the Dual-Luciferase Reporter Assay System (Promega) according to the manufacturer's instructions. Similarly, FL expression was measured by using the Luciferase Assay System (Promega) according to the manufacturer's instructions. RL and FL activity was measured in relative light units (RLU) using a luminometer (Lumac Biocounter M2500 or Turner Designs Model TD-20/20).

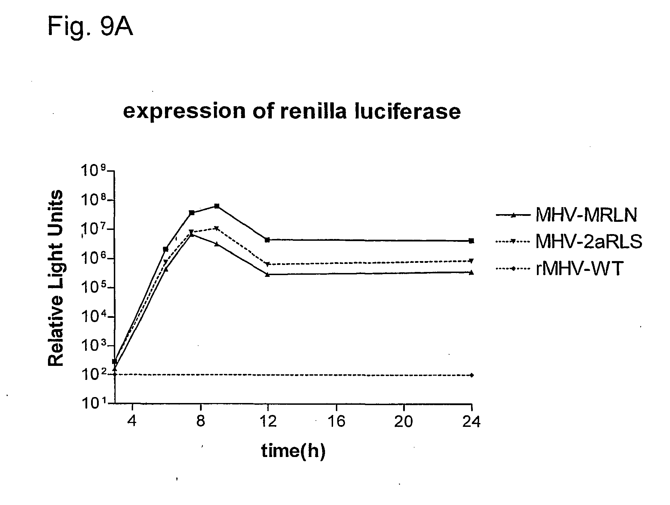

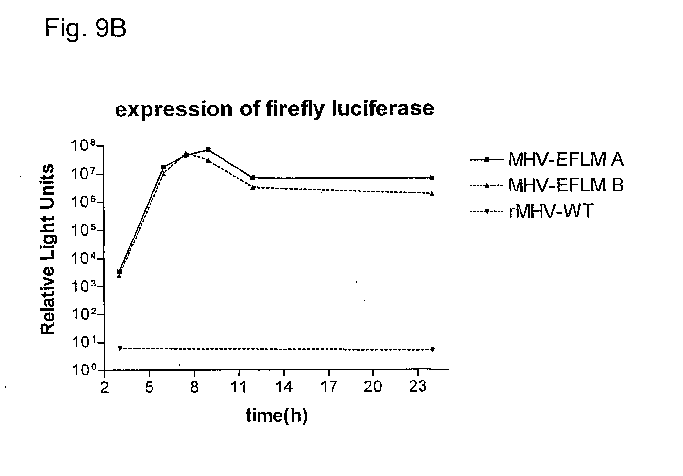

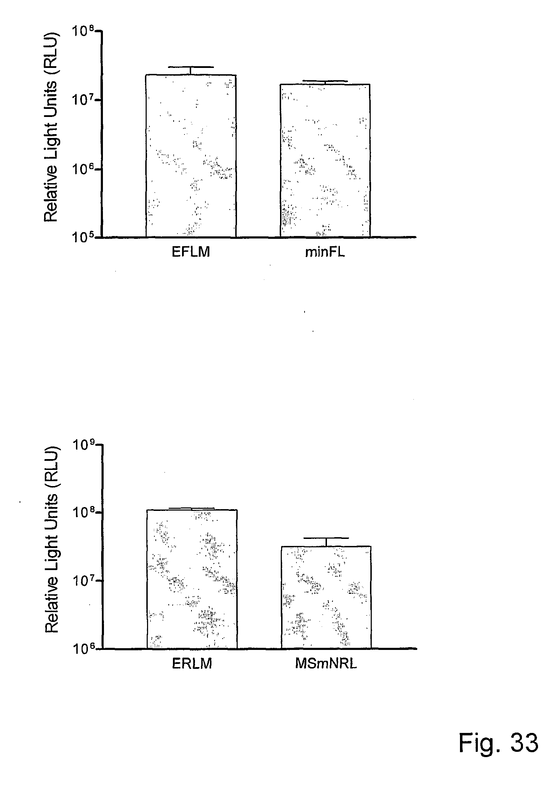

The results are shown graphically in Fig. 9A,9B and 33. All recombinant viruses except for the recombinant wild-type virus expressed high levels of luciferase, indicating that both the renilla and the firefly luciferase gene cassettes were functional at each genomic position tested. For most viruses the highest expression level was reached at 9h post-infection. The expression level of firefly luciferase at this time-point was determined at 1.6 ug/10⁶ cells. MHV-minFL expressed levels of luciferase activity that were comparable to those produced by the other recombinant viruses containing the FL gene, while expression of the renilla luciferase gene in cells infected with MHV-MSmNRL was approx. 10-fold lower than in cells infected with MHV-ERLM. This difference corresponds with the difference found in replication between MHV-MSmNRL and MHV-ERLM.

Conclusion: Foreign genes can be expressed by coronaviruses both by the additional insertion of such a gene, by using the genetic space created by deletion of nonessential genes, or in combination with a rearranged genome organization.

### I.3.e. Maintenance of foreign genes during viral passage

Aim: Evaluate the stability of the inserted luciferase genes during viral passage.

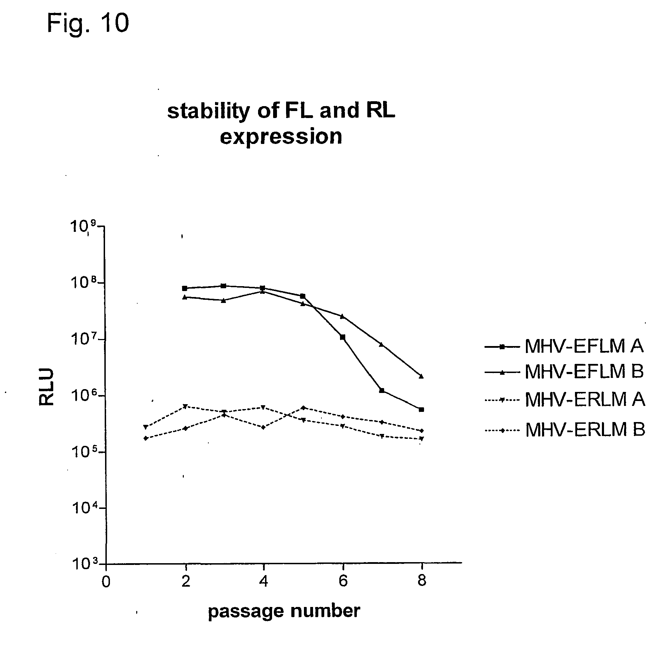

Procedure and Results: The recombinant viruses MHV-ERLM and MHV-EFLM were passaged 8 times over LR7 cells at low m.o.i. (<0.05). After each passage the viral infectivity (TCID50) in the culture medium at 9h post-infection was determined. Subsequently, the firefly and renilla luciferase activity was determined in a parallel expression experiment (m.o.i.=5) at 8 hr post-infection (Fig. 10). Both of MHV-ERLM and of MHV-EFLM two independently obtained clones A and B were analyzed. While the renilla luciferase expression was consistently stable for at least 8 passages, that of the firefly luciferase was stable only for 5 passages. After passage 5 of MHV-EFLM, the expression level clearly decreased for both independent clones. After 8 passages 10 viral clones were isolated by plaque assay both of MHV-ERLM and of MHV-EFLM and tested for luciferase expression. While for MHV-ERLM all 10 clones were positive, 9 of the MHV-EFLM clones no longer showed clearly detectable luciferase expression.

Conclusion: A foreign gene can be stably maintained in the coronaviral genome for at least 8 passages *in vitro.*

### I.3.f. Expression of firefly luciferase in mice

Aim: Establish whether the inserted luciferase gene is expressed in the natural host, the mouse.

Procedure and Results: Eight weeks old, MHV-negative, female BALB/c mice were used in the experiment. Mice were inoculated intranasally with 10⁶TCID⁵⁰ MHV-EFLM. Four animals per virus were used. Mice were sacrificed and the livers and brains were removed at day 4 post-infection. Organs were quick-frozen in liquid nitrogen and homogenized in Cell Culture Lysis Reagent provided with the Luciferase Assay System (Promega). FL activity was measured according to the manufacturer's instructions using a luminometer (Lumac Biocounter M2500). Clearly, as shown in Fig. 34, luciferase activity could be detected both in liver and brain. As an alternative way to evaluate whether the foreign gene was also expressed *in vivo,* i.e. in animals, a mouse was inoculated intraperitoneally with 10⁶ TCID⁵⁰ MHV-EFLM. Four days later the mouse was sedated and luciferain was administered subcutaneously. The luciferase expression was evaluated 5 min later by real time recording of the emission of light from the body of the sedated mouse using a sensitive screen coupled to a CCD camera. Light emanating from the liver area of the mouse was clearly observed.

Conclusion: A foreign gene can be expressed by coronaviruses in their natural host.

### I.3.g. Generation of MHV expressing two foreign genes from one genome

Aim: Establish whether two foreign genes can be expressed from a single genome.

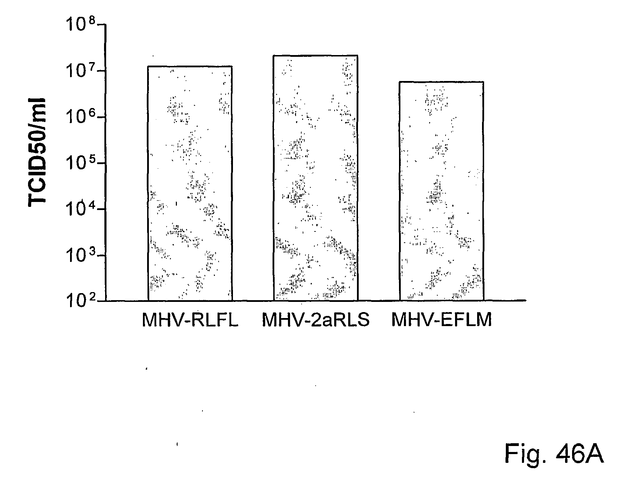

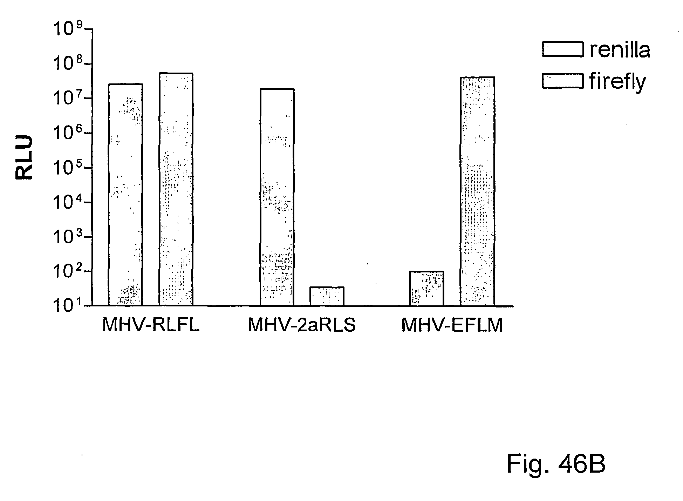

Procedure and Results: pXH2aRLSEFLM was generated by cloning the FL expression cassette into pXH2aRLS digested with EcoRV. After confirmation of the construct by restriction and sequence analysis, recombinant viruses were generated by RNA-RNA recombination between transcription vector run-off transcripts and the fMHV genome as described above. The resulting virus, MHV-RLFL, was genetically confirmed by RT-PCR analysis. Both renilla luciferase activity and firefly luciferase activity could be detected in individual plaques. After generation of a high titer stock, confluent monolayer cultures of LR7 cells were infected at a m.o.i. of 8 and the production of luciferase activity in the cells was monitored over time. RL expression in cells was measured by using the Renilla Assay System (Promega) according to the manufacturer's instructions. Similarly, FL expression was measured by using the Luciferase Assay System (Promega) according to the manufacturer's instructions at 8 hr post-infection. RL and FL activity was measured in relative light units (RLU) using a luminometer (Lumac Biocounter M2500). The results show that MHV-RLFL replicated to the same extent as MHV-2aRLS and MHVEFLM (Fig. 46A). MHV-RLFL expressed both renilla luciferase and firefly luciferase, MHV-2aRLS expressed renilla luciferase only, while MHV-EFLM expressed firefly luciferase only (Fig 46B). Conclusion: Two foreign genes can be expressed from a single coronavirus genome

### I.3.h. Generation of MHV expressing a chimaeric Spike-GFP gene

Aim: Establish whether recombinant MHV can be generated that expresses and incorporates chimaeric spike-GFP proteins.

Procedure and Results: The GFP gene was cloned in frame with the S gene in pMH54. After confirmation of the construct by restriction and sequence analysis, recombinant viruses were generated by RNA-RNA recombination between transcription vector run-off transcripts and the fMHV genome as described above. The resulting virus, MHV-SGFP, was genetically confirmed by RT-PCR analysis. Plaques were microscopically analyzed and showed expressing of GFP as evidenced by the green fluorescence. Immunoprecipitation analysis indicated that hybrid proteins were generated that could be precipitated with specific antibodies to MHV-S and GFP.

Conclusion: MHV can be generated that expresses a chimaeric S-GFP gene in stead of a wild-type S gene. This mutant virus is viable and could be propagated in cell culture indicating that these hybrid proteins are incorporated into the viral particle.

### I.4. Inhibition of infection and of cell fusion by spike protein derived peptide

General aim: inhibit coronaviral infection and the spread of an ongoing infection by interfering with membrane fusion using peptides.

Specific aim: produce a peptide constituting a sequence derived from the membrane-proximal heptad repeat region (HR2) of the MHV-A59 S protein and demonstrate its inhibitory effect on MHV-A59 entry into LR7 cells and on cell-cell fusion in an infected culture of these cells.

### Procedures:

### a. plasmid constructions:

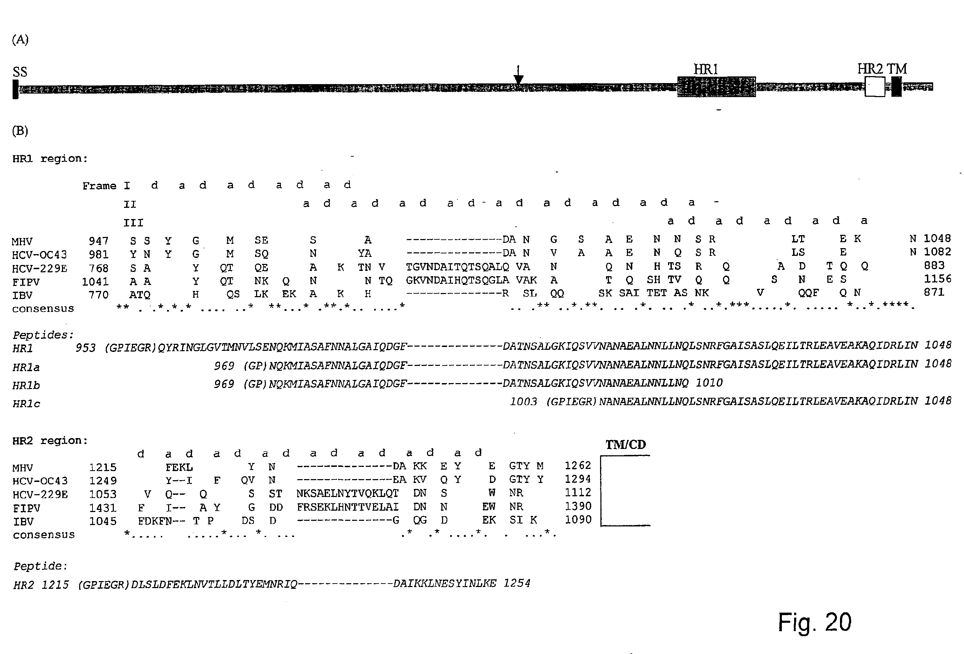

A PCR fragment from a template plasmid pTUMS (13) containing the MHV-A59 spike gene was obtained, corresponding to amino acid residues 1216-1254 (HR2) of the S protein (Fig. 20). The forward primer used was: 5' GCGGATCCATCGAAGGTCGTGATTTATCTCTCGATTTC 3'. This primer introduced an upstream BamHI site and a sequence encoding a factor Xa cleavage site immediately downstream the BamHI site into the amplified fragment. The reverse primer (5' CGAATTCATTCCTTGAGGTTGATGTAG 3') contained a downstream EcoRI site as well as a stop codon preceding the EcoRI site. The PCR fragment was cloned into the BamHI-EcoRI site of the pGEX-2T bacterial expression vector.

### b. bacterial protein expression and purification:

Freshly transformed,BL21 cells (NOVAGEN) were grown in 2YT medium to log phase (OD₆₀₀ of 1.0) and subsequently expression was induced by adding IPTG (GibcoBRL) to a final concentration of 0.4mM. Two hours after the start of induction the cells were pelleted, resuspended in 1/25 of culture volume 10mM Tris pH (8.0), 10mM EDTA, 1mM PMSF and sonicated on ice (5 times for 2 min with 1-min intervals). Cell lysates were centrifuged at 20,000 x g for 60 min at 4°C. Then, 2 ml glutathione-sepharose 4B (50% v/v in PBS) was added per 50 ml of supernatant and the suspension was incubated overnight (O/N) at 4°C under rotation. Beads were washed three times with 50ml PBS and resuspended in a final volume of 1ml PBS. Peptides were cleaved from the GST moiety on the beads using 20U of thrombin by incubation for 4 hours at room temperature (RT). Peptides in the supernatant were HPLC purified on a Phenyl column with a lineair gradient of acetonytrile containing 0.1% trifluoroacetic acid. Peptide containing fractions were vacuum-dried O/N and dissolved in water. Peptide concentration was determined by measuring the absorbance at A₂₈₀ nm or by BCA protein analysis (Micro BCA™ Assay Kit, PIERCE).

### c. virus-cell entry and cell-cell fusion inhibition assays:

The potency of the HR2 peptide in inhibiting viral infection was determined using the recombinant MHV-EFLM expressing the firefly luciferase. Confluent monolayers of LR7 cells in 96 wells plates were inoculated at 37°C in DMEM at a multiplicity of infection of 5 for 1h in the presence of varying concentrations of peptide ranging from 0.4 - 50µM. After 1h, cells were washed with DMEM and medium was replaced by peptide-free DMEM. At 5 h post infection cells were lysed for 15 minutes at RT in 50µl Lysis buffer, according to the manufacturer's protocol (Luciferase Assay System, Promega). Upon mixing of 10µl cell lysate with 40µl substrate, luciferase activity was measured immediately using a Wallac Betaluminometer. The 50% effective inhibitory concentration (EC₅₀ value) was calculated by fitting the inhibition data to an equilibrium binding equation: % luciferase activity = 100/(1+(C/EC₅₀).

The ability of the peptide HR2 to inhibit spike mediated cell-cell fusion was determined using a plaque assay. Monolayers of LR7 cells in 6 wells plates were inoculated with 50 PFU of MHV-A59 in DMEM at 37°C. After one hour the cells were washed with DMEM and an agar overlay was added containing the HR2 peptide at 50, 10, 2, 0.4 and 0.08 µM concentrations. Plaques were counted at 48h p.i., after staining and fixing with 0.9% formaldehyde/0.75% crystal violet.

### Results:

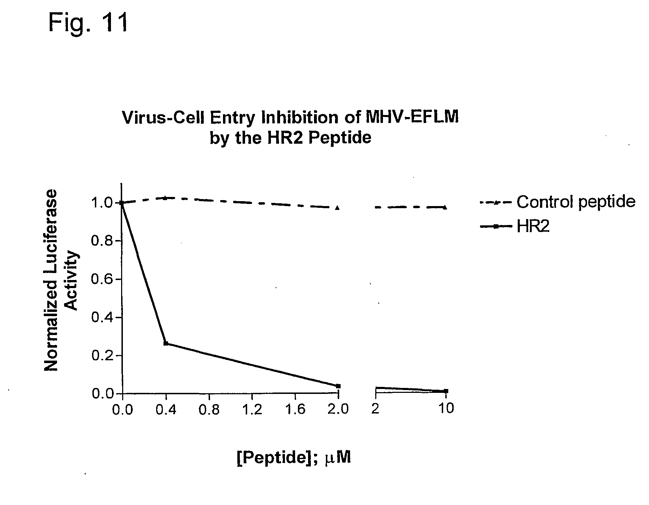

The potency of the HR2 peptide to inhibit virus entry was tested using a virus expressing a luciferase reporter gene as this allows extremely sensitive detection of infection. Inoculations of cells with the virus were carried out in the presence of different concentrations of HR2 peptide. After 1h of inoculation, cells were washed and incubated further in culture medium in the absence of peptide. At 4h p.i., before syncytium formation normally takes place, cells were lysed and tested for luciferase activity (Fig. 11). In this figure the normalized luciferase activity, representing the success of infection, was plotted against the peptide concentration present during inoculation. The HR2 peptide blocked viral entry very efficiently, infection being inhibited virtually completely at the concentration of 50µM. The effective concentration (EC₅₀) at which 50% of viral infection was inhibited was 0.15µM.

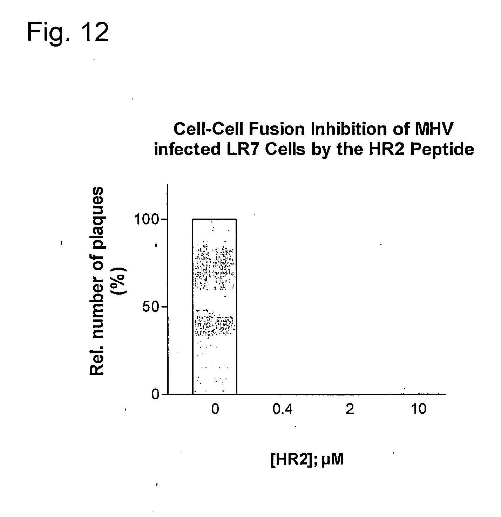

The ability of the HR2 peptide to inhibit cell-cell fusion mediated by the spike protein was examined by using a plaque assay. After inoculation of (parallel cultures of) cells in the absence of peptide, an overlay was applied containing different concentrations of HR2 peptide. The formation of plaques appeared to be completely abolished in the presence of HR2 peptide concentrations of up to 0.4µM (Fig. 12). At 0.08µM of the HR2 peptide only tiny plaques could be observed.

The specificity of the inhibition was demonstrated in all these assays by testing in parallel the effect of other peptides (Fig. 20) prepared identically and used at the same concentrations, including for instance the peptide corresponding to amino acids 1003-1048 of the MHV-A59 S protein (shown as "control peptide" in Fig. 11). Only the HR2 peptide was effective.

Conclusion: The HR2 peptide is a potent inhibitor both of virus entry into cells and of MHV-A59 spike mediated cell-cell fusion.

### II. Feline Infectious Peritonitis Virus (FIPV), strain 79-1146

### II.1. Generation of mFIPV, a feline coronavirus growing on murine cells

General aim: To set up a targeted RNA recombination system for feline coronavirus FIPV 79-1146 similar to the one described above for the genetic manipulation of the murine coronavirus MHV-A59.

Specific aim: Generate mFIPV, a FIPV derivative in which the spike protein ectodomain has been replaced genetically by that of the MHV-A59 S protein, thereby shifting the tropism of the chimaeric virus to murine instead of feline cells.

### I.1.a. Construction of a synthetic RNA transcription vector

Aim: Prepare a plasmid construct from which synthetic donor RNA can be transcribed for targeted RNA recombination and which consists of sequences derived from the 5' end of the FIPV genome fused to sequences derived from the 3' part of the viral genome, i.c. all sequences downstream of (and including) the 3'-terminal end of the ORF1B. Also: prepare a derivative of this plasmid in which the sequence encoding the spike ectodomain has been replaced by that encoding the corresponding domain of the MHV-A59 spike protein.

### Procedure and Results:

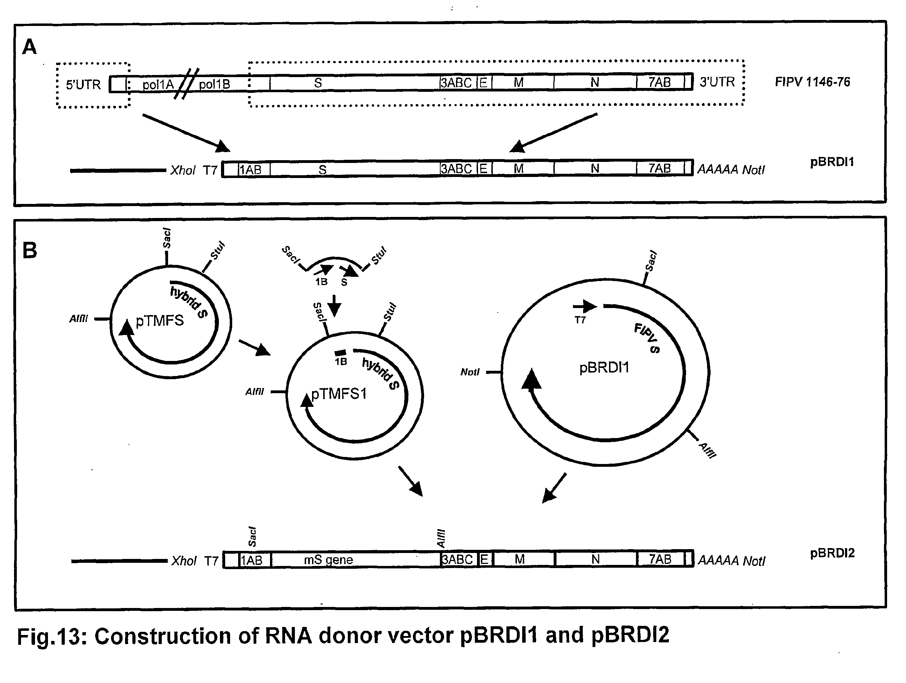

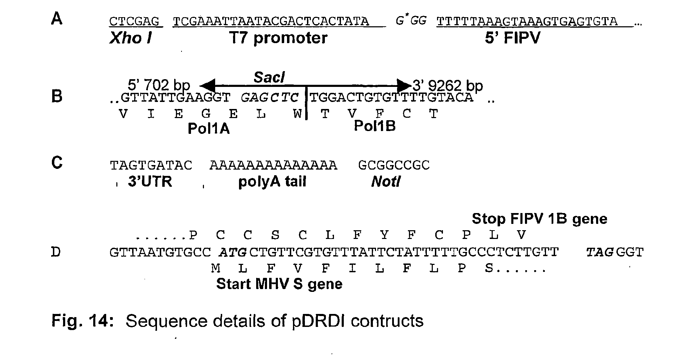

Using standard DNA cloning techniques the vector PBRDI1 was constructed which contains a cDNA copy of the 5'-most 702 bases ligated to the 3'-most 9.262 bases of the FIPV 79-1146 genome (Fig. 13A). The ligation was done in such a way that the ORF 1A (pol 1A) gene fragment was fused in frame at its 3'-end to the 5'-end of the ORF 1B (pol 1B) gene fragment (Fig.14B). Furthermore, at this point a unique *SacI* restriction site was introduced (Fig.14B). The feline coronavirus sequence was placed under the control of a bacteriophage T7 polymerase promoter sequence followed by a triple G (Fig.14A) to drive efficient *in vitro* RNA transcription using the T7 polymerase. At the 3'-end, the sequence was terminated by a polyA tail of 15 A's followed by a unique *NotI* restriction site (Fig.14C) to facilitate run-off transcription. The T7 promoter sequence is preceded by a unique *XhoI* restriction site (Fig.14A) such that the feline coronavirus cDNA could be cloned as a *XhoI-NotI* restriction fragment of 10.015 bp into the backbone vector pBRXN (11) resulting in pBRDI1 (Fig. 13A).

Plasmid pBRDI1 was subsequently used to prepare pBRDI2 in which the FIPV S gene was replaced by a chimaeric spike gene (mS) composed of a part encoding the ectodomain of the MHV spike protein and a part encoding the transmembrane and endodomain of the FIPV spike protein. To introduce this hybrid gene into pBRDI1, first the 3' end of the feline pol1B gene was fused to the 5' end of the murine spike gene (see Fig. 14D for sequence at junctions). This fusion product was cloned into pTMFS (12) as a *SacI-StuI* fragment, resulting in pTMFS1 (Fig. 13B). The hybrid gene was then isolated from pTMFS1 as a *SacI-AlfII* fragment and used to replace the FIPV spike gene from pBRDI1 resulting in pBRDI2 (Fig. 13B). The sequences of pBRDI1 and pBRDI2 are shown in addendum 1 and 2, respectively.

### II.1.b. Generation of mFIPV by RNA recombination

Aim: Generate mFIPV, an FIPV derivative targeted to murine cells.

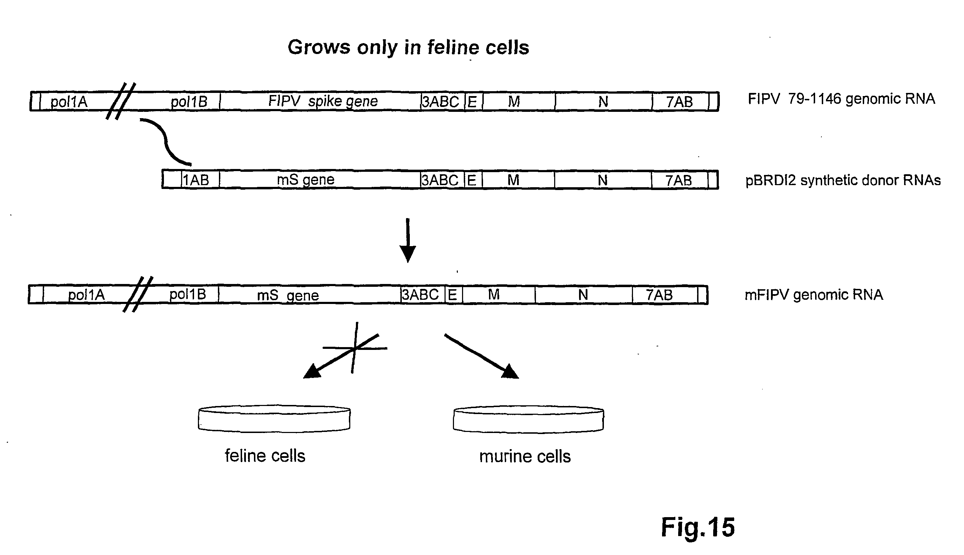

Procedure and Results: Capped, run-off donor RNA transcripts were synthesized from *NotI*-linearized pBRDI2 using a T7 RNA polymerase kit (Ambion) according to the instructions of the manufacturer. The transcripts were introduced into feline FCWF cells (80 cm² culture flask) that had been infected before with FIPV 79-1146 (m.o.i. of 1), by electroporation (Gene pulser electroporation apparatus, Biorad, 2 consecutive pulses; 0.3 kV/960 microF). The electroporated cells were cocultured in a 25cm² flask with murine LR7 cells (50% confluency) to allow recombination of the synthetic and genomic RNA (Fig. 15). After 24 h of incubation at 37°C massive syncytia could be observed of both murine LR7 cells and feline FCWF cells. Candidate mFIPV recombinants were selected by taking off the culture supernatant and passaging the virus by three consecutive end-point dilutions on LR7 cells. The resulting virus was unable to infect and cause cytopathic effect on FCWF cells.

Conclusion: A virus with the intended murine cell tropism was obtained.

### II.1.c. mFIPV protein analysis

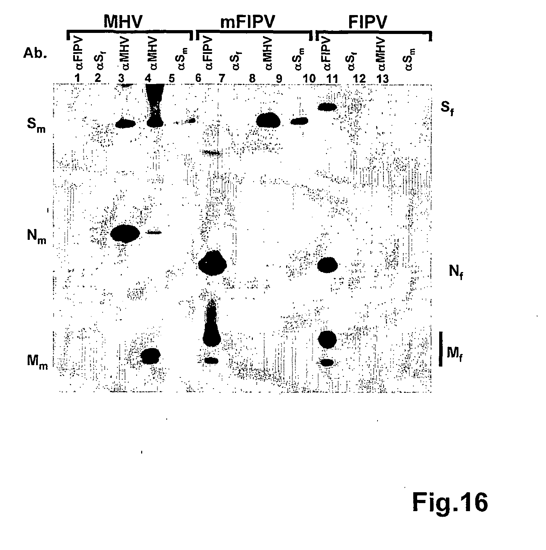

Aim: Confirm the identity of mFIPV at the level of viral protein synthesis. Procedure and Results: Murine LR7 cells were infected with mFIPV and the proteins were labeled with ³⁵S-labeled amino acids for 2 h starting at 5h p.i. As controls, we infected LR7 and FCWF cells with MHV and FIPV, respectively, and labeled them similarly from 5-7h p.i. After the labeling, cell lysates were prepared and immunoprecipitations were carried out in the presence of detergent as described (12). The following antibodies were used (for references, see 12): G73 (αFIPV), an ascitis fluid obtained from an FIPV-infected cat (provided by H.Vennema); K134 (αMHV), a rabbit serum raised against purified MHV-A59; WA3.10 (αSₘ), a Mab against an epitope present in the MHV-A59 S ectodomain; 23F4.5 (αS_{f}), a Mab against an epitope in the FIPV S ectodomain. The immunoprecipitated proteins were taken up in electrophoresis sample buffer and heated for 2 min at 95°C except for one protein sample (lane 4 in Fig. 16) which was kept at room temperature to prevent aggregation of the MHV M protein. The proteins were analyzed by electrophoresis in SDS-12.5% polyacrylamide gel. The electrophoretic patterns are shown in Fig. 16. As expected, the anti-FIPV antibodies precipitated the FIPV proteins S, M and N from the lysate of FIPV-infected cells (lane 10), but none of the MHV proteins from lysate of MHV-infected cells (lane 1). The 23F4.5 Mab precipitated the feline S of FIPV (lane 11) but not the murine S of MHV (lane 2), as expected. Also, the anti-MHV serum precipitated the MHV proteins S, N and M (lane 3 and 4) but not the FIPV proteins (lane 12), and the WA3.10 Mab precipitated the MHV S protein (lane5) but not the FIPV S protein (lane 13). When looking at the proteins precipitated from the mFIPV-infected cell lysates, it is clear that the anti-FIPV serum G73 precipitated the M and N proteins, but not the S protein (lane 6). S protein was also not precipitated by the 23F4.5 Mab (lane 7). The mFIPV S protein was, however, precipitated by the anti-MHV serum (lane 8) as well as by the Mab WA3.10 (lane 9).

Conclusion: Cells infected by mFIPV express the predicted viral proteins, particularly the hybrid S protein with the MHV-derived ectodomain.

### II.1.d. mFIPV growth characteristics

Aim: Compare titers obtained for mFIPV with those for FIPV and MHV-A59.

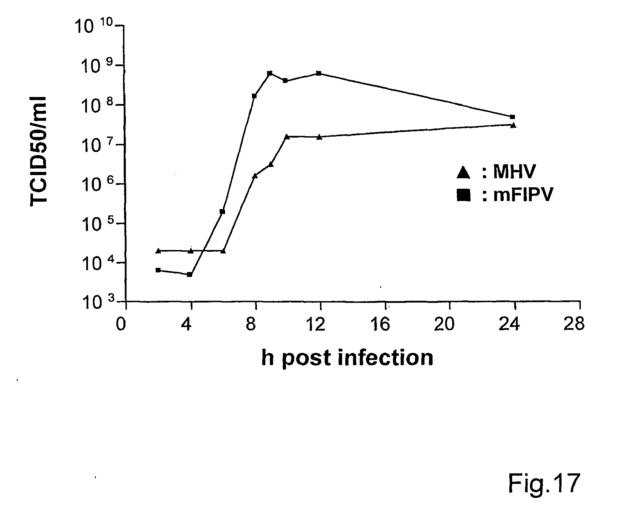

Procedure and Results: Infection and titration experiments revealed that the recombinant virus mFIPV was no longer able to infect feline FCWF cells but did grow efficiently in murine LR7 cells showing similar growth characteristics as MHV-A59. This is demonstrated , for instance, by a comparison of their one-step growth curves in these cells shown in Fig. 17. In this experiment cells were infected with mFIPV and MHV-A59 (m.o.i. of 5 each) after which samples from the culture fluid were taken at various time points and titrated on LR7 cells by end-point dilution. Also, mFIPV induced extensive syncytia and cytopathic effects upon infection of these cells. Stocks of the recombinant mFIPV grown in LR7 cells reached titers of the same order of magnitude as FIPV did in FCWF cells (5.10⁷ PFU/ml). This was, however, an order of magnitude lower than was observed for MHV-A59 in LR7 cells.

Conclusion: The replacement of the spike protein ectodomain has resulted in a recombinant mFIPV that replicates well in its "new" host cells and has apparently not lost much of its biological fitness.

### II.2. Generation of live attenuated FIPV vaccine by gene deletions

General aim: Delete gene sequences from the FIPV genome that are non-essential for viral replication in *vitro* to obtain deletion viruses that are attenuated in feline animals and are therefore viral (vector) vaccine candidates.

### II.2.a. Construction of synthetic RNA transcription vectors

Aim: Construct the plasmids for the synthesis of donor RNA transcripts lacking the genes 3ABC and/or 7AB for targeted recombination with mFIPV RNA (Fig. 18, top part).

Procedure and Results: Deletions of genes 3ABC and 7AB were introduced into the plasmid pBRDI1 using SOE-PCR. For the primers used, see Table 2 and Fig. 18, left side.

To delete the 3ABC cluster, combinations of primers 1 and 4 and of 2 and 3 were used to generate fragments of 375 bp (A) and 1012 bp (B), respectively (Fig. 18, left side). Fragments A and B were fused using the overlap between both fragments through primers 3 and 4, and amplified using primers 1 and 2 resulting in a 1366 bp fragment (C). Fragment C was digested with *AflII* and *SnaBI* and cloned into *AflII* and *SnaBI-*digested pBRDI1, resulting in pBRDI1Δ3ABC (Fig. 18).

To delete the 7AB genes, combinations of primers 5 and 8 and of primers 6 and 7 were used to generate fragments of 1215 bp (D) and of 324 bp (E), respectively (Fig. 18). Fragments D and E were fused using the overlap between both fragments through primers 7 and 8, and amplified using primers 5 and 6 resulting in a 1524 bp fragment (F). Fragment F was digested with *MluI* and *NotI* and cloned into *MluI* and *NotI-* digested pBRDI1, resulting in pBRDI1Δ7AB (Fig. 18). The correctness of the sequences of fragments C and F was confirmed by DNA sequencing.

To construct pBRDI1Δ3ABC+ Δ7AB, the 1524 bp *MLuI*/*NotI* fragment of pBRDI1Δ7AB was introduced into *MluI*/*NotI* -digested pBRDI1Δ3ABC (Fig. 18).

Conclusion: The pBRDI1-derived donor RNA constructs lacking the gene clusters 3ABC and/or 7AB were obtained.

### II.2.b. Generations of recombinant FIPVs lacking genes

Aim: Generate the recombinant FIPVs that lack the sequences for the 3ABC genes, for the 7AB genes, and for both these gene clusters.

Procedure and Results: Capped, run-off donor transcripts were synthesized from *NotI*-linearized pBRDI1, pBRDI1Δ3ABC, pBRDI1Δ7AB and pBRDI1Δ3ABC+ Δ7AB, respectively, using a T7 RNA polymerase kit (Ambion) as specified by the manufacturer. The donor transcripts were introduced into murine LR7 cells (80 cm² flask), that had been infected before with mFIPV (m.o.i. of 0.4), by electroporation (Gene pulser electroporation apparatus, Biorad, 2 consecutive pulses; 0.85 kV/50 microF). The electroporated cells were cocultured in a 25cm² culture flask with feline FCWF cells (50% confluency). After 24 h incubation at 37°C massive syncytia could be detected in both the murine LR7 cells and the feline FCWF cells. Candidate deletion viruses released into the mixed cell culture supernatant were purified by two rounds of plaque purification on FCWF cells.

Conclusion: Viruses that had acquired the ability to grow in feline FCWF cells had been obtained from the recombination experiment with mFIPV.

### II.2.c. Genetic analysis of recombinant FIPVs lacking genes

Aim: Confirm the genetic make-up of the putative deletion viruses.

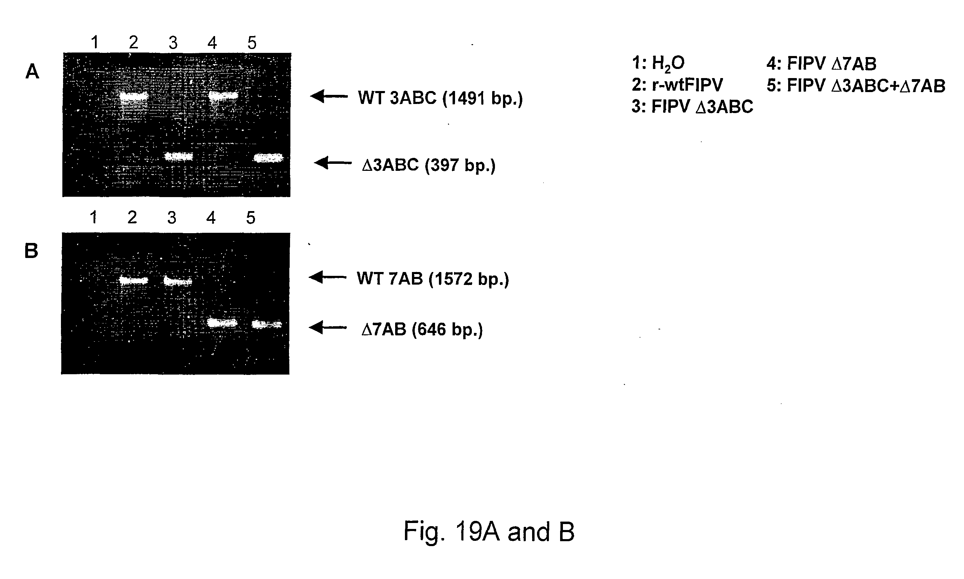

Procedure and Results: To evaluate whether the intended deletions indeed occurred in the various FIPV deletion mutants, RT-PCR on the genomic viral RNA's was performed focussing on the 3ABC and the 7AB region. The primers used and DNA sizes expected are indicated in Table 2 and Fig. 18 (right side). The results of the RT-PCR analyses are shown in Fig. 19. Fig. 19A reveals that the recombinant wild-type virus (r-wtFIPV) and FIPVΔ7AB are each carrying the 3ABC region whereas this region is lacking in the viruses FIPVΔ3ABC and FIPVΔ3ABC+Δ7AB, as judged by the sizes of the amplified fragments. Fig. 19B demonstrates that r-wtFIPV and FIPVΔ3ABC are still carrying the 7AB region whereas the viruses FIPVΔ7AB and FIPVΔ3ABC+Δ7AB are lacking this region. The 397 bp and 646 bp fragments, indicative of a 3ABC and 7AB deletion, respectively, were cloned and sequenced which confirmed the expected DNA sequences.

Conclusion: The deletion viruses had precisely the intended genomic deletions.

### II.2.d. Growth characteristics of FIPVs lacking genes

Aim: Check cell tropism of the deletion viruses and evaluate their in *vitro* growth.

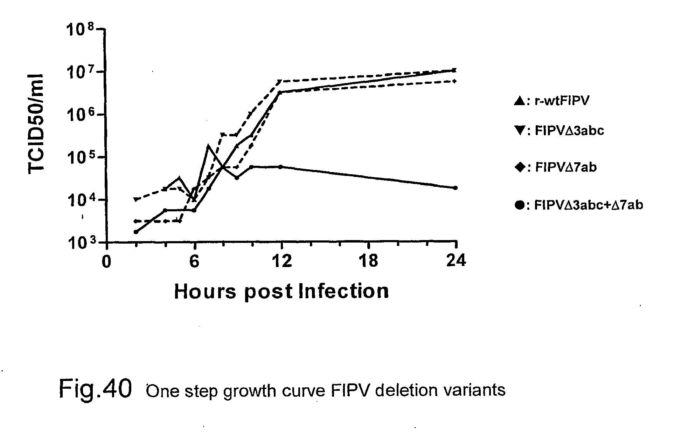

Results: All 4 recombinant viruses were inoculated onto mouse LR7 cells but failed to produce any cytopathic effects. They did, however, grow efficiently in feline FCWF cells, as expected. The viruses r-wtFIPV, FIPVA3ABC and FIPVA7AB reached titers that were similar to those obtained with the parent FIPV strain 79-1146 on these cells. The titers were all in the order of 5.10⁷ PFU/ml (Fig. 40). However, the double mutant FIPVΔ3ABC+Δ7AB grew less efficient; titers obtained with this virus were generally 1 to 2 log units lower (Fig. 40).

Conclusion: The sequences comprising the gene clusters 3ABC and 7AB are not essential for the viability of FIPV. Apparently, neither the nucleotide sequences nor the proteins encoded by the genes are essential for the replication of the virus.

### II.2.e. Virulence of recombinant viruses in cats

Aim: Establishing whether the genetic deletions affect virulence.

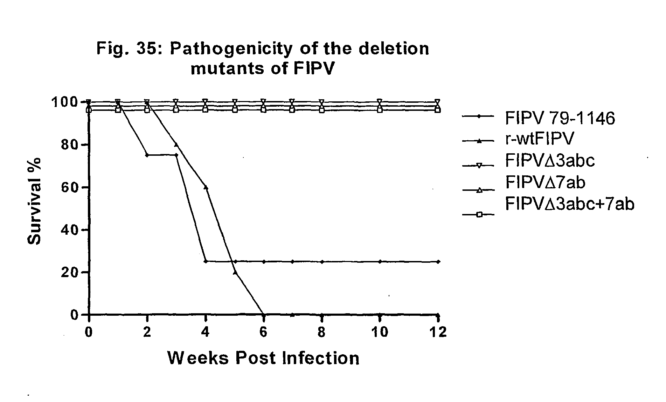

Procedure and Results: The recombinant deletion viruses were characterized in their natural host, the cat. To this purpose, 24 SPF cats (5 months old) were placed into 5 groups and inoculated oronasally (100 pfu) with FIPV strain 79-1146 (n=4), r-wtFIPV (n=5), FIPVΔ3ABC (n=5), FIPVΔ7AB (n=5) and FIPVΔ3ABC+Δ7AB (n=5), respectively, and followed for (at least) 3 months. Clinical disease signs were scored as shown in Table 5. Inoculation of the cats with the deletion variants did not induce clinical signs of disease. Rather, all cats remained totally healthy throughout the experiment (Table 6 and Fig. 35). In contrast, infection with the wild type controls FIPV 79-1146 and r-wtFIPV induced a rapid onset of clinical disease characterized by depression, anorexia, jaundice, weight loss and leukopenia (Table 6). Three out of four and five out of five cats inoculated with FIPV 79-1146 and r-wtFIPV, respectively, had to be euthanized due to advanced symptoms of FIP between day 14 and 42 after infection (Fig. 35).

Conclusions: 1) FIPV 79-1146 and its wild type recombinant equivalent r-wtFIPV are equally virulent; and 2) Viruses with deletions of the sequences specifying the genes 3abc or 7ab or of the combination of some or all these genes exhibit a significantly attenuated phenotype in cats.

### II.2.f. Immune response induced by recombinant viruses

Aim: Determining the FIPV-neutralizing activity in cat sera.

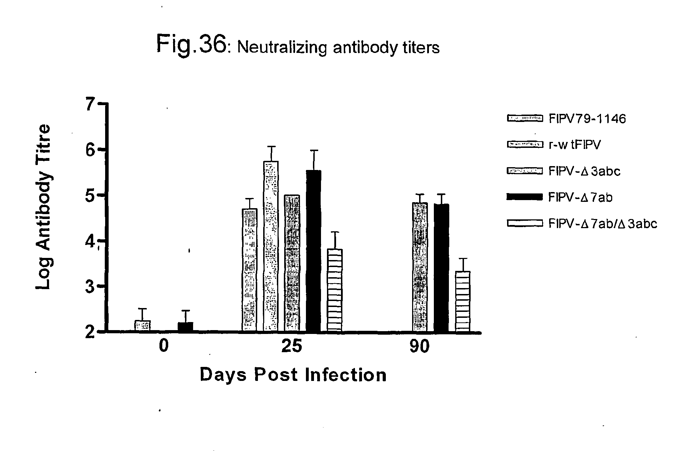

Procedure and Results: FIPV-specific antibody responses of the cats inoculated with the deletion viruses were characterized. To this purpose, blood samples were obtained at days 0, 21 and 90 post infection, and heat-inactivated sera were prepared and incubated with FIPV 79-1146 (10.000 PFU) after which the FIPV-neutralizing activity was determined using FCWF cells in a 96-well microplate assay. Titres were expressed as the reciprocal of the lowest dilution that no longer inhibited viral cytopathic effects. As expected, at day 0 none of the cat sera showed a significant FIPV-neutralizing activity. At day 21, all cats had seroconverted and showed high titers of neutralizing antibodies. The titers observed in cats inoculated with FIPV 79-1146, r-wtFIPV, FIPVΔ3ABC and FIPVΔ7AB were comparable whereas the titers observed in FIPVΔ3ABC+Δ7AB infected cats were approximately 50 fold lower (Fig. 36). Overall, the titers remained high for at least 90 days (end of experiment).

Conclusion: Despite the absence of clinical disease signs, high titers of neutralizing antibodies are observed in cats that had been inoculated with FIPV deletion variants. This strongly suggests that the deletion viruses are viable and replicate in the cat leading to a strong immune response in the form of an antibody response.

### II.2.g. FIPV deletion viruses serve as attenuated, live vaccines

Aim: To study whether previous inoculation with the attenuated deletion variants protects the cats against a FIPV 79-1146 challenge.

Procedure and Results: The cats previously inoculated with the attenuated deletion variants were challenged oranasally with FIPV 79-1146 (100 pfu) at day 90. As a control group, 4 untreated cats of similar age were challenged identically. The control group showed a rapid onset of clinical disease characterized by depression, anorexia, jaundice, weight loss and leukopenia (day 7). A similar rapid onset of symptoms was observed with 3 out 5 cats previously inoculated with FIPVΔ3ABC+ΔAB, whereas all cats previously infected with FIPVΔ3ABC or FIPVΔ7AB remained generally healthy and without typical FIP symptoms throughout the experiment (Table 7), although 2 out 5 cats previously inoculated with FIPVΔ3ABC showed temporary weight loss.

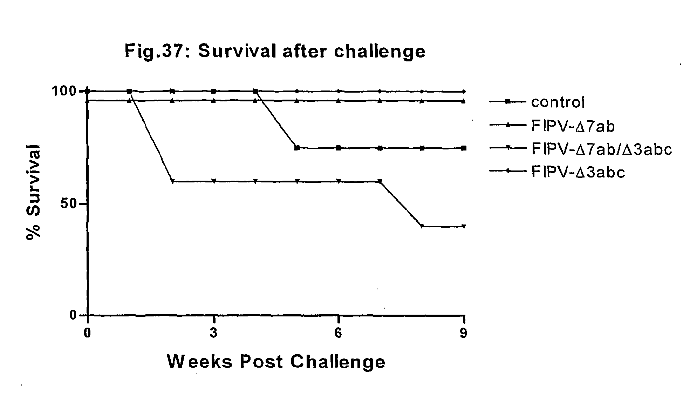

Due to advanced symptoms of FIP, one cat out of the control group had to be euthanized at day 32, whereas the other cats in the control group recovered from their initial FIP symptoms. A lethality score of 25% is lower than observed in the previous experiments. This is supposedly due to the advanced age of the cats which is known to lead to reduced susceptibility for FIPV. The three FIPVΔ3ABC+Δ7AB vaccinated cats with initial symptoms remained ill and were euthanized between days 11 and 56 (Fig 37). Apparently, the combined deletion of the two gene clusters 3ABC and 7A which we found to reduce the fitness of FIPVΔ3ABC+Δ7AB - as judged by its decreased *in vitro* growth - also affects the replication rate of the virus in the cats, as testified by the lower levels of neutralizing antibodies induced. Thus, the virus is over-attenuated and only capable of partially protecting against a FIPV challenge. Full protection would require a higher or repeated dose.

Conclusion: Prior vaccination with FIPVΔ3ABC or FIPVΔ7AB protects cats against disease caused by a FIPV challenge.

### II.3. Insertion and expression of foreign genes

Aim: Establish whether foreign genes can be inserted at different positions in the viral genome, either as an additional gene or replacing deleted non-essential genes; and establish whether these genes are expressed and whether they are stably maintained during *in vitro* passage of the virus.

### II.3.a. Construction of recombinant viruses carrying reporter genes

Aim: Generate FIPV 79-1146 viruses with foreign gene insertions.

Procedure: A virus was constructed containing a foreign reporter gene in its genome at the position of the 3abc genes. The reporter gene, renilla luciferase (RL), was placed under the transcriptional control of the IGS preceeding gene 3a. To delete the 3abc cluster and introduce the RL gene into the plasmid pBRDI1 combinations of primers 1244 (5'-GCCATTCTCATTGATAAC-3') and 1514 (5'-CTGAGTCTAGAGTAGCTAGCTAATGACTAATAAGTTTAG-3') and of 1245 (5'-GCTTCTGTTGAGTAATCACC-3') and 1513 (5'-GCTAGCTACTCTAGACTCAGGCGGTTCTAAAC-3') were used to generate fragments of 336 bp (A) and 1068 bp (B) via PCR, respectively. In primer 1513 and 1514, the underlined and bold sequences represent a *NheI* and *XbaI* restriction site, respectively. Fragments A and B were fused using the overlap between both fragments through primers 1514 and 1513 and amplified using primers 1244 and 1245, using SOE-PCR, resulting in a 1384 bp fragment (C). Fragment C was cloned into the pGEM-T Easy vector (Promega), resulting in pGEM-C. The RL gene derived from pRL-null (Promega) was introduced as a *NheI*/*XbaI* fragment into *NheI* and *XbaI* digested pGEM-C, resulting in pGEM-C+luc. Fragment C+luc was introduced as a *AflII* / *SnaBI* fragment into *AflII* and *SnaBI-* digested pBRDI1 resulting in pBRDI1Δ3ABC+luc.

After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis.

Conclusion: The foreign reporter gene renilla luciferase can be placed into the genome of the type I coronavirus FIPV. The intended recombinant viruses are viable.

### II.3.b. One-step growth of viruses

Aim: Compare the growth characteristics of the viruses with wild-type virus.

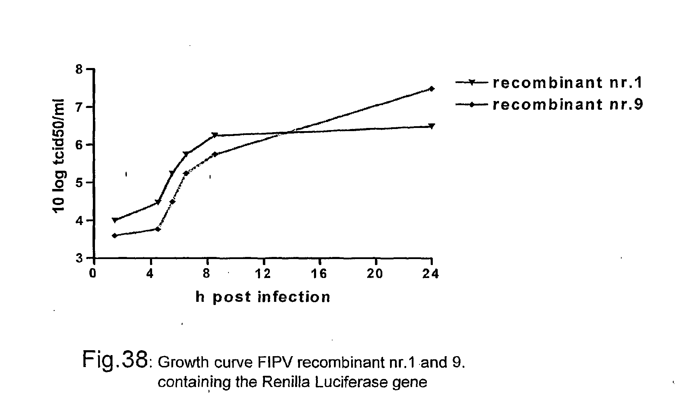

Procedure and Results: After two rounds of plaque purification virus stocks of 2 independently obtained recombinants under II.3.a were prepared, titrated and used for high m.o.i (m.o.i. of 8) infection of FCWF cells after which the viral infectivities in the culture media were monitored. The results are represented by the growth curves shown in Fig. 38. Both independently obtained recombinants grew to a 1 to 2 log lower titer as compared to that of recombinant wild-type virus.

Conclusion: The recombinant viruses with inserted expression cassette grew normally *in vitro* but their yields were affected.

### II.3.c. Expression of renilla luciferase

Aim: Establish whether the inserted expression cassette was functional.

Procedure and Results: Confluent monolayer cultures of FCWF cells were infected at a m.o.i. of 8 and the production of luciferase activity in the cells was monitored over time. RL expression in cells was measured by using the Dual-Luciferase Reporter Assay System (Promega) according to the manufacturer's instructions. RL was measured in relative light units (RLU) using a luminometer (Lumac Biocounter M2500 or Turner Designs Model TD-20/20).

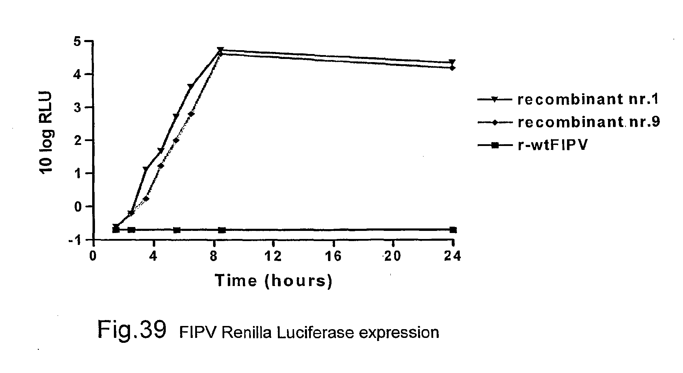

The results are shown graphically in Fig. 39. In contrast to the recombinant wild-type virus, the two recombinant luciferase gene containing viruses expressed high levels of luciferase activity, indicating that the renilla luciferase gene is functional. Expression started as early as 2 h post-infection. The highest expression level was reached at 9h post-infection.

Conclusion: Foreign genes can be expressed by coronaviruses by using the genetic space created by deletion of nonessential genes.

### II.4 Generation of multivalent FIPV-based vaccines

Aim: Development of multivalent vaccines based on a live attenuated FIPV strain as a vector.

Approach: Genes (or gene fragments) from other feline and canine pathogens encoding antigens known to induce protective immunity against these pathogens were selected. Expression cassettes of these genes were introduced into the FIPV genome in combination with attenuating deletions of non-essential genes. The expression cassettes contain the FIPV TRS in front of (parts of) the gene to be expressed.

### II.4.a. Construction of a multivalent Feline leukemia virus (FeLV) vaccine based on FIPV vector

Aim: Development of FeLV vaccine based on a live attenuated FIPV strain as a vector.

Procedure: (Parts of) the protection related FeLV genes gag and env were placed into expression cassettes and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the FeLV gag and env genes was confirmed by immunoprecipitation analysis.

Conclusion: (Parts of) the protection related FeLV genes *gag* and *env* can be introduced into and expressed by a live attenuated FIPV strain. These recombinants can therefore function as a multivalent vaccine against a Feline leukemia virus and FIPV infection.

### II.4.b. Construction of a multivalent Feline immunodeficiency virus (FIV) vaccine based on a FIPV vector

Aim: Development of FIV vaccine based on a live attenuated FIPV strain as a vector.

Procedure: (Parts of) the protection related FIV genes *gag* and env were placed into expression cassettes and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the FIV *gag* and env genes was confirmed by immunoprecipitation analysis.

Conclusion: (Parts of) the protection related FIV genes gag and *env* can be introduced into and expressed by a live attenuated FIPV strain. These recombinant viruses can therefore function as a multivalent vaccine against a Feline immunodeficiency virus and FIPV infection.

### II.4.c. Construction of a multivalent Feline calicivirus (FCV) vaccine based on a FIPV vector

Aim: Development of FCV vaccine based on a live attenuated FIPV strain as a vector.

Procedure: (Parts of) the protection related FCV capsid gene was placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the FCV capsid gene was confirmed by immunoprecipitation analysis.

Conclusion: (Parts of) the protection related FCV capsid gene can be introduced into and expressed by a live attenuated FIPV strain. These recombinant viruses can therefore function as a multivalent vaccine against a Feline calicivirus and FIPV infection.

### II.4.d. Construction of a multivalent Feline panleucopenia virus (FPV) vaccine based on a FIPV vector

Aim: Development of FPV vaccine based on a live attenuated FIPV strain as a vector.

Procedure: (Parts of) the protection related R3 gene, encoding the VP1 and VP2 capsid proteins was placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of (parts of ) the R3 gene was confirmed by immunoprecipitation analysis.

Conclusion: (Parts of) the protection related R3 gene can be introduced into and expressed by a live attenuated FIPV strain. These recombinant viruses can therefore function as a multivalent vaccine against a Feline panleucopenia virus and FIPV infection.

### II.4.e. Construction of a multivalent Feline herpes virus (FHV) vaccine based on a FIPV vector

Aim: Development of FHV vaccine based on a live attenuated FIPV strain as a vector.

Procedure: (Parts of) the protection related gB, gC, gD, gE, gH, gI, gK, gL, gM, gM, ICP0, ICPland ICP4 feline herpes virus genes were placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the inserted FHP genes was confirmed by immunoprecipitation analysis.

Conclusion: (Parts of) the protection related gB, gC, gD, gE, gH, gI, gK, gL, gM, ICP0, ICP1and ICP4 feline herpes virus genes can be introduced and expressed in a live attenuated FIPV strain. These recombinant viruses can therefore function as a multivalent vaccine against Feline herpes virus and FIPV.

### II.4.f. Construction of a multivalent FIPV serotype I and II vaccine based on a FIPV serotype II vector

Aim: Development of a vaccine protecting both against serotype I and against II feline coronaviruses, based on a live attenuated FIPV serotype II strain as a vector.

Procedure: (Parts of) the protection related spike gene of a serotype I feline coronavirus of which the region encoding the signal sequence was deleted was placed into an expression cassette and subsequently introduced into pBRDI1Δ3ABC and pBRDI1Δ7AB, respectively. For example: in one case a spike gene construct was used from which the region encoding the signal sequence had been deleted; in another case a truncated spike gene was used, from which the region encoding the transmembrane domain + endodomain had been deleted. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the inserted the serotype I spike gene construct was confirmed by immunoprecipitation analysis.

Conclusion: (Parts of) the protection related spike gene of feline coronavirus serotype I can be introduced and expressed in a live attenuated FIPV serotpye II strain and can therefore function as a multivalent vaccine against both serotype I and II feline coronaviruses.

### II.5. Generation of recombinant viruses with rearranged gene order

Aim: Development of a FIPV vaccine in which deletion of non-essential genes is combined with a rearranged gene order by moving the N gene upstream of the S gene in the FIPV genome.

Procedure and Results: Capped, run-off donor transcripts were synthesized from *NotI*-linearized pBRDI1Δ3ABC, pBRDI1Δ7AB and pBRDI1Δ3ABC+ Δ7AB vectors in which the N gene, together with its TRS, was inserted at a position upstream of the S. The donor transcripts were introduced into murine LR7 cells (80 cm² flask), that had been infected before with mFIPV (m.o.i. of 0.4), by electroporation (Gene pulser electroporation apparatus, Biorad, 2 consecutive pulses; 0.85 kV/50 microF). The electroporated cells were co-cultured in a 25cm² culture flask with feline FCWF cells (50% confluency). After 24 h incubation at 37°C massive syncytia could be detected in the cell cultures. Deletion mutant viruses with rearranged genomes released into the mixed cell culture supernatant were purified by two rounds of plaque purification on FCWF cells.

Conclusion: Deletion mutant FIPVs with rearranged gene order could be generated. These viruses can function as live attenuated FIPV vaccines. By virtue of their rearranged gene order these viruses represent safer vaccines because of their reduced ability to generate viable progeny by recombination with viruses circulating in the field.

### II.6 Generation of FIPV based vaccines against canine pathogens

General aim: Development of vaccines based on a live attenuated FIPV serotype II strain as a vector against canine pathogens.

Approach: Since type II feline coronaviruses express canine coronavirus-like spike proteins (2a), such feline coronavirus vectors can be used as vaccines in canines. Therefore, the live attenuated FIPV vaccine that we developed can also be used for vaccination of canines against canine coronavirus (CCV). Furthermore, multivalent vaccines can be generated by introducing expression cassettes of protection related genes of other canine pathogens into the FIPV genome in combination with attenuating deletions of non-essential genes and with genome rearrangements. The expression cassettes contain the FIPV TRS in front of (parts of) the gene to be expressed.

### II.6.a. Generation of FIPV based vaccine against canine distemper

Aim: Development of canine distemper vaccine based on a live attenuated FIPV strain as a vector.

Procedure: (Parts of) the protection related H and F genes were placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of (parts of) the H and F genes was confirmed by immunoprecipitation analysis.

Conclusion: (Parts of) the protection related H and F genes of canine distemper virus can be introduced into and expressed by a live attenuated FIPV strain. These recombinant viruses can therefore function as a vaccine against canine distemper virus and CCV infection.

### II.6.b. Generation of FIPV based vaccine against canine parvo disease

Aim: Development of canine parvovirus vaccine based on a live attenuated FIPV strain as a vector.

Procedure: (Parts of) the protection related R3 gene, encoding the VP1 and VP2 capsid proteins were placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of (parts of) the R3 gene was confirmed by immunoprecipitation analysis.

Conclusion: (Parts of) the protection related R3 gene of canine parvovirus can be introduced into and expressed by a live attenuated FIPV strain. These recombinant viruses can therefore function as a vaccine against canine parvovirus disease and CCV infection.

### II.6.c. Generation of FIPV based vaccine against infectious canine hepatitis

Aim: Development of canine adenovirus 1 vaccine based on a live attenuated FIPV strain as a vector.

Procedure: (Parts of) the protection related structural protein genes of canine adenovirus 1 were placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of (parts of) the protection related structural protein genes of canine adenovirus 1 was confirmed by immunoprecipitation analysis.

Conclusion: (Parts of) the protection related structural protein genes of canine adenovirus 1 can be introduced into and expressed by a live attenuated FIPV strain. These recombinant viruses can therefore function as a vaccine against infectious canine hepatitis and CCV infection.

### II.6.d. Generation of FIPV based vaccine against hemorrhagic disease of pups

Aim: Development of canine herpesvirus 1 vaccine based on a live attenuated FIPV strain as a vector.

Procedure: (Parts of) the protection related gB, gC, gD, gE, gH, gI, gK, gL, gM, gM, ICP0, ICPland ICP4 canine herpes virus genes were placed into an expression cassette and subsequently introduced into pBRDI1Δ3ABC and pBRDI1Δ7AB, respectively. After confirmation of all constructs by restriction and sequence analysis, recombinant viruses were generated by RNA-RNA recombination between transcription vector run-off transcripts and the mFIPV genome as described above. The resulting viruses were genetically confirmed by RT-PCR analysis. Expression of the inserted genes was confirmed by immunoprecipitation analysis.

Conclusion: (Parts of) the protection related gB, gC, gD, gE, gH, gI, gK, gL, gM, ICP0, ICPland ICP4 canine herpes virus genes can be introduced and expressed in a live attenuated FIPV strain. These recombinant viruses can therefore function as a multivalent vaccine against canine herpes virus 1 and CCV.

### III. Transmissible gastro-enteritis virus (TGEV)

### III.1. Generation of a live attenuated vaccine against TGEV

Aim: Development of a live attenuated vaccine against TGEV.

Approach: To this aim recombinant TGEV deletion mutant viruses were generated that lack the non-essential genes 3ab and/or 7.

Procedure: Recombinant viruses were generated as described by Almazan *et al.* (2000). From pBAC-TGEV^{FL}, an infectious TGEV cDNA clone placed behind a CMV promoter in pBeloBAC11, the 3ab and 7 genes were deleted via standard cloning techniques, leaving the surrounding open reading frames and transcription regulatory sequences intact. Epithelial swine testis (ST) cells were transfected with this pBAC-TGEV^{FL} derivative which lacks the 3ab and 7 genes (pBAC-TGEV^{FL)}). Recombinant TGEV viruses were plaque purified and characterised by RT-PCR for the absence of the 3ab and/or 7 genes. Large amounts of the recombinant TGEV deletion viruses were generated by infecting ST cells.

Conclusion: Recombinant TGEV was generated that lacked the genes 3ab and 7. These viruses can function as a live attenuated vaccine against TGEV.

### III.2 Generation of multivalent TGEV-based vaccines

Aim: Development of multivalent vaccines based on a live attenuated TGEV as a vector.

Approach: Expression cassettes of protection related genes of porcine pathogens were introduced into the TGEV genome in combination with attenuating deletions of non-essential genes and genome rearrangements. The expression cassettes contain the TGEV TRS in front of (parts of) the gene to be expressed.

### III.2.a. Construction of a multivalent Porcine Parvovirus (PPV) vaccine based on a TGEV vector

Aim: Development of a vaccine based on a live attenuated TGEV strain as a vector.

Procedure: (Parts of) the protection related R3 gene, encoding the VP1 and VP2 capsid proteins, was placed into an expression cassette and subsequently introduced into a pBAC-TGEV^{FL} derivative which lacks genes 3ab and 7. After confirmation of all constructs by restriction and sequence analysis, recombinant viruses were generated by transfecting ST cells with this pBAC-TGEV^{FL} deletion derivative which contains (parts of) the protection related R3 gene. The resulting viruses were genetically confirmed by RT-PCR analysis. Expression of the inserted R3 gene was confirmed by immunoprecipitation analysis.

Conclusion: (Parts of) the protection related R3 gene can be introduced into and expressed by a live attenuated TGEV strain. These recombinant viruses can therefore function as a multivalent vaccine against a Porcine Parvovirus and TGEV infection.

### III.2.b. Construction of a multivalent swine influenza virus vaccine based on a TGEV vector

Aim: Development of a swine influenza virus vaccine based on a live attenuated TGEV strain as a vector.

Procedure: (Parts of) the protection related hemagglutinin (HA) and neuraminidase (NA) genes were placed into an expression cassette and subsequently introduced into a pBAC-TGEV^{FL} derivative which lacks the genes 3ab and 7. After confirmation of all constructs by restriction and sequence analysis, recombinant viruses were generated by transfecting ST cells with this pBAC-TGEV^{FL} deletion derivative which contains (parts of) the protection related hemagglutinin (HA) and neuraminidase (NA) genes. The resulting viruses were genetically confirmed by RT-PCR analysis. Expression of the inserted hemagglutinin (HA) and neuraminidase (NA) genes was confirmed by immunoprecipitation analysis.

Conclusion: (Parts of) the protection related hemagglutinin (HA) and neuraminidase (NA) genes can be introduced into and expressed by a live attenuated TGEV strain. These recombinant viruses can therefore function as a multivalent vaccine against a swine influenza virus and TGEV infection.

### III.2.c. Construction of a multivalent African swine fever virus vaccine based on a TGEV vector

Aim: Development of an African swine fever virus vaccine based on a live attenuated TGEV strain as a vector.

Procedure: (Parts of) the protection related structural protein encoding genes were placed into an expression cassette and subsequently introduced into a pBAC-TGEV^{FL} derivative which lacks the genes 3ab and 7. After confirmation of all constructs by restriction and sequence analysis, recombinant viruses were generated by transfecting ST cells with this pBAC-TGEV^{FL} deletion derivative which contains (parts of) the protection related structural-protein encoding genes. The resulting viruses were genetically confirmed by RT-PCR analysis. Expression of the structural-protein encoding genes was confirmed by immunoprecipitation analysis.

Conclusion: (Parts of) the protection related structural protein encoding genes can be introduced into and expressed by a live attenuated TGEV strain. These recombinant viruses can therefore function as a multivalent vaccine against an African swine fever virus and TGEV infection.

### III.2.d. Construction of a multivalent Porcine circovirus type 2 vaccine based on a TGEV vector

Aim: Development of a Porcine circovirus type 2 vaccine based on a live attenuated TGEV strain as a vector.

Procedure: (Parts of) the protection related C1, C2, V1 and V2 genes of the Porcine circovirus type 2 were placed into an expression cassette and subsequently introduced into a pBAC-TGEV^{FL} derivative which lacks the genes 3ab and 7. After confirmation of all constructs by restriction and sequence analysis, recombinant viruses were generated by transfecting ST cells with this pBAC-TGEV^{FL} deletion derivative which contains (parts of) the protection related C1, C2, V1 and V2 genes of the Porcine circovirus type 2. The resulting viruses were genetically confirmed by RT-PCR analysis. Expression of the C1, C2, V1 and V2 genes of the Porcine circovirus type 2 was confirmed by immunoprecipitation analysis.

Conclusion: (Parts of) the protection related C1, C2, V1 and V2 genes of the Porcine circovirus type 2 can be introduced into and expressed by a live attenuated TGEV strain. These recombinant viruses can therefore function as a multivalent vaccine against a Porcine circovirus type 2, and TGEV infection.

### III.2.e. Construction of a multivalent Porcine respiratory and reproductive syndrome virus vaccine based on a TGEV vector

Aim: Development of a Porcine respiratory and reproductive syndrome virus vaccine based on a live attenuated TGEV strain as a vector.

Procedure: (Parts of) the protection related ORF2 to ORF7 of the Porcine respiratory and reproductive syndrome virus were placed into an expression cassette and subsequently introduced into a pBAC-TGEV^{FL} derivative which lacks the genes 3ab and 7. After confirmation of all constructs by restriction and sequence analysis, recombinant viruses were generated by transfecting ST cells with this pBAC-TGEV^{FL} deletion derivative which contains (parts of) the protection related ORF2 to ORF7 of the Porcine respiratory and reproductive syndrome virus. The resulting viruses were genetically confirmed by RT-PCR analysis. Expression of ORF2 to ORF7 of the Porcine respiratory and reproductive syndrome virus was confirmed by immunoprecipitation analysis.

Conclusion: (Parts of) the protection related ORF2 to ORF7 of the Porcine respiratory and reproductive syndrome virus can be introduced into and expressed by a live attenuated TGEV strain. These recombinant viruses can therefore function as a multivalent vaccine against a Porcine respiratory and reproductive syndrome virus and TGEV infection.

### III.2.e. Construction of a multivalent foot-and-mouth disease virus vaccine based on a TGEV vector

Aim: Development of a food-and-mouth disease virus vaccine based on a live attenuated TGEV strain as a vector.

Procedure: (Parts of) the protection related sequences encoding VP1 to VP4 of the foot-and-mouth disease virus were placed into an expression cassette and subsequently introduced into a pBAC-TGEV^{FL} derivative which lacks the genes 3ab and 7. After confirmation of all constructs by restriction and sequence analysis, recombinant viruses were generated by transfecting ST cells with this pBAC-TGEV^{FL} deletion derivative which contains (parts of) the protection related sequences encoding VP1 to VP4 of the food-and-mouth disease virus. The resulting viruses were genetically confirmed by RT-PCR analysis. Expression of the sequences encoding VP1 to VP4 of the foot-and-mouth disease virus was confirmed by immunoprecipitation analysis.

Conclusion: (Parts of) the protection related sequences encoding VP1 to VP4 of the foot-and-mouth disease virus can be introduced into and expressed by a live attenuated TGEV strain. These recombinant viruses can therefore function as a multivalent vaccine against a foot-and-mouth disease virus and TGEV infection.

### III.3. Generation of recombinant viruses with rearranged gene order

Aim: Development of a TGEV vaccine in which deletion of non-essential genes is combined with a rearranged gene order by moving the N gene upstream of the S gene in the TGEV genome.

Procedure and Results: Recombinant viruses were generated as described by Almazan et al. (2000). From pBAC-TGEV^{FL}, an infectious TGEV cDNA clone placed behind a CMV promoter in pBeloBAC11, the 3ab and 7 genes were deleted via standard cloning techniques, leaving the surrounding open reading frames and transcription regulatory sequences intact. In addition the N gene was cloned to a position in the genome upstream of the S gene. Epithelial swine testis (ST) cells were transfected with this pBAC-TGEV^{FL} derivative which lacks the 3ab and 7 genes and has a rearranged genome. Recombinant TGEV viruses were plaque purified and characterized by RT-PCR for the absence of the 3ab and/or 7 genes. Large amounts of the TGEV deletion recombinants were generated by infecting ST cells.

Conclusion: Recombinant TGEV was generated that lacked the genes 3ab and 7, and which has a rearranged gene order. These viruses function as a live attenuated vaccine against TGEV, as well as against other porcine pathogens when genes encoding relevant antigens are appropriately incorporated.

### IV. Avian Infectious Bronchitis Virus (IBV)

### IV.1. Generation of a live attenuated vaccine against IBV

Aim: Development of a live attenuated vaccine against IBV.

Approach: Recombinant IBV deletion mutant viruses were generated that lack the non-essential genes 3a/b and/or 5a/b. In order to acquire vaccine viruses that protect against multiple IBV serotypes, spike gene constructs derived from such different viruses were incorporated.

Procedure: Recombinant viruses were generated as described by Casais et al. (2001). Infectious IBV cDNA clones were assembled in the vaccinia virus genome by using sequential *in vitro* ligation of cDNA fragments derived from pFRAG1, pFRAG2, and pFRAG3 derived plasmids, followed by direct cloning into the genome of vaccinia virus vNotI/tk as described (1a). Genes 3a/b and 5a/b were removed from pFRAG3, by PCR mutagenesis leaving the surrounding open reading frames and transcription regulatory sequences intact, resulting in pFRAG3Δ. The S gene in pFRAG3Δ could be replaced by S genes derived from different IBV serotypes by using RT-PCR. Recombinant vaccinia viruses were generated by recombination between the fowlpox virus HP 1.441 and the vNotI/tk-IBV in *vitro* ligation mixture. Recombinant viruses were plaque purified and characterized by PCR and Southern blot analysis. Finally, infectious IBV lacking genes 3a/b and/or 5a/b was generated as follows: Chick kidney (CK) cells were infected with recombinant fowlpox virus expressing T7 RNA polymerase. Subsequently cells were transfected with DNA isolated from the recombinant vaccinia virus containing the IBV cDNA clone lacking genes 3a/b and 5a/b. At 3 days post-infection the culture medium was collected and used to infect CK cells to generate large amounts of recombinant IBV. The recombinant viruses were genetically confirmed by RT-PCR analysis.

Conclusion: Recombinant IBV was generated that lacked the genes 3a/b and/or 5a/b. These viruses can function as a live attenuated vaccine against IBV. By replacing the S gene, recombinant IBVs could be obtained that function as live attenuated vaccines against different IBV serotypes.

### IV.2. Generation of multivalent IBV-based vaccines

Aim: Development of multivalent vaccine based on a live attenuated IBV strain as a vector.

Approach: Expression cassettes of protection related genes of chicken pathogens were introduced into the IBV genome in combination with attenuating deletion of non-essential genes. The expression cassettes contain the IBV TRS (CTTAACAA) in front of (parts of) the gene to be expressed.

### IV.2.aI. Construction of a multivalent vaccine based on an IBV vector that protects against more than one IBV serotype.

Aim: Development of a IBV vaccine based on a live attenuated IBV strain that lacks non-essential genes and protects against more than one serotype.

Procedure: (Parts of) the protection related IBV S gene from a different IBV serotype were placed into expression cassettes, and subsequently introduced into FRAG3Δ. The largest construct contained a complete extra copy of the S gene except for the sequence encoding the signal peptide; another construct had a carboxy-terminally truncated S gene lacking the code for the transmembrane domain and endodomain. After confirmation of all the constructs by restriction and sequence analysis, recombinant viruses were generated as described above. The recombinant viruses were genetically confirmed by RT-PCR analysis. The proper expression of the heterologous S gene constructs was verified by immunoprecipitation analysis with type-specific antisera using radiolabeled recombinant virus infected cell lysates. Conclusion: (Parts of) the protection related IBV S gene from a different IBV serotype can be introduced in and expressed from a live attenuated IBV strain. These recombinant viruses can therefore function as multivalent vaccines to protect against infection by more than one IBV serotype.

### IV.2.aII. Construction of a multivalent vaccine based on an IBV vector that protects against Newcastle Disease.

Aim: Development of a Newcastle Disease Virus (avian paramyxovirus type I; APMV-1) vaccine based on a live attenuated IBV strain that lacks non-essential genes.

Procedure: (Parts of) the protection related APMV-1 genes HN and F were placed in expression cassettes, and subsequently introduced into FRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related APMV-1 genes HN and F can be introduced in and expressed from a live attenuated IBV strain. The recombinant viruses can therefore function as a multivalent vaccine to protect against APMV-1 and IBV infections.

### IV.2.b. Construction of a multivalent vaccine based on an IBV vector that protects against Avian Influenza.

Aim: Development of an Avian Influenza virus vaccine based on a live attenuated IBV strain that lacks non-essential genes.

Procedure: (Parts of) the protection related Avian Influenza genes H and N were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related Avian Influenza genes H and N can be introduced in and expressed from a live attenuated IBV strain. These recombinant viruses can therefore function as a multivalent vaccine to protect against Avian Influenza and IBV infections.

### IV.2.c. Construction of a multivalent vaccine based on an IBV vector that protects against Chicken Anemia Virus (CAV) disease.

Aim: Development of a CAV vaccine based on a live attenuated IBV strain that lacks non-essential genes.

Procedure: (Parts of) the protection related CAV genes V1, V2, and V3 were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related CAV genes V1, V2, and V3 can be introduced in and expressed from a live attenuated IBV strain. These recombinant viruses can therefore function as a multivalent vaccine to protect against CAV and IBV infections.

### IV.2.d. Construction of a multivalent vaccine based on an IBV vector that protects against avian reovirus disease.

Aim: Development of an avian reovirus vaccine based on a live attenuated IBV strain that lacks non-essential genes.

Procedure: (Parts of) the protection related avian reovirus genes Φ1, Φ2, and 83 were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related avian reovirus genes Φ1, Φ2, and 83 can be introduced in and expressed from a live attenuated IBV strain. These recombinant viruses can therefore function as a multivalent vaccine to protect against avian reovirus and IBV infections.

### IV.2.e. Construction of a multivalent vaccine based on an IBV vector that protects against Infectious Bursal Disease.

Aim: Development of an Infectious Bursal Disease Virus (IBDV) vaccine based on a live attenuated IBV strain that lacks non-essential genes.

Procedure: (Parts of) the protection related IBDV gene VP2 were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related IBDV gene VP2 can be introduced in and expressed from a live attenuated IBV strain. These recombinant viruses can therefore function as a multivalent vaccine to protect against avian IBDV and IBV infections.

### IV.2.f. Construction of a multivalent vaccine based on an IBV vector that protects against Marek's Disease.

Aim: Development of a Gallid herpesvirus 2 (Marek's disease virus) vaccine based on a live attenuated IBV strain that lacks non-essential genes.

Procedure: (Parts of) the protection related Gallid herpesvirus 2 gene gB were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related Gallid herpesvirus 2 gene gB can be introduced in and expressed from a live attenuated IBV strain. These recombinant viruses can therefore function as a multivalent vaccine to protect against Gallid herpesvirus 2 and IBV infections.

### IV.2.g. Construction of a multivalent vaccine based on an IBV vector that protects against Infectious laryngotracheitis.

Aim: Development of a Gallid herpesvirus 1 (Infectious laryngotracheitis virus) vaccine based on a live attenuated IBV strain that lacks non-essential genes.

Procedure: (Parts of) the protection related Gallid herpesvirus 1 genes gB, gC, gD, gE, gH, gI, gK, gL, and gM were placed into expression cassettes, and subsequently introduced into pFRAG3Δ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related Gallid herpesvirus 1 genes can be introduced in and expressed from a live attenuated IBV strain. These recombinant viruses can therefore function as a multivalent vaccine to protect against Gallid herpesvirus 1 and IBV infections.

### IV.3. Generation of recombinant viruses with rearranged gene order

Aim: Development of an IBV vaccine in which deletion of non-essential genes is combined with a rearranged gene order by moving the N gene upstream of the S gene in the IBV genome.

Procedure: Recombinant viruses were generated as described by Casais et al. (2001). Infectious IBV cDNA clones were assembled in the vaccinia virus genome by using sequential *in vitro* ligation of cDNA fragments derived from pFRAG1, pFRAG2, and pFRAG3 derived plasmids, followed by direct cloning into the genome of vaccinia virus vNotI/tk as described (1a). Genes 3a/b and 5a/b were removed from pFRAG3, by PCR mutagenesis leaving the surrounding open reading frames and transcription regulatory sequences intact. In addition, the N gene together with its TRS was moved to a position upstream of the S gene resulting in pFRAG3Δrearranged. Recombinant vaccinia viruses were generated by recombination between the fowlpox virus HP1.441 and the vNotI/tk-IBV *in vitro* ligation mixture. Recombinant viruses were plaque purified and characterized by PCR and Southern blot analysis. Finally, infectious IBV lacking genes 3a/b and 5a/b and with a rearranged gene order was generated as follows: Chick kidney (CK) cells were infected with recombinant fowlpox virus expressing T7 RNA polymerase. Subsequently cells were transfected with DNA isolated from the recombinant vaccinia virus containing the IBV cDNA clone lacking genes 3a/b and 5a/b in combination with the rearranged gene order. At 3 days post-infection the culture medium was collected and used to infect CK cells to generate large amounts of recombinant IBV. The recombinant viruses were genetically confirmed by RT-PCR analysis.

Conclusion: Recombinant IBV was generated that lacked the genes 3a/b and 5a/b in combination with a rearranged gene order. These viruses function as a live attenuated vaccine against IBV. When combined with S gene constructs from other IBV serotypes and/or gene constructs from other avian pathogens additional, multivalent vaccines can be generated.

### V. Human Coronavirus (HCoV) strain 229E (HCoV-229E)

### V.1. Generation of a live vaccine based on attenuated HCoV-229E

Aim: Development of a live attenuated vaccine against HCoV-229E.

Approach: Recombinant HCoV deletion mutant viruses were generated that lack the non-essential genes 4a/b.

Procedure: Recombinant viruses were generated essentially as described by Thiel et al. (2001). Infectious HCoV cDNA clones were assembled in the vaccinia virus genome. Vaccinia virus vHCoV-vec-1 contains a 22.5 kbp cDNA fragment encoding the 5'end of the HCoV genome. This fragment was removed from the vaccinia genome by digestion with Bsp120I, digested with MluI and ligated to a cDNA fragment of pMEΔ that was obtained by digestion with MluI/EagI. pMEΔ contains a cDNA fragment that encodes the 3'end of the HCoV genome, but lacks gene 4a/b, without disturbing the other genes or transcription regulatory sequences. pMEΔ was derived from pME by using conventional PCR mutagenesis methods. The ligation products were ligated to vNotI/tk vaccinia virus DNA. Recombinant vaccinia viruses were generated as described above. Recombinant viruses were plaque purified and characterized by PCR and Southern blot analysis. Finally, infectious HCoV lacking genes 4a/b was generated as follows: Chick kidney (CK) cells were infected with recombinant fowlpox virus expressing T7 RNA polymerase. Subsequently cells were transfected with DNA isolated from the recombinant vaccinia virus containing the HCoV cDNA clone lacking genes 4a/b. At 3 days post-infection the culture medium was collected and used to infect human long fibroblast cells (MRC-5) to generate large amounts of recombinant HCoV. The recombinant viruses were genetically confirmed by RT-PCR analysis.

Conclusion: Recombinant HCoV was generated that lacked the genes 4a/b. These viruses can function as a live attenuated vaccine against HCoV-229E.

### V.2. Generation of a live attenuated vaccine against HCoV strain OC43

Aim: Development of a live attenuated vaccin against HCoV-OC43.

Approach: Recombinant HCoV deletion mutant viruses were generated that lack the non-essential genes 4a/b, and contain a hybrid S protein gene, which encodes the ectodomain of HCoV-OC43.

Procedure: Recombinant viruses were generated essentially as described by Thiel et al. (2001). Infectious HCoV cDNA clones were assembled in the vaccinia virus genome. Vaccinia virus vHCoV-vec-1 contains a 22.5 kbp cDNA fragment encoding the 5'end of the HCoV genome. This fragment was removed from the vaccinia genome by digestion with Bsp120I, digested with MluI and ligated to a cDNA fragment of pMEΔ-SOC43 that was obtained by digestion with MluI/EagI. pMEΔ-SOC43 contains a cDNA fragment that encodes the 3'end of the HCoV genome, lacks gene 4a/b, and contains a hybrid S protein gene, without disturbing the other genes or transcription regulatory sequences. The hybrid S protein gene contains the region of the S gene of HCoV-OC43 that encodes the S protein ectodomain, while the remainder of the gene is derived from the HCoV-229E gene. pMEΔ-SOC43 was derived from pMEΔ by using conventional (RT-)PCR mutagenesis methods. The ligation products were ligated to vNotI/tk vaccinia virus DNA. Recombinant vaccinia viruses were generated as described above. Recombinant viruses were plaque purified and characterized by PCR and Southern blot analysis. Finally, infectious HCoV lacking genes 4a/b was generated as follows: Chick kidney (CK) cells were infected with recombinant fowlpox virus expressing T7 RNA polymerase. Subsequently cells were transfected with DNA isolated from the recombinant vaccinia virus containing the HCoV cDNA clone lacking genes 4a/b. At 3 days post-infection the culture medium was collected and used to infect human long fibroblast cells (MRC-5) to generate large amounts of recombinant HCoV. The recombinant viruses were genetically confirmed by RT-PCR analysis.

Conclusion: Recombinant HCoV was generated that lacked the genes 4a/b. These viruses contain the ectodomain of the S protein of HCoV-OC43 and therefore function as a live attenuated vaccine against HCoV-OC43.

### V.3. Generation of multivalent HCoV -based vaccines

Aim: Development of multivalent vaccine based on a live attenuated HCoV strain as a vector.

Approach: Expression cassettes of protection related genes of human pathogens were introduced into the HCoV genome in combination with attenuating deletion of non-essential genes. The expression cassettes contain the HCoV TRS (TCTCAACT) in front of (parts of) the gene to be expressed.

### V.3.a. Construction of a multivalent vaccine based on an HCoV vector that protects against Respiratory Syncytial Virus (RSV)

Aim: Development of a RSV vaccine based on a live attenuated HCoV strain that lacks non-essential genes.

Procedure: (Parts of) the protection related RSV genes HN and F were placed into expression cassettes, and subsequently introduced into pMEΔ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related RSV genes HN and F can be introduced in and expressed from a live attenuated HCoV strain. These recombinant viruses can therefore function as a multivalent vaccine to protect against RSV and HCoV infections.

### V.3.b. Construction of a multivalent vaccine based on an HCoV vector that protects against rotavirus

Aim: Development of a rotavirus vaccine based on a live attenuated HCoV strain that lacks non-essential genes.

Procedure: (Parts of) the protection related rotavirus genes VP4, VP6 and VP7 were placed into expression cassettes, and subsequently introduced into pMEΔ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related rotavirus genes VP4, VP6 and VP7 can be introduced in and expressed from a live attenuated HCoV strain. These recombinant viruses can therefore function as a multivalent vaccine to protect against rotavirus and HCoV infections.

### V.3.c. Construction of a multivalent vaccine based on an HCoV vector that protects against Norwalk-like viruses

Aim: Development of a Norwalk-like virus vaccine based on a live attenuated HCoV strain that lacks non-essential genes.

Procedure: (Parts of) the protection related Norwalk-like virus capsid gene was placed into expression cassettes, and subsequently introduced into pMEΔ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related Norwalk-like virus capsid gene can be introduced in and expressed from a live attenuated HCoV strain. This recombinant virus can therefore function as a multivalent vaccine to protect against Norwalk-like virus and HCoV infections.

### V.3.d. Construction of a multivalent vaccine based on an HCoV vector that protects against influenza virus

Aim: Development of an influenza virus vaccine based on a live attenuated HCoV strain that lacks non-essential genes.

Procedure: (Parts of) the protection related influenza virus genes H and N were placed into expression cassettes, and subsequently introduced into pMEΔ. After confirmation of all the constructs by restriction and sequence analysis, recombinant viruses were generated as described above. The recombinant viruses were genetically confirmed by RT-PCR analysis. Expression of the foreign gene was confirmed by immunoprecipitation analysis.

Conclusion: (Parts of) the protection related influenza virus genes H and N can be introduced in and expressed from a live attenuated HCoV strain. These recombinant viruses can therefore function as a multivalent vaccine to protect against influenzavirus and HCoV infections.

### V.4. Generation of recombinant viruses with rearranged gene order

Aim: Development of a HCoV vaccine in which deletion of non-essential genes is combined with a rearranged gene order by moving the N gene upstream of the S gene in the HCoV genome.

Procedure: Recombinant viruses were generated essentially as described by Thiel et al. (2001). Infectious HCoV cDNA clones were assembled in the vaccinia virus genome. Vaccinia virus vHCoV-vec-1 contains a 22.5 kbp cDNA fragment encoding the 5'end of the HCoV genome. This fragment was removed from the vaccinia virus genome by digestion with Bsp120I, digested with MluI and ligated to a cDNA fragment of pMEΔrearranged that was obtained by digestion with MluI/EagI. pMEΔrearranged contains a cDNA fragment that encodes the 3'end of the HCoV genome, but lacks gene 4a/b, without disturbing the other genes or transcription regulatory sequences, and in which the N gene, together with its TRS, was positioned upstream of the S gene. pMEΔrearranged was derived from pME by using conventional PCR mutagenesis methods. The ligation products were ligated to vNotI/tk vaccinia virus DNA. Recombinant vaccinia viruses were generated as described above. Recombinant viruses were plaque purified and characterized by PCR and Southern blot analysis. Finally, infectious HCoV lacking genes 4a/b was generated as follows: Chick kidney (CK) cells were infected with recombinant fowlpox virus expressing T7 RNA polymerase. Subsequently cells were transfected with DNA isolated from the recombinant vaccinia virus containing the HCoV DNA clone lacking genes 4a/b in combination with the rearranged gene order. At 3 days post-infection the culture medium was collected and used to infect human long fibroblast cells (MRC-5) to generate large amounts of recombinant HCoV. The recombinant viruses were genetically confirmed by RT-PCR analysis.

Conclusion: Recombinant HCoV was generated that lacked the genes 4a/b in combination with a rearranged gene order. These viruses can function as a live attenuated vaccine against HCoV-229E. When combined with S gene constructs from HcoV-OC43 and/or gene constructs from other human pathogens additional, multivalent vaccines can be generated.

### References

1. Bredenbeek, P. J., C. J. Pachuk, A. F. Noten, J. Charite, W. Luytjes, S. R. Weiss, and W. J. Spaan. 1990. The primary structure and expression of the second open reading frame of the polymerase gene of the coronavirus MHV-A59; a highly conserved polymerase is expressed by an efficient ribosomal frameshifting mechanism. Nucleic Acids Res. 18:1825-32.
1a Casais, R., V. Thiel, S.G. Siddell, D. Cavanagh, and P. Britton. 2001. Reverse genetics system for the avian coronavirus infectious bronchitis virus. J. Virol. 75:12359-12369.
1c Almazan, F., J.M. Gonzalez, Z. Penzes, A. Izeta, E. Calvo, J. Plana-Duran, and L. Enjuanes. 2000. Engineering the largest RNA virus genome as an infectious bacterial artificial chromosome. Proc. Natl. Acad. Sci. USA 97:5516-5521.
1d Fischer, F., C.F. Stegen, C.A. Koetzner, and P. S. Masters. 1997. Analysis of a recombinant Mouse Hepatitis Virus Expressing a foreign gene reveals a novels aspect of coronavirus transcription. J. Virol. 71:5148-5160.
2. Fischer, F., D. Peng, S. T. Hingley, S. R. Weiss, and P. S. Masters. 1997. The internal open reading frame within the nucleocapsid gene of mouse hepatitis virus encodes a structural protein that is not essential for viral replication. J. Virol. 71:996-1003.
2a. Herrewegh, A.A., I. Smeenk, M.C. Horzinek, P.J.M. Rottier, and R.J. de Groot. 1998. Feline coronavirus type II strains 79-1683 and 79-1146 originate from a double recombination between feline coronavirus type I and canine coronavirus. J Virol. 72:4508-4514.
3. Hsue, B., T. Hartshorne, and P. Masters. 2000. Characterization of an essential RNA secondary structure in the 3' untranslated region of the murine coronavirus genome. J. Virol. 74:6911-6921.
4. Hsue, B., and P. S. Masters. 1999. Insertion of a new transcriptional unit into the genome of mouse hepatitis virus. J. Virol. 73:6128-6135.
5. Jacobs, L., W. Spaan, M. Horzinek, and B. van der Zeijst. 1981. Synthesis of subgenomic mRNA's of mouse hepatitis virus is initiated independently: evidence from UV transcription mapping. J. Virol. 39:401-406.
6. Jendrach, M., V. Thiel, and S. Siddell. 1999. Characterization of an internal ribosome entry site within mRNA 5 of murine hepatitis virus. Arch. Virol. 144:921-933.
7. Kuo, L., G. Godeke, M. Raamsman, P. Masters, and P. Rottier. 2000. Retargeting of coronavirus by substitution of the spike glycoprotein ectodomain: crossing the host cell species barrier. J. Virol. 74:1393-1406.
8. Leibowitz, J., K. Wilhelmsen, and C. Bond. 1981. The virus-specific intracellular RNA species of two murine coronaviruses: MHV-a59 and MHV-JHM. Virology. 114:39-51.
9. Masters, P. S. 1999. Reverse genetics of the largest RNA viruses. 53:245-264,. Adv. Virus Res. 53:245-264.
10. Masters, P. S., C. A. Koetzner, C. A. Kerr, and Y. Heo. 1994. Optimization of targeted RNA recombination and mapping of a novel nucleocapsid gene mutation in the coronavirus mouse hepatitis virus. J Virol. 68:328-37.
10a. Thiel, V., J. Herold, B. Schelle, and S. Siddell. 2001. Infectious RNA transcribed in vivo from a cDNA copy of the human coronavirus genome cloned in vaccinia virus. J. Gen. Virol. 82, 1273-1281
11. de Vries, A.A.F, A.L.Glaser, M.J.B.Raamsman, C.A.M.de Haan, S.Sarnataro, G.-J.Godeke, and P.J.M.Rottier. 2000. Genetic manipulation of equine arteritis virus using full-length cDNA clones: separation of overlapping genes and expression of a foreign epitope. Virology 270:84-97.
12. Godeke, G.-J., C.A.M.de Haan, J.W.A.Rossen, H.Vennema, and P.J.M.Rottier. 2000. Assembly of spikes into coronavirus particles is mediated by the carboxy-terminal domain of the spike (S) protein. J.Virol. 74:1566-1571.
13. Vennema,H., G.-J.Godeke, J.W.A.Rossen, W.F.Voorhout, M.C.Horzinek, D.-J.E.Opstelten, and P.J.M.Rottier. 1996. Nucleocapsid-independent assembly of coronavirus-like particles by coexpression of viral envelope proteins. EMBO J. 15:2020-2028.

## Claims

1. An isolated or recombinant virus-like particle capable of replication, said particle derived from a coronavirus, wherein the genes for the structural proteins do not occur in the order 5'-S-E-M-N-3'.

2. A particle according to claim 1 from which at least a functional fragment from a nucleic acid encoding a viral gene product other than a polymerase or a structural protein N, M, E, or S, is deleted.

3. A particle according to anyone of claims 1 to 2 provided with at least one biologically active protein or fragment thereof associated with the surface of said virus-like particle other than the natural ectodomain of any one protein of the original corona virus.

4. A particle according to anyone of claims 1 to 2 provided with at least one biologically active protein or fragment thereof associated with the inside of said virus-like particle other than the natural endodomain of any one protein of the original corona virus.

5. A particle according to anyone of claims 1 to 2 provided with at least one functional targeting means associated with the surface of said virus-like particle other than the natural spike protein of the original corona virus.

6. A particle according to anyone of claims 1 to 5 wherein said particle is provided with a coronavirus genome wherein a foreign gene or parts thereof have been inserted.

7. A particle according to anyone of claims 1 to 6 which is attenuated.

8. A particle according to anyone of claims 1 to 7 which is a gene delivery vehicle.

9. A particle according to any of claims 1 to 7 which is an antigen or epitope delivery vehicle.

10. A composition comprising a particle according to any of claims 1 to 9 for therapeutic use.

11. A composition comprising a particle according to any of claims 1 to 9 and a pharmaceutically acceptable carrier for use as an immunogen or vaccine.

12. A composition comprising a particle according to any of claims 1 to 9 for diagnostic use.

## Patentansprüche

1. Isoliertes oder rekombinantes virusähnliches Partikel, das zur Replikation fähig ist, wobei das Partikel von einem Coronavirus stammt, wobei die Gene für die strukturellen Proteine nicht in der Anordnung 5'-S-E-M-N-3' auftreten.

2. Partikel nach Anspruch 1, von dem mindestens ein funktionelles Fragment aus einer Nucleinsäure, die ein anderes virales Genprodukt als eine Polymerase oder ein strukturelles Protein N, M, E oder S codiert, deletiert ist.

3. Partikel nach einem der Ansprüche 1 bis 2, das mit mindestens einem biologisch aktiven Protein oder Fragment davon versehen ist, das mit der Oberfläche des virusähnlichen Partikels assoziiert ist, das nicht die natürliche Ektodomäne eines beliebigen Proteins des ursprünglichen Coronavirus ist.

4. Partikel nach einem der Ansprüche 1 bis 2, das mit mindestens einem biologisch aktiven Protein oder Fragment davon versehen ist, das mit dem Inneren des virusähnlichen Partikels assoziiert ist, das nicht die natürliche Endodomäne eines beliebigen Proteins des ursprünglichen Coronavirus ist.

5. Partikel nach einem der Ansprüche 1 bis 2, das mit mindestens einem funktionellen Targeting-Mittel versehen ist, das mit der Oberfläche des virusähnlichen Partikels assoziiert ist, das nicht das natürliche Spike-Protein des ursprünglichen Coronavirus ist.

6. Partikel nach einem der Ansprüche 1 bis 5, wobei das Partikel mit einem Coronavirus-Genom versehen ist, in das ein fremdes Gen oder Teile davon insertiert wurden.

7. Partikel nach einem der Ansprüche 1 bis 6, das abgeschwächt ist.

8. Partikel nach einem der Ansprüche 1 bis 7, das ein Genabgabe-Vehikel ist.

9. Partikel nach einem der Ansprüche 1 bis 7, das ein Antigen- oder Epitopabgabe-Vehikel ist.

10. Zusammensetzung, die ein Partikel nach einem der Ansprüche 1 bis 9 umfasst, zur therapeutischen Verwendung.

11. Zusammensetzung, die ein Partikel nach einem der Ansprüche 1 bis 9 und einen pharmazeutisch verträglichen Träger umfasst, zur Verwendung als Immunogen oder Impfstoff.

12. Zusammensetzung, die ein Partikel nach einem der Ansprüche 1 bis 9 umfasst, zur diagnostischen Verwendung.

## Revendications

1. Une particule de type virus isolée ou recombinée capable de réplication, ladite particule dérivant d'un coronavirus, dans laquelle les gènes des protéines structurelles ne se présentent pas dans la succession 5'-S-E-M-N-8'-

2. Une particule selon la revendication 1, dont au moins un fragment fonctionnel d'un acide nucléique codant le produit d'un gène viral autre qu'une polymérase ou une protéine structurale N, M, E ou S est supprimé.

3. Une particule selon une quelconque des revendications 1 et 2, comportant au moins une protéine biologiquement active ou un fragment en dérivant associée à la surface de la dite particule de type virus autre que l'ectodomaine naturel d'une quelconque protéine du coronavirus d'origine.

4. Une particule selon une quelconque des revendications 1 et 2, comportant au moins une protéine biologiquement active ou un fragment en dérivant associée à l'intérieur de la dite particule de type virus autre que l'endodomaine naturel d'une quelconque protéine du coronavirus d'origine.

5. Une particule selon une quelconque des revendications 1 et 2, comportant au moins un moyen fonctionnel de ciblage associé à la surface la dite particule de type virus autre que la protéine spicule (spike) naturelle du coronavirus d'origine.

6. Une particule selon une quelconque des revendications 1 à 5, ladite particule comporte un génome de coronavirus dans lequel a été inséré un gène étranger ou des parties en dérivant.

7. Une particule selon une quelconque des revendications 1 à 6, qui a été atténuée.

8. Une particule selon une quelconque des revendications 1 à 7, consistant en un véhicule délivrant un gène.

9. Une particule selon une quelconque des revendications 1 à 7, consistant en un véhicule délivrant un antigène ou un épitope.

10. Une composition comprenant une particule selon une quelconque des revendications 1 à 9, pour un usage thérapeutique.

11. Une composition comprenant une particule selon une quelconque des revendications 1 à 9 et un support pharmaceutiquement acceptable pour un usage en tant qu'immunogène ou vaccin.

12. Une composition comprenant une particule selon une quelconque des revendications 1 à 9, pour un usage diagnostique.
